# FASCICULE DE BREVET EUROPEEN

(11) **EP 3 519 386 B1**
(45) Date de publication et mention de la délivrance du brevet: **29.03.2023**
(21) Numéro de dépôt: 17790800.1
(22) Date de dépôt: 27.09.2017
(51) Int. Cl.: C07D 209/88, C09K 11/07, H01L 51/50, C07C 211/61

(54) **NOUVEAUX MATERIAUX PI-CONJUGUES, LEURS PROCEDES DE PREPARATION ET LEURS UTILISATIONS COMME SEMICONDUCTEURS**
NEUARTIGE PI-KONJUGIERTE STOFFE, VERFAHREN ZUR HERSTELLUNG DAVON UND VERWENDUNGEN DAVON ALS HALBLEITER
NOVEL PI-CONJUGATED MATERIALS, METHODS FOR PREPARING SAME AND USES THEREOF AS SEMICONDUCTORS

(30) Priorité: 27.09.2016 FR 1659124
(43) Date de publication de la demande: 07.08.2019
(73) Titulaire: Degbia, Martial, 37000 Tours (FR)
(72) Inventeur: Degbia, Martial, 37000 Tours (FR)
(74) Mandataire: Grosset-Fournier, Chantal Catherine
(86) Numéro de dépôt international: PCT/FR2017/052634
(87) Numéro de publication internationale: WO 2019/063886

(56) Documents cités:
- WO-A1-2016/026122
- WO-A1-2016/117522
- CN-A- 102 219 700
- FR-A1- 3 056 591
- KR-A- 20140 132 562
- US-A1- 2006 051 690
- ASTA MICHALEVICIUTE ET AL: "Star-Shaped Carbazole Derivatives for Bilayer White Organic Light-Emitting Diodes Combining Emission from Both Excitons and Exciplexes", JOURNAL OF PHYSICAL CHEMISTRY C, vol. 116, no. 39, 6 septembre 2012 (2012-09-06), pages 20769-20778, XP055049978, ISSN: 1932-7447, DOI: 10.1021/jp306824u
- HYEJU CHOI ET AL: "Aqueous electrolyte based dye-sensitized solar cells using organic sensitizers", NEW JOURNAL OF CHEMISTRY, vol. 37, no. 2, 1 janvier 2013 (2013-01-01), pages 329-336, XP055425228, GB ISSN: 1144-0546, DOI: 10.1039/C2NJ40577F
- LENGVINAITE ET AL: "Carbazole-based aromatic amines having oxetanyl groups as materials for hole transporting layers", SYNTHETIC METALS, ELSEVIER SEQUOIA, LAUSANNE, CH, vol. 157, no. 13-15, 1 juillet 2007 (2007-07-01), pages 529-533, XP022234715, ISSN: 0379-6779, DOI: 10.1016/J.SYNTHMET.2007.05.015

## Description

La présente invention concerne des synthons contenant un noyau carbazole et/ou un noyau fluorène, leur préparation et leur utilisation pour la préparation de composés π-conjugués utiles dans l'optoélectronique organique en général tels qu'une cellule solaire photovoltaïque à colorant photosensible (DSSC), une cellule solaire photovoltaïque à quantum dot, une cellule solaire photovoltaïque hybride, une cellule solaire photovoltaïque organique, une cellule solaire photovoltaïque à pérovskite, une diode électroluminescente organique (OLEDs) et un transistor organique à effet de champ (OFETs).

De nombreuses molécules organiques π-conjuguées ont été utilisées dans l'électronique organique et en particulier dans les cellules solaires photovoltaïques organiques et hybrides. La synthèse de la plupart de ces molécules demeure encore complexe, de tels composés possèdent un coût (de production) élevé et cela limite leurs utilisations à grande échelle.

Les triarylamines, en général, sont largement utilisées en électronique organique. Les triarylamines dérivées de carbazole, en particulier, sont également utilisées. Des exemples de tels composés ont par exemple été décrits dans des publications telles que A. Michaliviciute et al. (Journal of Physical Chemistry C, vol.116, 36, 2012, pages 20769-20778) et H. Choi et al. (New Journal of Chemistry, vol. 37, 2, 2013, page 329-336) et dans les demandes CN 102 219 700 et WO2016/026122.

Une des limitations liées à ces composés π-conjugués réside cependant dans leur faible solubilité, qui limite en particulier leur dépôt en solution.

La présente invention propose de nouveaux dérivés du carbazole ou du fluorène ayant une solubilité améliorée par rapport à ceux de l'art antérieur et leur utilisation pour la préparation de composés π-conjugués, tels que des triarylamines. La présente invention concerne également les composés π-conjugués obtenus à partir de ces nouveaux dérivés du carbazole ou du fluorène.

Dans un **premier** aspect, la présente description concerne l'utilisation de composés de formule (I) : 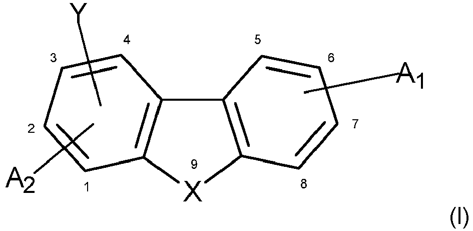 dans laquelle :
X est choisi dans le groupe constitué de :
**X¹** : N-[(CH₂)ₙ-O)]ₚ-R² ;
**X²** : N-R₁-{O-[(CH₂)ₙ-O)]ₚ-R²}ₘ ;
**X³** : CH-[(CH₂)ₙ-O)]ₚ-R² ;
**X⁴** : CH-R¹-{O-[(CH₂)ₙ-O)]ₚ₋R2}ₘ ;
**X⁵** : C-{[(CH₂)ₙ-O)]ₚ-R²}₂ ; et
**X⁶** : C-(R¹-{O-[(CH₂)ₙ-O)]ₚ-R²}ₘ)₂ ; où :
   R¹ est choisi dans le groupe constitué de aryle ; hétéroaryle ; alkyl-aryle ; cycloalkyle en C₃ à C₈ ; hétérocycloalkyle en C₃ à C₈; notamment aryle ou hétéroaryle, de préférence aryle ;
   n est un entier de 1 à 6, notamment 2 ou 3, en particulier 2 ;
   p est un entier de 1 à 20, notamment de 2 à 10, de préférence 4 ;
   m est un entier de 1 à 6, notamment de 1 à 3, en particulier 1 ;
   et lorsque m est supérieur à 1, les valeurs de p et de n dans chaque groupement -{O-[(CH₂)ₙ-O)]ₚ- peuvent être identiques ou différentes les unes des autres.
   R² est un alkyle en C₁ à C₁₂; aryle ; hétéroaryle ; cycloalkyle en C₃ à C₈ ; hétérocycloalkyle en C₃ à C₈ ;
   A¹, situé de préférence en position 6 ou 7 du cycle, est choisi dans le groupe constitué de H ; alkyle en C₁ à C₂₀ ; alcényle en C₂ à C₂₀ ; alcynyle en C₂ à C₂₀ ; aryle, hétéroaryle ; cycloalkyle en C₃ à C₈ ; NO₂, CF₃ ; CN ; N₃ ; F ; Cl ; NR^{a}R^{b}; OR^{c} ; SR^{c} ; C(=O)R^{d} ; ou un chromophore ; notamment H ou un chromophore ; où :
      R^{a} est choisi dans le groupe constitué de H ; alkyle en C₁ à C₂₀ ; aryle, hétéroaryle, cycloalkyle en C₃ à C₈ ; ou R^{a} et R^{b} forment ensemble un (C₃-C₇)hétérocyclyle ;
      R^{b} est choisi dans le groupe constitué de alkyle en C₁ à C₂₀ ; aryle, hétéroaryle, cycloalkyle en C₃ à C₈; ou R^{a} et R^{b} forment ensemble un (C₃-C₇)hétérocyclyle ;
      R^{c} est choisi dans le groupe constitué de alkyle en C₁ à C₂₀ ; aryle, hétéroaryle, cycloalkyle en C₃ à C₈; C(=O)-alkyle en C₁ à C₂₀ ; C(=O)-aryle, C(=O)-hétéroaryle, C(=O)-cycloalkyle en C₃ à C₈ ;
      R^{d} représente alkyle en C₁ à C₂₀ ; aryle, hétéroaryle, cycloalkyle en C₃ à C₈ ; NR^{a}R^{b}; OR^{c} ; SR^{c};
      Y est choisi dans le groupe constitué de NHP, Br ; I, B(OR^{e})₂ ; BF₃K ; Sn(Bu)₃ ; OSO₂CF₃ ; OSO₂C₄F₉ ; N₂⁺ ; où :
         Rₑ est choisi dans le groupe constitué de H, alkyle en C₁ à C₆, ou B(OR^{e})₂ est un ester boronique notamment choisi parmi l'ester pinacolique et l'ester glycolique ;
         le groupement Y est en position 2 du cycle lorsque X représente **X³** : CH-[(CH₂)ₙ-O)]ₚ-R² ; **X⁴** : CH-R¹-{O-[(CH₂)ₙ-O)]ₚ-R²}ₘ ; **X⁵** : C-{[(CH₂)ₙ-O)]ₚ-R²}₂ ; ou **X⁶** : C-(R¹-{O-[(CH₂)ₙ-O)]ₚ-R²}ₘ)₂ ; ou
         le groupement Y est en position 2 ou 3, de préférence en position 3, du cycle lorsque X représente N-[(CH₂)ₙ-O)]ₚ-R² ou N-R₁-{O-[(CH₂)-O)]ₚ₋R²}ₘ ;
         P représente H ou un groupement protecteur d'amine, notamment choisi dans le groupe constitué des benzyles, des carbamates, des amides et des imides cycliques ; notamment H ;
         A², situé de préférence en position 2 ou 3 du cycle est choisi dans le groupe constitué de H ; alkyle en C₁ à C₂₀ ; alcényle en C₂ à C₂₀ ; alcynyle en C₂ à C₂₀ ; aryle, hétéroaryle ; cycloalkyle en C₃ à C₈ ; NO₂, CF₃ ; CN ; N₃ ; F ; Cl ; NR^{a}R^{b}; OR^{c} ; SR^{c} ; C(=O)R^{d} ; et un chromophore ; où :
            R^{a} est choisi dans le groupe constitué de H ; alkyle en C₁ à C₂₀ ; aryle, hétéroaryle, cycloalkyle en C₃ à C₈ ; ou R^{a} et R^{b} forment ensemble un (C₃-C₇)hétérocyclyle ;
            R^{b} est choisi dans le groupe constitué de alkyle en C₁ à C₂₀ ; aryle, hétéroaryle, cycloalkyle en C₃ à C₈; ou R^{a} et R^{b} forment ensemble un (C₃-C₇)hétérocyclyle ;
            R^{c} est choisi dans le groupe constitué de alkyle en C₁ à C₂₀ ; aryle, hétéroaryle, cycloalkyle en C₃ à C₈ ; C(=O)-alkyle en C₁ à C₂₀ ; C(=O)-aryle, C(=O)-hétéroaryle, C(=O)-cycloalkyle en C₃ à C₈ ;
            R^{d} représente alkyle en C₁ à C₂₀ ; aryle, hétéroaryle, cycloalkyle en C₃ à C₈ ; NR^{a}R^{b}; OR^{c} ; SR^{c}.
            pour la préparation de composés π-conjugués, en particulier des composés π-conjugués utiles en tant que semi-conducteurs, notamment des semi-conducteurs organiques, ou pour la préparation de colorants.

Les composés selon l'invention présentent l'avantage de combiner l'extension de conjugaison et une solubilité améliorée de la molécule finale. En effet, la position 9 du carbazole peut être fonctionnalisée par des groupements aromatiques, ce qui permet d'obtenir des synthons ayant une conjugaison étendue. Ces derniers peuvent être combinés à des synthons dérivés de fluorène ou de carbazole portant des groupements susceptibles d'améliorer la solubilité. Le fait d'avoir des dérivés de triarylamines permet une disposition en 3D, ce qui peut limiter les interactions du type π-stacking et permet d'obtenir facilement des verres moléculaires. Pour une utilisation dans les cellules solaires photovoltaïques par exemple, les propriétés attendues pour ces composés sont les suivantes :
▪ une bonne solubilité afin de permettre un dépôt en solution,
▪ une bonne stabilité électrochimique permettant une oxydation réversible sans dégradation rapide,
▪ une bonne stabilité thermique afin d'avoir une utilisation à haute température,
▪ une bonne propriété de verre moléculaire afin d'avoir une couche homogène et continue lors du dépôt et éviter ainsi les cristallisations,
▪ une mobilité élevée afin d'avoir un transport efficace des charges (pour les semi-conducteurs)
▪ une forte absorption de photons dans les domaines d'émission du soleil (pour les colorants)

Les composés selon la présente invention se différencient de ceux connus de l'art antérieur par la présence en position 9 du carbazole ou du fluorène d'un groupement de type oligo ou polyglycol. La présence de groupements oligo ou polyglycol permet d'améliorer la solubilité du composé π-conjugué.

Par « composés utiles en tant que semi-conducteurs, notamment des semi-conducteurs organiques », on entend au sens de la présente invention un composé organique sous la forme d'un monomère ou d'un polymère capable de conduction par les électrons et/ou les trous.

De tels composés ont par exemple été décrits dans Journal of Power Sources, 2014 vol. 253, pp. 230-238 ; Synthetic Metals, 2014 vol. 195, pp. 328-334 ; Chem. Commun., vol. 51, no. 45, pp. 9305-9308, 2015 et J. Mater. Chem. A, vol. 4, no. 4, pp. 1186-1190, 2016.

Par « composés utiles en tant que colorants », on entend au sens de la présente invention des composés semi-conducteurs susceptibles d'absorber des photons dans les domaines d'émission du soleil. Il s'agit notamment de colorants permettant l'absorption de photons conçus en s'inspirant de la structure des semiconducteurs précédemment décrits. Parmi les groupements chromophores susceptibles d'être utilisés pour la préparation de colorants, on peut notamment citer les groupes de type cyano, les cyano acides et les dérivés de rhodamine.

Il s'agit notamment des structures suivantes :

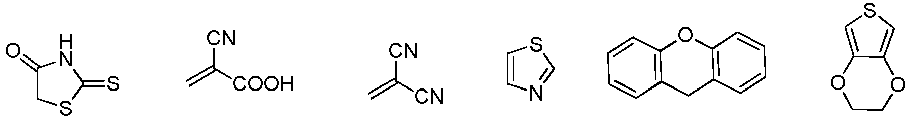

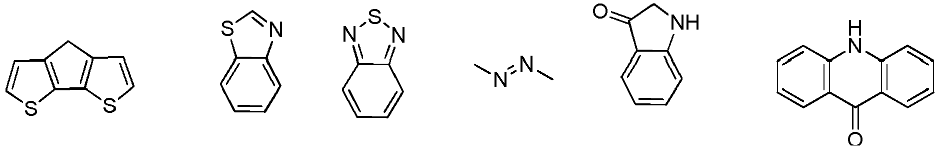

Dans la suite de la présente demande, on utilisera l'expression « carbazole selon la présente invention » pour désigner un résidu de formule suivante lié au reste de la molécule : 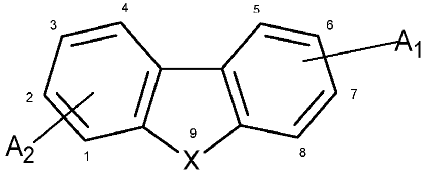 dans laquelle :
A¹ et A² sont tels que définis dans les revendications,
X est choisi dans le groupe constitué de **X¹** ou X² tels que définis dans les revendications.

Dans la suite de la présente demande, on utilisera l'expression « fluorène selon la présente invention » pour désigner un résidu lié au reste de la molécule de formule suivante : 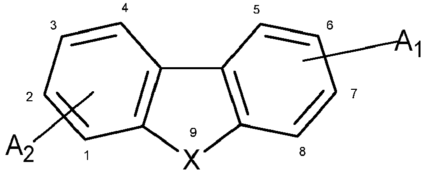 dans laquelle :
A¹ et A² sont tels que définis dans les revendications,
X est choisi dans le groupe constitué de **X³, X⁴, X⁵** ou **X⁶** tel que défini dans les revendications.

Au sens de la description, l'expression « alkyle en C₁ à C₂₀» désigne une chaine carbonée acyclique, saturée, linéaire ou ramifiée, comprenant 1 à 20 atomes de carbone. Des exemples d'alkyle en C₁ à C₂₀ incluent les groupes méthyle, éthyle, propyle, butyle, pentyle, hexyle ou heptyle. La définition de propyle, butyle, pentyle, hexyle ou heptyle inclut tous les isomères possibles. Par exemple, le terme butyle comprend n-butyle, iso-butyle, sec-butyle et ter-butyle.

Au sens de la description, l'expression « alcényle en C₂ à C₂₀» désigne une chaine carbonée acyclique, saturée, linéaire ou ramifiée, comprenant 2 à 20 atomes de carbone et au moins une liaison C=C. Des exemples d'alcényle en C₂ à C₂₀ incluent les groupes éthényle, propényle, butényle, pentényle, hexényle ou heptényle. La définition de propényle, butényle, pentényle, hexényle ou heptényle inclut tous les isomères possibles, notamment les isomères *cis* et *trans.*

Au sens de la description, l'expression « alcynyle en C₂ à C₂₀» désigne une chaine carbonée acyclique, saturée, linéaire ou ramifiée, comprenant 2 à 20 atomes de carbone et au moins une triple liaison carbone-carbone. Des exemples d'alcynyle en C₂ à C₂₀ incluent les groupes éthynyle, propynyle, butynyle, pentynyle, hexynyle ou heptynyle. La définition de propynyle, butynyle, pentynyle, hexynyle ou heptynyle inclut tous les isomères possibles.

Au sens de la présente invention, l'expression «cycloalkyle en C₃ à C₈ » désigne un mono-, bi- ou tri-cycle saturé ou partiellement saturé, comprenant 3 à 8 atomes de carbone. Des exemples de (C₃-C₇)carbocyclyle incluent les groupes cyclopropyle, cyclobutyle, cyclopentyle, cyclohexyle ou cycloheptyle.

Au sens de la présente invention, l'expression « hétérocyclyle en C₃ à C₇» désigne un hétérocycle saturé comprenant 3, 4, 5, 6, ou 7 atomes dans le cycle dont 1, 2 ou 3 hétéroatomes, choisis parmi O, S ou N, remplacent respectivement 1, 2 ou 3 atomes de carbone. Des exemples d' hétérocyclyles en C₃ à C₇ incluent pyrrolidinyle, pipéridinyle, pipérazinyle et morpholinyle. L'hétérocyclyle peut être substitué par un ou plusieurs groupes tels que méthyle, cyclohexyle, cyclopentyle, halogène, ou (C₁-C₃)alkyle-halogène.

Le terme « aryle » désigne un monocycle aromatique comprenant de 5 à 6 atomes de carbone, pouvant être lui-même fusionné avec un second cycle saturé, insaturé ou aromatique. Le groupe aryle peut être substitué par un ou plusieurs groupes indépendamment choisis parmi les groupes alkyle, halogène, (C₁-C₃)alkyle-halogène, hydroxyle, alkoxy, amino, nitro, cyano, trifluoro, acide carboxylique ou ester carboxylique. Des exemples d'aryles substitués incluent, sans restrictions, le 2-, 3- ou 4-(N, N-diméthylamino)-phényle, 2-, 3- ou 4-cyanophényle, le 2-, 3- ou 4-nitrophényle, le 2-, 3- ou 4-fluoro-, chloro-, bromo- ou iodo-phényle. Des exemples préférés d'aryle sont phényle, 4,4-biphényle, naphtyle, anthracényle, pyrényle et terphényle.

L'hétéroaryle est notamment choisi dans le groupe constitué de 2-, 3-ou 4-pyridinyle, 2- ou 3-pyrrolyle ; 2- ou 3-thiényle.

Au sens de la présente Invention, le terme « *hétéroaryle* » désigne un aryle mono- ou polycyclique tel que décrit ci-dessus, dans lequel un ou plusieurs atomes de carbone ont été remplacés par un ou plusieurs hétéroatomes choisis parmi N, O ou S. Le terme hétéroaryle inclut tous les isomères possibles. Des exemples d'hétéroaryles incluent les groupes furanyle, thiényle, pyrrolyle, N-alkyle pyrrolyle, indolyle, aza-indolyle, benzofuranyle, benzothiophényle, pyridyle, oxazolyle, thiazolyle, imidazolyle, pyrimidyle, pyrazinyle, triazolyle, N-alkyle triazolyle, thiadiazolyle, oxadiazolyle, tétrazolyle and N-alkyle tétrazolyle. Le groupe hétéroaryle peut être de plus substitué par un ou plusieurs groupes indépendamment choisis parmi les groupes alkyle, (C₁-C₃)alkyle-halogène, alkoxy, halogène, hydroxyle, amino, nitro, cyano, trifluoro, acide carboxylique ou ester carboxylique.

La présente description concerne l'utilisation d'un composé de formule (I) pour la préparation d'une triarylamine de formule (II) : 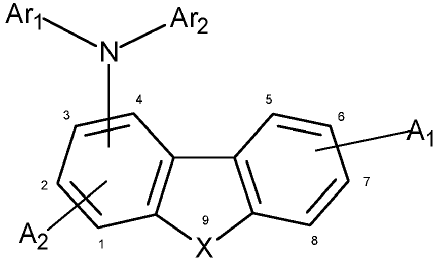 dans laquelle :
Ar¹ et Ar², identiques ou différents, représentent un aryle ou un hétéroaryle.

L'aryle est notamment choisi dans le groupe constitué de phényle, biphényle, naphtyle, anthracényle, pyrényle et terphényle ou un aryle choisi dans la liste suivante :

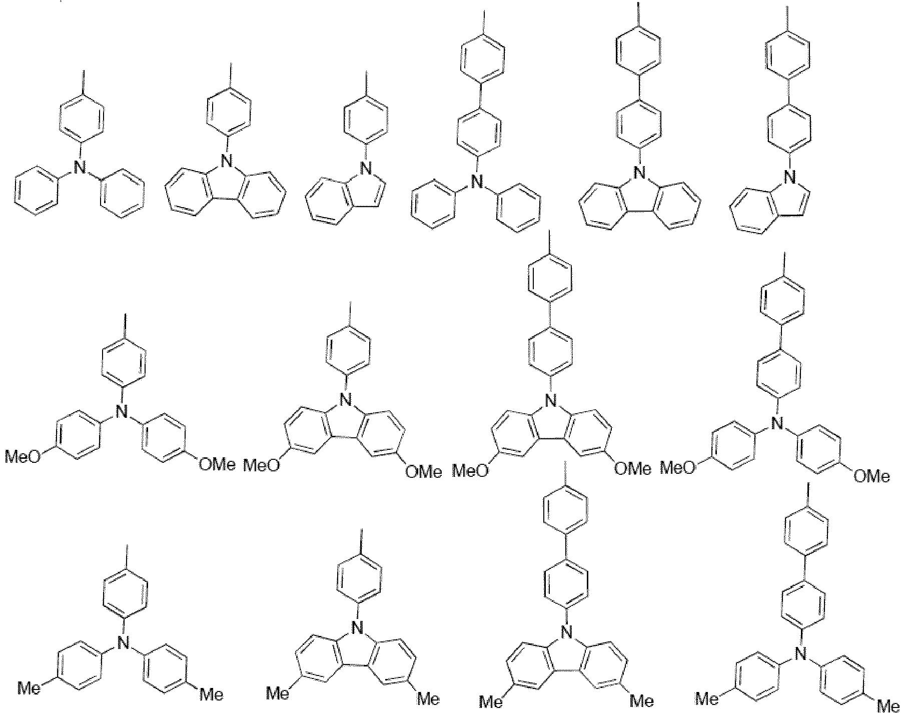

L'hétéroaryle est notamment choisi dans le groupe constitué de 2-, 3-ou 4-pyridinyle, 2- ou 3-pyrrolyle ; 2- ou 3-thiényle, 2- ou 3-furanyle, 2-,3-,4-,5-, 6- ou 7-indolyle.

Ledit groupement aryle ou hétéroaryle peut éventuellement être substitué par un ou plusieurs groupements, en particulier les groupements permettant d'optimiser les propriétés semi-conductrices de la triarylamine. Ils peuvent être déterminés par l'Homme du Métier selon les propriétés recherchées pour la triarylamine.

A titre illustratif mais non limitatif, le composé de formule (II) peut par exemple être obtenu par formation d'une liaison carbone-azote entre une diarylamine de formule Ar¹Ar²NH et un composé de formule (I) dans laquelle Y est choisi dans le groupe constitué de Br ; I, B(OR^{e})₂ ; BF₃K et OSO₂CF₃, de préférence catalysée par un métal de transition tel que le cuivre ou le palladium. Les conditions de formation de liaisons carbone-azote par ces métaux de transition ont été décrites dans de nombreux ouvrages ou ont fait l'objet de revues.

A titre illustratif mais non limitatif, le composé de formule (II) peut également être obtenu par exemple par formation d'une liaison carbone-azote entre un composé de formule (I) dans laquelle Y est un groupement NHP tel que défini ci-dessus et un halogénure d'aryle. Lorsque Ar¹ et Ar² sont différents, le produit de formule (III) peut être préparé en deux étapes à partir d'un composé de formule (I) dans laquelle P est un groupement protecteur d'amine en mettant en oeuvre une étape intermédiaire de déprotection de la diarylamine selon la séquence R-NP-Ar¹ -> R-NH-Ar¹ -> R-N Ar¹Ar² où R représente un carbazole selon la présente description ou un fluorène selon la présente description.

La présente description concerne l'utilisation d'un composé de formule (I) tel que défini ci-dessus pour la préparation d'un semi-conducteur organique de type triarylamine dans lequel deux des trois groupements aryles sont un carbazole selon la présente description ou un fluorène selon la présente description.

La présente description concerne également l'utilisation de deux composés de formule (I), identiques ou différents, pour la préparation d'une triarylamine de formule (III) suivante : 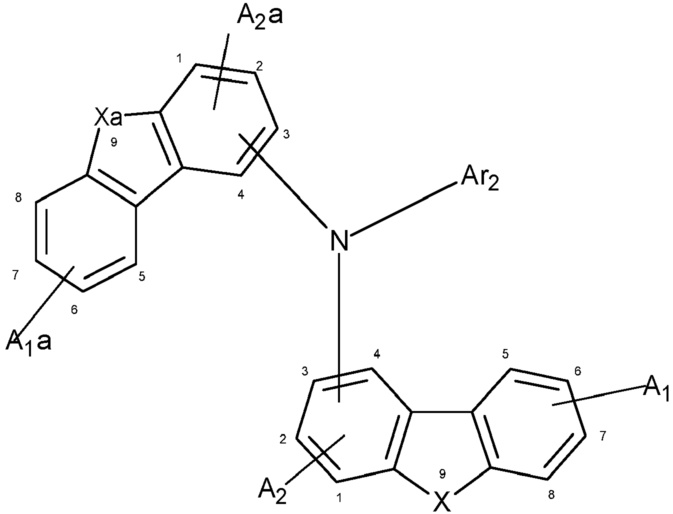 dans laquelle :
A^{2a} et A^{1a} ont la signification donnée pour A¹ et A² dans la formule (I), A^{2a} et A² pouvant être identiques ou différents l'un de l'autre et A^{1a} et A¹ pouvant être identiques ou différents l'un de l'autre,
X et X^{a}, identiques ou différents l'un de l'autre, sont tels que définis dans la formule (I),
Ar² est tel que défini dans la formule (II).

Les composés de formule (III) peuvent donc être des composés dans lesquels sont présents :
- deux carbazoles selon la présente description identiques entre eux,
- deux carbazoles selon la présente description différents l'un de l'autre,
- un carbazole selon la présente invention et un fluorène selon la présente description, les groupements A^{2a} et A² pouvant être identiques ou différents l'un de l'autre et A^{1a} et A¹ pouvant être identiques ou différents l'un de l'autre,
- deux fluorènes selon la présente description identiques entre eux,
- deux fluorènes selon la présente description différents l'un de l'autre.

A titre illustratif mais non limitatif, le composé de formule (III) peut par exemple être obtenu par formation d'une liaison carbone-azote entre une arylamine de formule Ar²NH₂ et un premier composé de formule (I) dans laquelle Y est choisi dans le groupe constitué de Br ; I, B(OR^{e})₂ ; BF₃K et OSO₂CF₃, de préférence catalysée par un métal de transition tel que le cuivre ou le palladium, puis par formation d'une liaison carbone-azote avec un deuxième composé de formule (I) dans laquelle Y est choisi dans le groupe constitué de Br ; I, B(OR^{e})₂ ; BF₃K et OSO₂CF₃, de préférence catalysée par un métal de transition tel que le cuivre ou le palladium.

A titre illustratif mais non limitatif, lorsque les deux carbazoles selon la présente description d'une part ou les deux fluorènes selon la présente description d'autre part sont identiques entre eux, le composé de formule (III) est avantageusement préparé en une seule étape par formation des deux liaisons carbone-azote simultanément (procédé di *one pot*).

Avantageusement, la présente description concerne des composés de formule (III-1) : 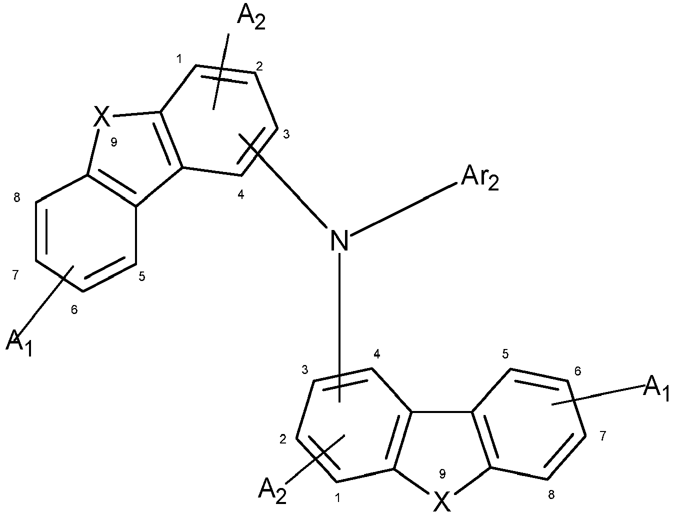 dans laquelle :
A² et A¹ sont tels que définis dans la formule (I),
Ar² est tel que défini dans la formule (II),
X est tel que défini dans la formule (I).

De manière plus avantageuse, les composés de formule (III-1) sont des triarylamines de formule (III-1-a) dans lesquelles l'atome d'azote est lié à deux carbazoles identiques et X représente **X¹** ou **X²**, des triarylamines de formule (III-1-b) dans lesquelles l'atome d'azote est lié à deux fluorènes identiques et X représente **X³** ou **X⁴** et des triarylamines de formule (III-1-c) dans lesquelles l'atome d'azote est lié à deux fluorènes identiques et X représente **X⁵** ou **X⁶**.

La présente description concerne l'utilisation d'un composé de formule (I-2) dans laquelle A² représente H pour la préparation d'un composé de formule (III-2) : 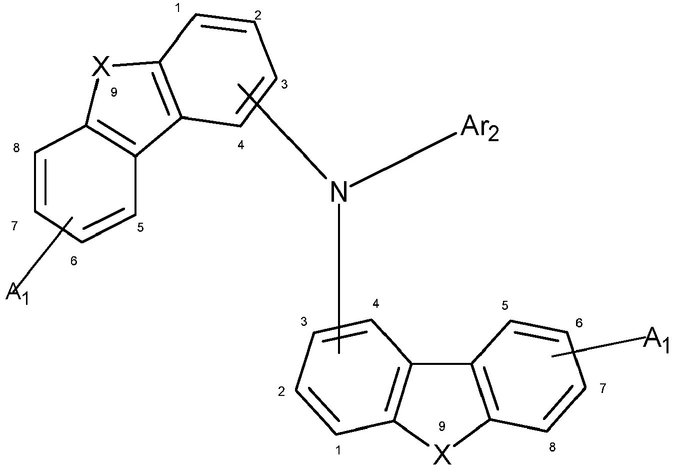 dans laquelle les deux groupements A¹ sont identiques entre eux et sont choisis parmi H, aryle ou un chromophore et Ar² est tel que défini dans la formule (II).

Dans un mode de réalisation spécifique, A¹ et A² représentent H et la présente description concerne l'utilisation d'un composé de formule (I-1-1) : 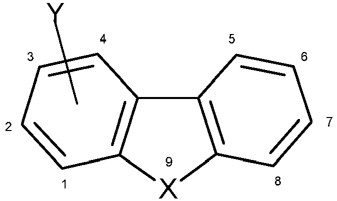 pour la préparation d'un composé de formule (III-3) :

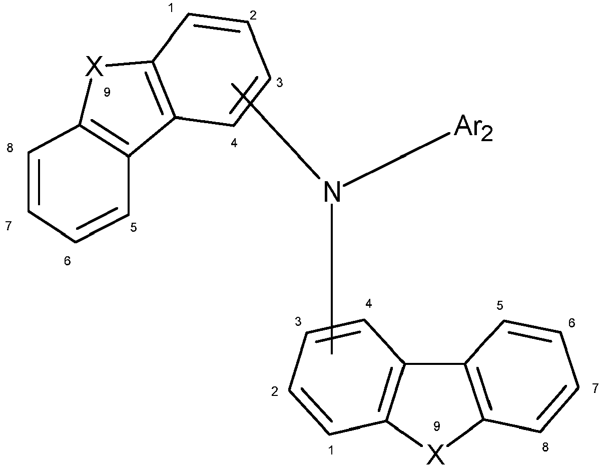

La présente description concerne en particulier l'utilisation d'un composé de formule (I) pour la préparation des composés de formule suivante (III-3-1) : 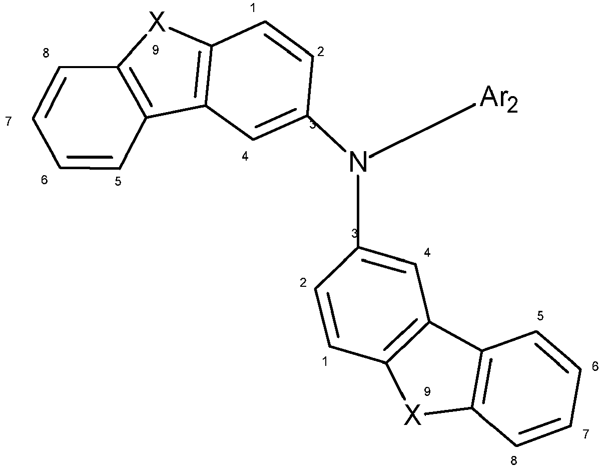
dans laquelle les groupements X, identiques entre eux, sont choisis parmi **X¹** et **X²**,
ou de formule suivante (III-3-2) : 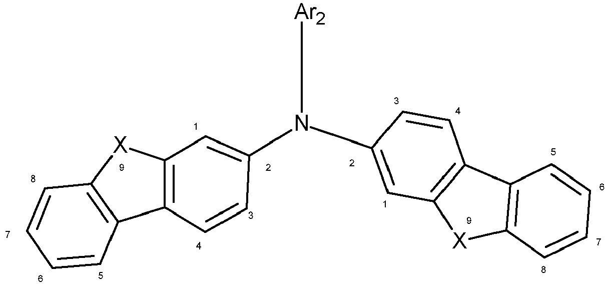
dans laquelle les groupements X, identiques entre eux, sont choisis parmi **X³**, **X⁴**, **X⁵** et **X⁶**.

Dans un troisième mode de réalisation de ce premier aspect, la présente invention concerne l'utilisation d'un composé de formule (I) tel que défini ci-dessus pour la préparation d'un semi-conducteur organique de type triarylamine dans lequel les trois groupements aryles sont des carbazoles selon la présente invention ou des fluorènes selon la présente invention.

Dans un mode de réalisation, la présente invention concerne l'utilisation d'un composé de formule (I), pour la préparation d'une triarylamine de formule (IV) suivante : 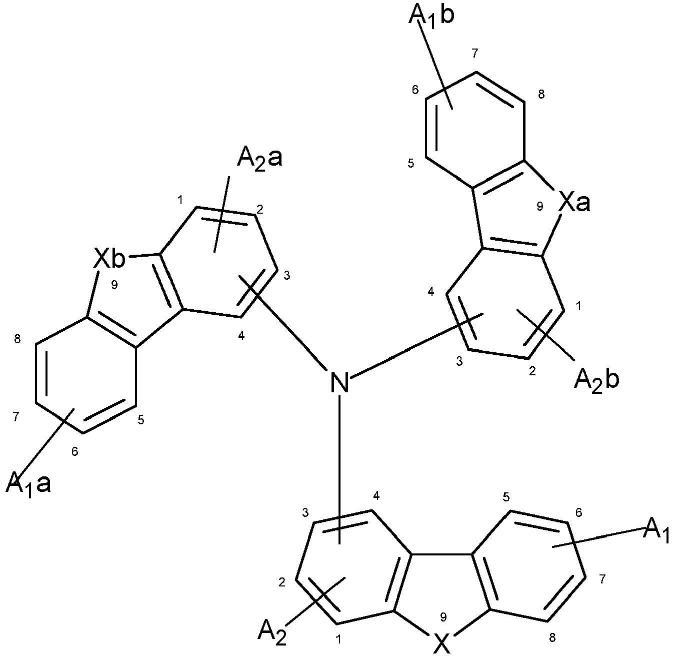 dans laquelle A¹, A², A^{1a}, A^{2a}, A^{1b} et A^{2b} ont la signification donnée pour A¹ et A² dans la formule (I), A^{2a}, A^{2b} et A² pouvant être identiques ou différents l'un de l'autre et A^{1a}, A¹ et A^{1b} d'autre part pouvant être identiques ou différents l'un de l'autre et X, X^{a} et X^{b}, identiques ou différents, ont la signification donnée pour X dans la formule (I).

Lorsque les trois carbazoles selon la présente invention ou fluorènes selon la présente invention sont différents les uns des autres, le composé de formule (IV) peut par exemple être obtenu par formation séquentielle de liaisons carbone-azote entre un composé de formule (I) dans laquelle Y représente NHP et un premier composé de formule (I) dans laquelle Y est choisi dans le groupe constitué de Br ; I, B(OR^{e})₂ ; BF₃K et OSO₂CF₃ et un second composé de formule (I) dans laquelle Y est choisi dans le groupe constitué de Br ; I, B(OR^{e})₂ ; BF₃K et OSO₂CF₃.

Lorsque deux carbazoles selon la présente invention ou fluorènes selon la présente invention sont différents les uns des autres, les composés de formule (IV) peuvent être obtenus par la formation de deux liaisons carbone-azote entre un composé de formule (I) dans laquelle Y représente NHP et deux équivalents d'un composé de formule (I) dans laquelle Y est choisi dans le groupe constitué de Br ; I, B(OR^{e})₂ ; BF₃K et OSO₂CF₃.

Lorsque les trois carbazoles selon la présente invention ou fluorènes selon la présente invention sont identiques entre eux, les composés de formule (IV) peuvent être obtenus par formation d'une liaison carbone-azote entre les composés de formule (I) dans laquelle Y est choisi dans le groupe constitué de Br ; I, B(OR^{e})₂ ; BF₃K et OSO₂CF₃ et l'ammoniac ou un équivalent synthétique de l'ammoniac. Alternativement, ces composés peuvent être préparés par la formation de liaisons carbone azote entre un composé de formule (I) dans laquelle Y est NHP et deux équivalents de composés de formule (I) dans laquelle Y est choisi dans le groupe constitué de Br ; I, B(OR^{e})₂ ; BF₃K et OSO₂CF₃.

De manière avantageuse, la présente invention concerne les composés de formule (IV-1) : 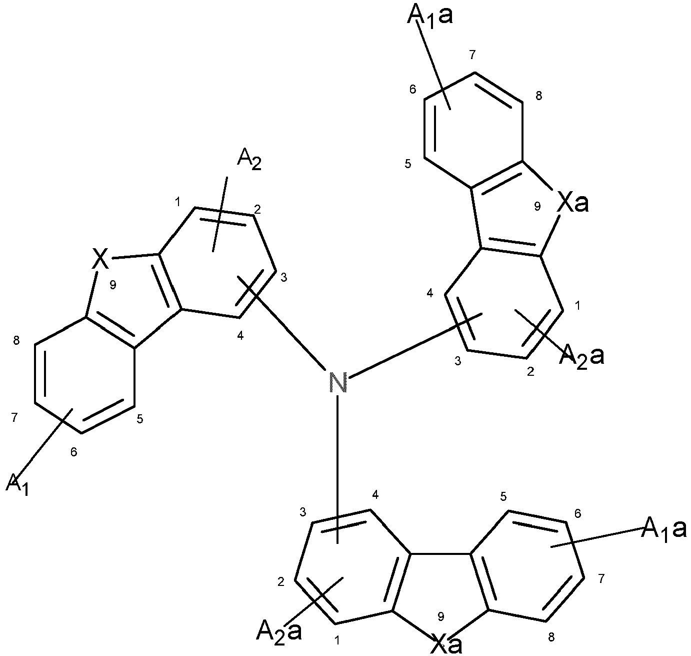 dans laquelle X représente X¹ ou X² tel que défini dans la formule (I) et X^{a} représente X³, X⁴, X⁵ ou X⁶ ; lesdits composés comprenant alors deux carbazoles identiques entre eux,
ou
dans laquelle X représente X³, X⁴, X⁵ ou X⁶ tel que défini dans la formule (I) et X^{a}, identiques, représentent X¹ ou X², lesdits composés comprenant alors deux fluorènes identiques entre eux,
A^{2a} et A² d'une part pouvant être identiques ou différents, et A^{1a} et A¹ d'autre part pouvant être identiques ou différents.

Selon un mode de réalisation encore plus avantageux, la présente invention concerne l'utilisation d'un composé de formule (I) : 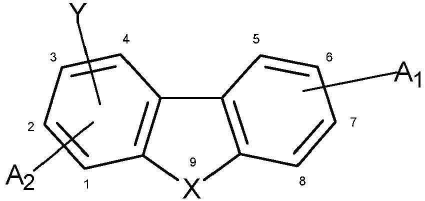 pour la préparation d'un composé de formule (IV-1) : 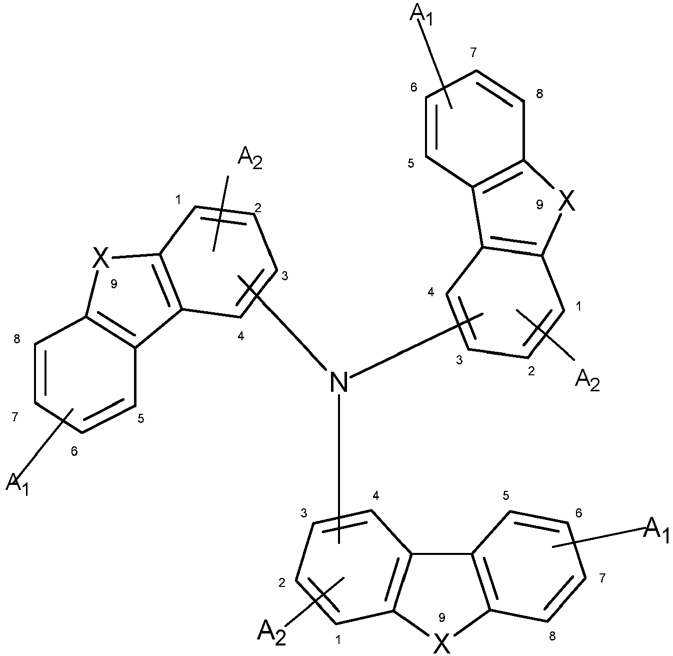 dans laquelle A¹, A², X et Y sont tels que définis à la formule (II).

Selon un premier mode de réalisation, la présente description concerne l'utilisation d'une amine de formule (I-2) : 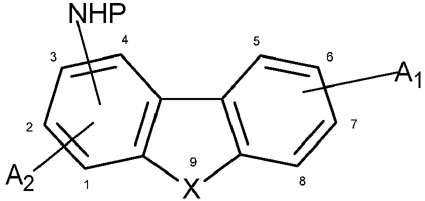 dans laquelle A1, A2, P et X sont tels que définis dans la formule (I) pour la préparation de composés π-conjugués, en particulier des composés π-conjugués utiles en tant que semi-conducteurs, notamment des semi-conducteurs organiques, ou pour la préparation de colorants.

Les amines de formule (I-2) peuvent être utilisées pour la préparation de triarylamines de formule (II) telle que définie ci-dessus :

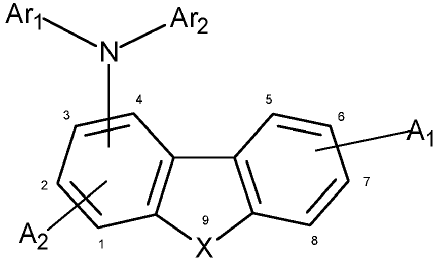

Les amines de formule (I-2) telles que définies ci-dessus peuvent également être utilisées pour la préparation de triarylamines de formule (III) telle que définie ci-dessus :

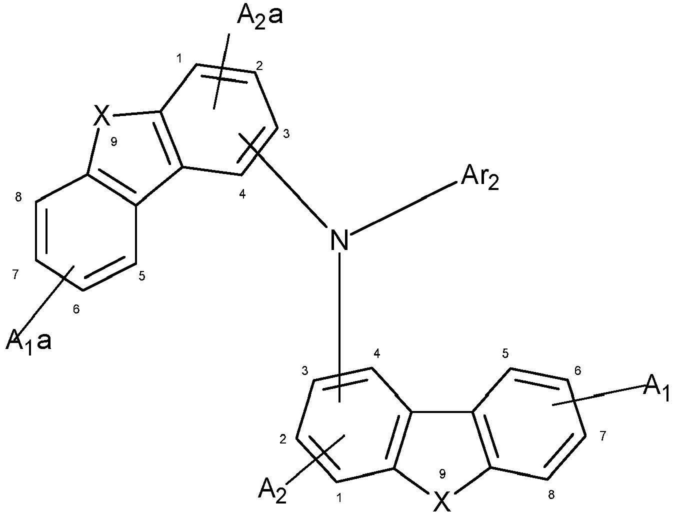

Les amines de formule (I-2) peuvent également être utilisées pour la préparation de triarylamines de formule (IV) telle que définie ci-dessus :

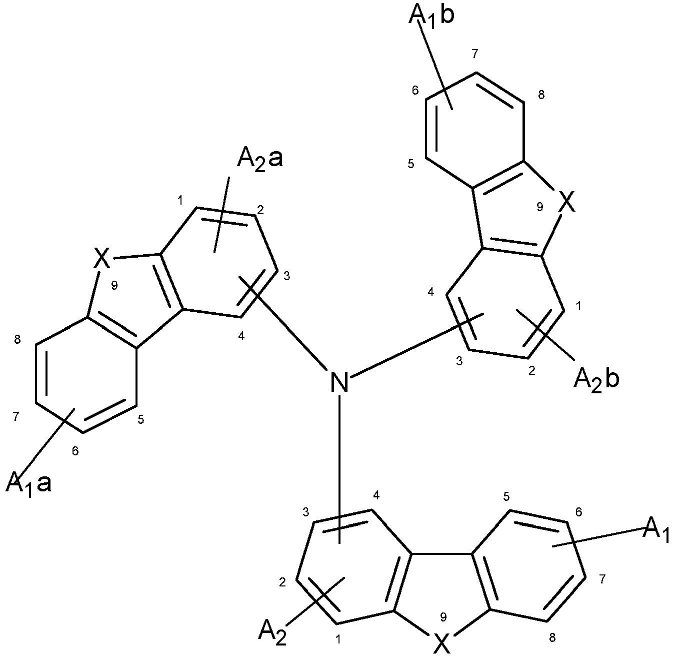

Selon un deuxième mode de réalisation, la présente invention concerne l'utilisation d'un halogénure d'aryle de formule (I-3) : 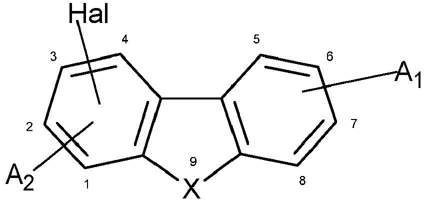 dans laquelle A1, A2 et X sont tels que définis dans la formule (I), et
Hal est choisi dans le groupe constitué de Br ; I, B(OR^{e})₂ ; BF₃K; Sn(Bu)₃; OSO₂CF₃ ; OSO₂C₄F₉ ; N₂⁺ ;
pour la préparation de composés π-conjugués, en particulier des composés π-conjugués utiles en tant que semi-conducteurs, notamment des semi-conducteurs organiques, ou pour la préparation de colorants.

Au sens de la présente invention, on entend par « halogénure d'aryle » un composé susceptible de conduire à la formation d'une liaison C-N ou une liaison C-C, notamment par voie de catalyse par le palladium ou le cuivre. Les réactions correspondantes sont connues de l'Homme du Métier. Il s'agit du couplage de type Buchwald-Hartwig, Ullmann (formation de liaisons C-N), du souplage de Suzuki, du couplage de Stille, du couplage de Kumada ou du couplage de Negishi (formation de laisions C-C).

Ces halogénures d'aryle de formule (I-3) peuvent être utilisés pour la préparation de triarylamines de formule (II) telle que définie ci-dessus :

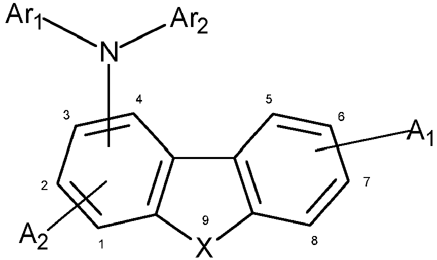

Ces halogénures d'aryle de formule (I-3) peuvent également être utilisés pour la préparation de triarylamines de formule (III) telle que définie ci-dessus :

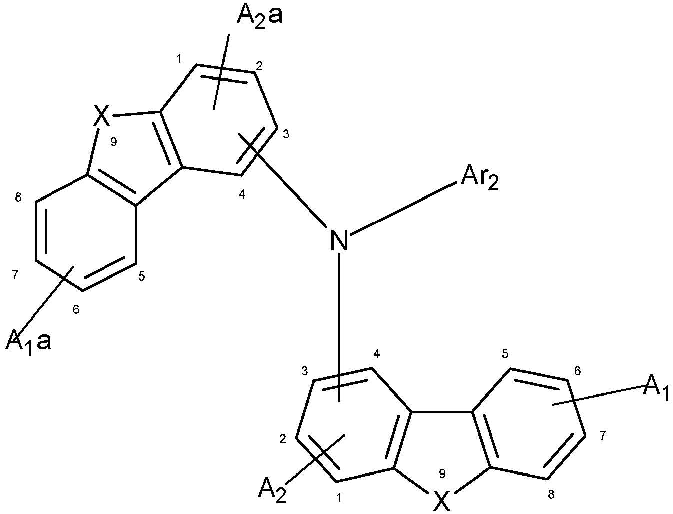

Ces halogénures d'aryle de formule (I-3) peuvent être utilisés pour la préparation de triarylamines de formule (IV) telle que définie ci-dessus :

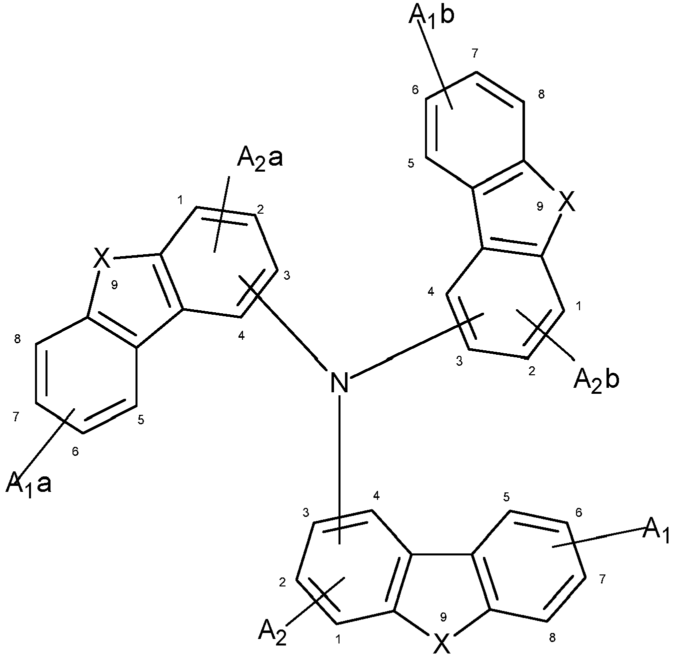

Dans un troisième mode de réalisation, la présente description concerne l'utilisation d'un composé de formule (I-3) : 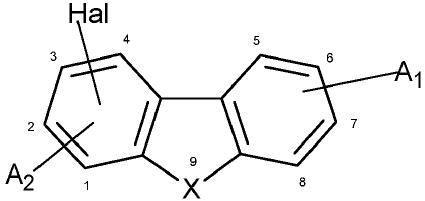 dans laquelle X est tel que défini dans la formule (I),
Hal est choisi dans le groupe constitué de Br ; I, B(OR^{e})₂ ; BF₃K; Sn(Bu)₃; OSO₂CF₃ ; OSO₂C₄F₉ ; N₂⁺ ;
pour la préparation de composés de formule (V) : 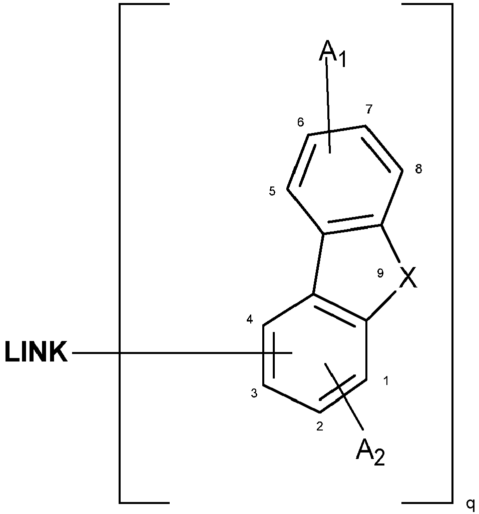 dans laquelle :
   **LINK** représente un aryle, un hétéroaryle, un polyaryle ou une triarylamine,
   q est 2,3, 4, 5 ou 6

Avantageusement, mais sans y être limité, le groupement **LINK** est choisi dans le groupe constitué de : 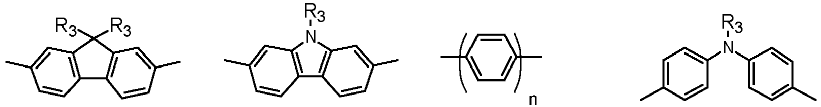 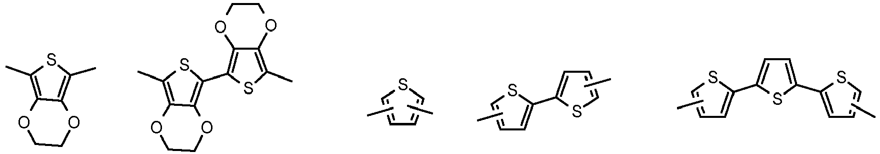 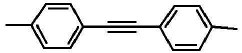 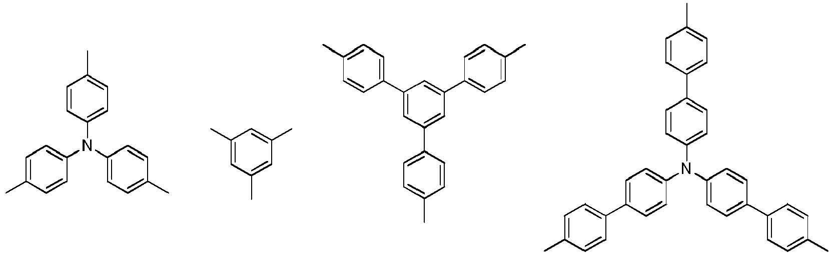 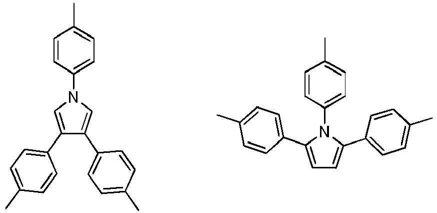 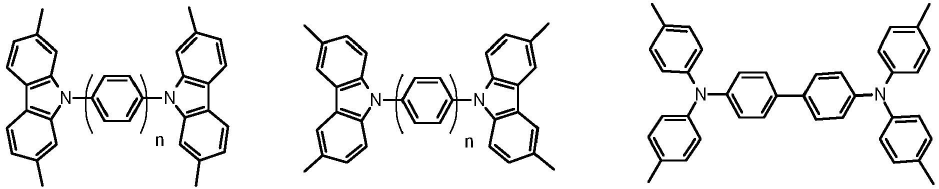 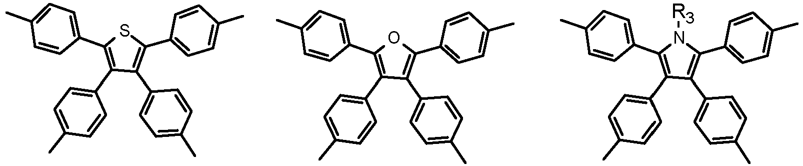 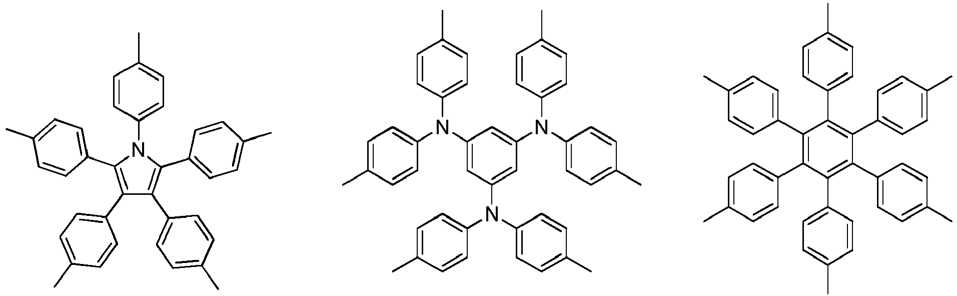 dans laquelle R³ est choisi dans le groupe constitué de alkyle en C₁ à C₂₀ ; alcényle en C₂ à C₂₀ ; alcynyle en C₂ à C₂₀ ; aryle, hétéroaryle ; cycloalkyle en C₃ à C₈ ; C(=O)R^{e} ; où R^{e} est choisi dans le groupe constitué de alkyle en C₁ à C₂₀ ; aryle, hétéroaryle, cycloalkyle en C₃ à C₈.

Dans un autre mode de réalisation, la présente description concerne l'utilisation d'un composé de formule (I-4) : 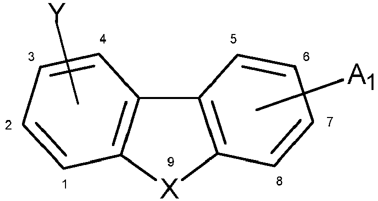 dans laquelle A¹ est un chromophore, notamment choisi parmi les groupes de type cyano, les cyano acides et les dérivés de rhodamine, en particulier : 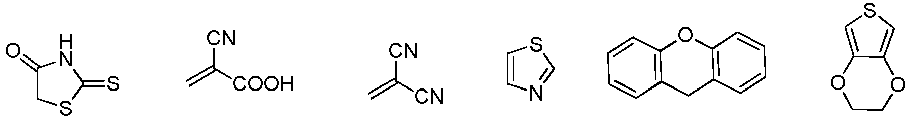 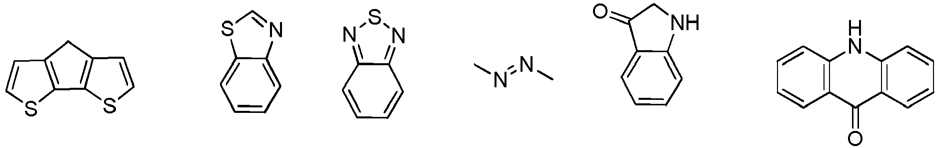 pour la préparation d'un composé de formule (II-4) :

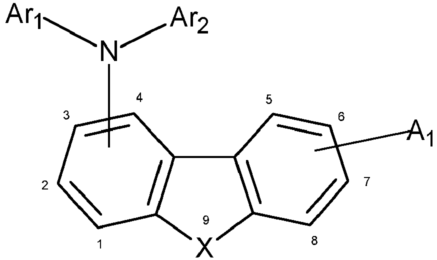

Dans un autre mode de réalisation, la présente description concerne l'utilisation d'un composé de formule (I-4) : 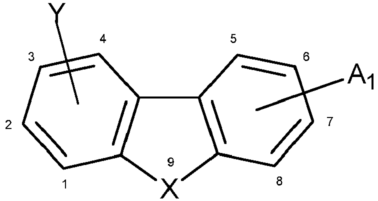 dans laquelle A¹ est un chromophore, notamment choisi parmi les groupes de type cyano, les cyano acides et les dérivés de rhodamine, en particulier : 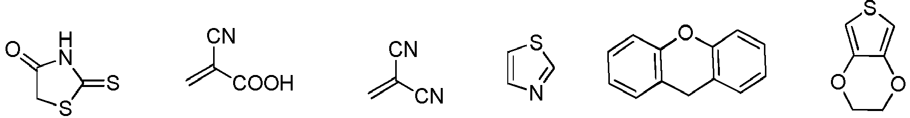 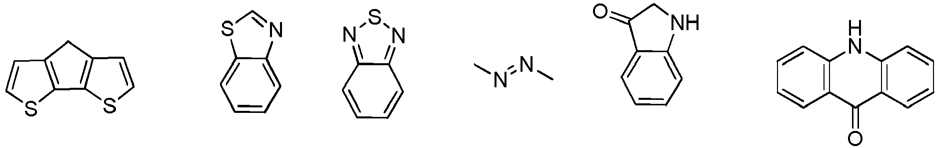 pour la préparation d'un composé de formule (III-4) : 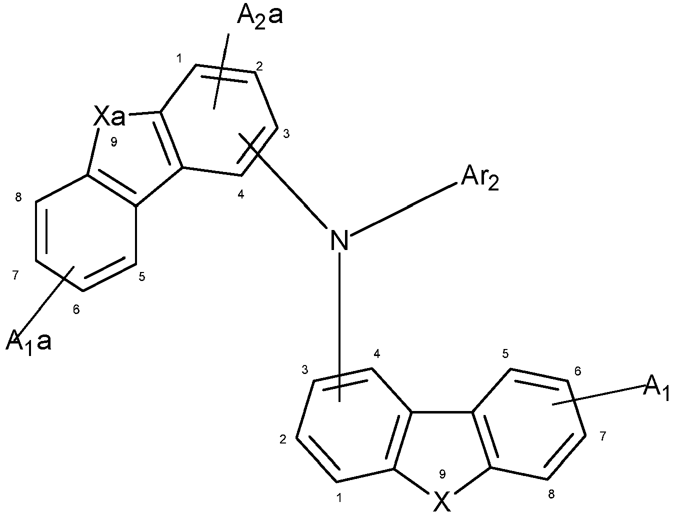 dans laquelle A^{1a}, A^{2a} et X^{a} sont tels que définis dans la formule (I).

Dans un autre mode de réalisation, la présente invention concerne l'utilisation d'un composé de formule (I-4) : 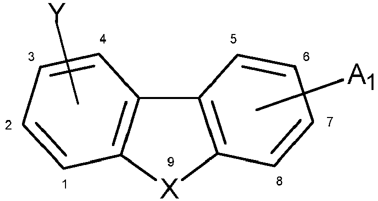 dans laquelle A¹ est un chromophore, notamment choisi parmi les groupes de type cyano, les cyano acides et les dérivés de rhodamine, en particulier : 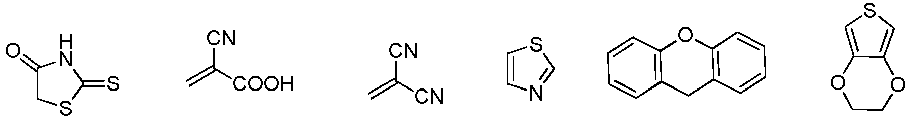 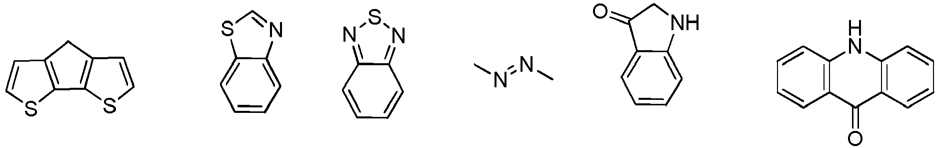 pour la préparation d'un composé de formule (IV-4) : 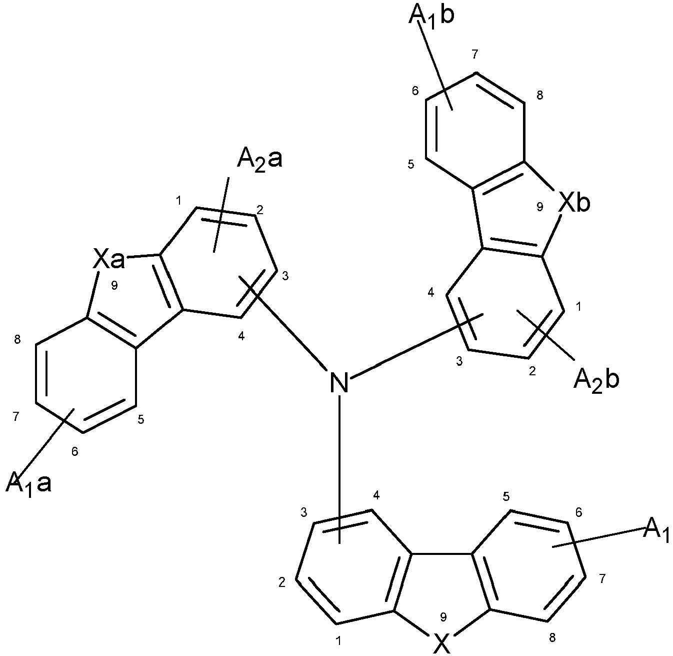 dans laquelle A^{1a} et A^{1b}, identiques ou différents l'un de l'autre, A^{2a} et A^{2b}, identiques ou différents l'un de l'autre et X^{a} et X^{b}, identiques ou différents l'un de l'autre sont tels que définis dans la formule (I).

Dans un **deuxième aspect**, les composés de formule (I) sont des fluorènes et la présente invention concerne l'utilisation de composés de formule (I-5) : 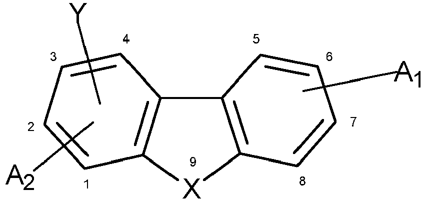 dans laquelle :
X est choisi dans le groupe constitué de :
**X³** : CH-[(CH₂)ₙ-O)]ₚ-R² ;
**X⁴** : CH-R¹-{O-[(CH₂)ₙ-O)]ₚ₋R2}ₘ ;
**X⁵** : C-{[(CH₂)ₙ-O)]ₚ-R²}₂ ; et
**X⁶** : C-(R¹-{O-[(CH₂)ₙ-O)]ₚ-R²}ₘ)₂ ;
et Y, A¹ et A² sont tels que définis dans la formule (I)
pour la préparation de composés π-conjugués, en particulier des composés π-conjugués utiles en tant que semi-conducteurs, notamment des semi-conducteurs organiques, ou pour la préparation de colorants.

La présente description concerne donc dans un mode de réalisation l'utilisation d'un composé de formule (I-5) pour la préparation de triarylamines de formule (II-5) : 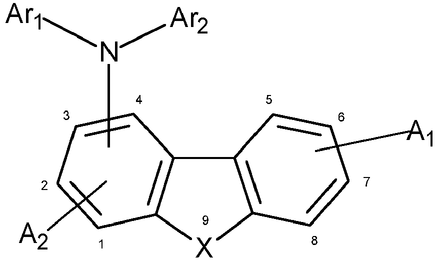 dans laquelle A¹ et A² sont tels que définis pour la formule (I) et Ar¹ et Ar² sont tels que définis dans la formule (II).

La présente description concerne également l'utilisation d'un composé de formule (I-5) pour la préparation de triarylamines de formule (III-5) : 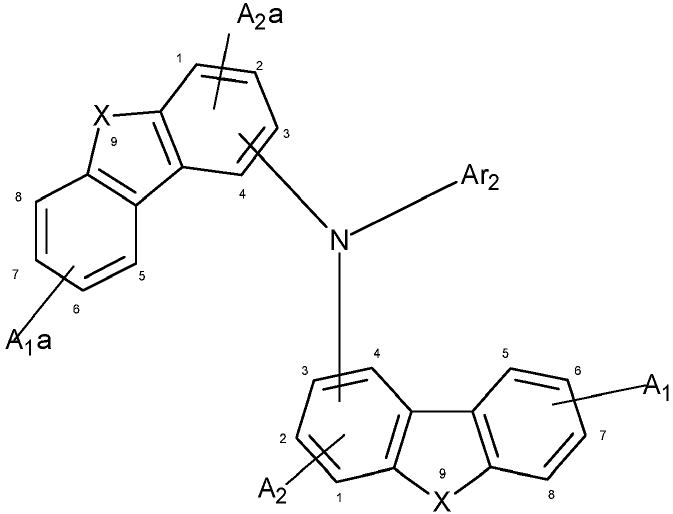 dans laquelle
dans laquelle A¹ et A², A^{2a} et A^{1a} sont tels que définis dans la formule (I) et Ar² est tel que défini dans la formule (II).

La présente invention concerne également l'utilisation d'un composé de formule (I-5) pour la préparation de triarylamines de formule (IV-5) suivante : 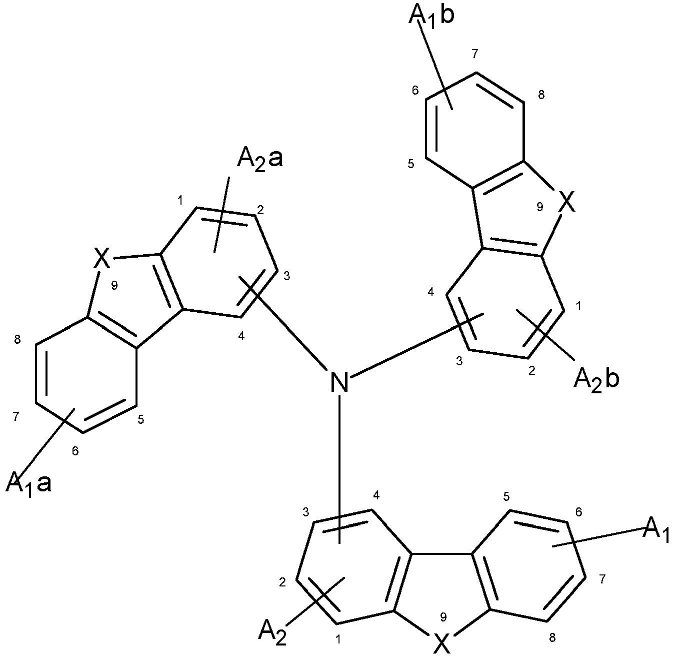 dans laquelle A¹, A², A^{1a}, A^{2a}, A^{1b} et A^{2b} sont tels que définis à la formule (I).

Dans un **troisième aspect**, les composés de formule (I) sont des fluorènes et la présente invention concerne l'utilisation de composés de formule (I-6) : 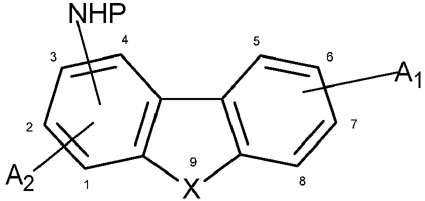 dans laquelle :
**X³** : CH-[(CH₂)ₙ-O)]ₚ-R² ;
**X⁴** : CH-R¹-{O-[(CH₂)ₙ-O)]ₚ₋R2}ₘ ;
**X⁵** : C-{[(CH₂)ₙ-O)]ₚ-R²}₂ ; et
**X⁶** : C-(R¹-{O-[(CH₂)ₙ-O)]ₚ-R²}ₘ)₂ et NHP et Y, A¹ et A² sont tels que définis dans la formule (I)
pour la préparation de composés π-conjugués, en particulier des composés π-conjugués utiles en tant que semi-conducteurs, notamment des semi-conducteurs organiques, ou pour la préparation de colorants.

La présente description concerne donc dans un mode de réalisation l'utilisation d'un composé de formule (I-6) pour la préparation de triarylamines de formule (II-6) : 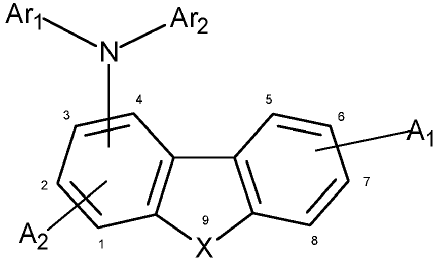 dans laquelle A¹ et A² sont tels que définis pour la formule (I) et Ar¹ et Ar² sont tels que définis dans la formule (II).

La présente description concerne également l'utilisation d'un composé de formule (I-6) pour la préparation de triarylamines de formule (III-6) : 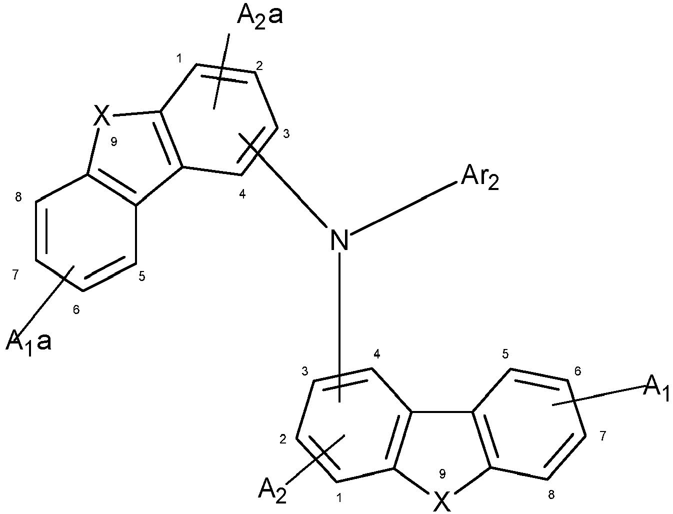 dans laquelle
dans laquelle A¹ et A², A^{2a} et A^{1a} sont tels que définis dans la formule (I) et Ar² est tel que défini dans la formule (II).

La présente invention concerne également l'utilisation d'un composé de formule (I-6) pour la préparation de triarylamines de formule (IV-6) suivante : 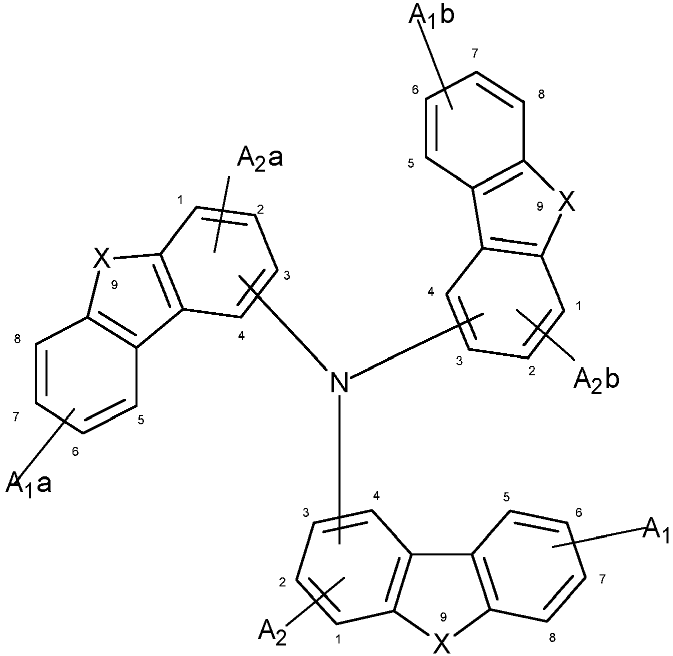 dans laquelle A¹, A², A^{1a}, A^{2a}, A^{1b} et A^{2b} sont tels que définis à la formule (I).

Dans un quatrième aspect, les composés de formule (I) sont des fluorènes et la présente invention concerne l'utilisation de composés de formule (I-7) : 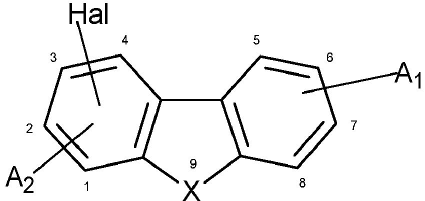 dans laquelle :
**X³** : CH-[(CH₂)ₙ-O)]ₚ-R² ;
**X⁴** : CH-R¹-{O-[(CH₂)ₙ-O)]ₚ₋R2}ₘ ;
**X⁵** : C-{[(CH₂)ₙ-O)]ₚ-R²}₂ ; et
**X⁶** : C-(R¹-{O-[(CH₂)ₙ-O)]ₚ-R²}ₘ)₂
et Hal, A¹ et A² sont tels que définis pour la formule (I).
pour la préparation de composés π-conjugués, en particulier des composés π-conjugués utiles en tant que semi-conducteurs, notamment des semi-conducteurs organiques, ou pour la préparation de colorants.

La présente description concerne donc dans un mode de réalisation l'utilisation d'un composé de formule (I-7) pour la préparation de triarylamines de formule (II-7) : 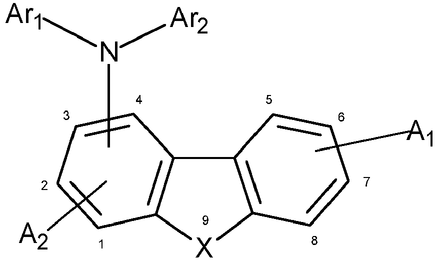 dans laquelle A¹ et A² sont tels que définis pour la formule (I) et Ar¹ et Ar² sont tels que définis dans la formule (II).

La présente description concerne également l'utilisation d'un composé de formule (I-7) pour la préparation de triarylamines de formule (III-7) : 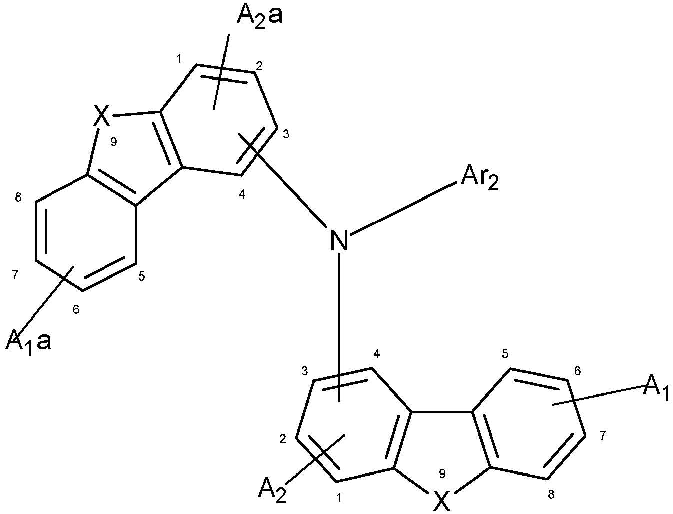 dans laquelle A¹ et A², A^{2a} et A^{1a} sont tels que définis dans la formule (I) et Ar² est tel que défini dans la formule (II).

La présente invention concerne également l'utilisation d'un composé de formule (I-7) pour la préparation de triarylamines de formule (IV-7) suivante : 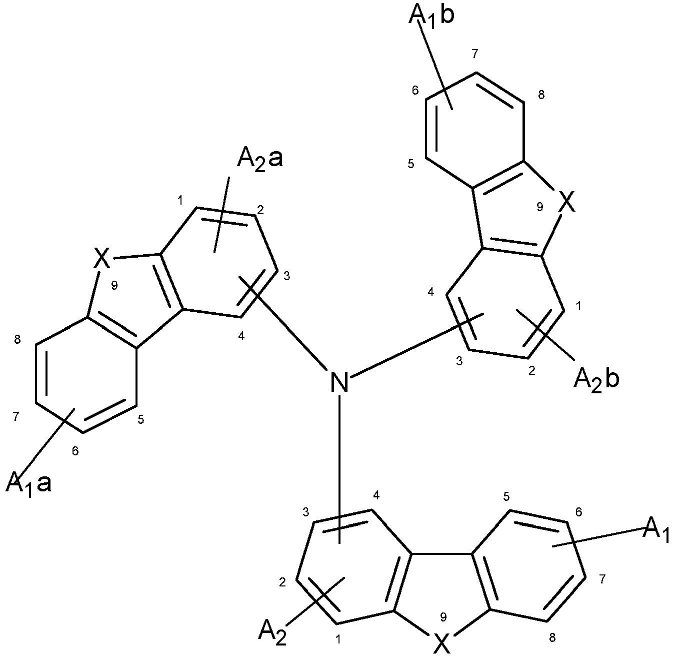 dans laquelle A¹, A², A^{1a}, A^{2a}, A^{1b} et A^{2b} sont tels que définis à la formule (I).

Dans un cinquième aspect, les composés de formule (I) sont des fluorènes et la présente description concerne l'utilisation de composés de formule (I-7) : 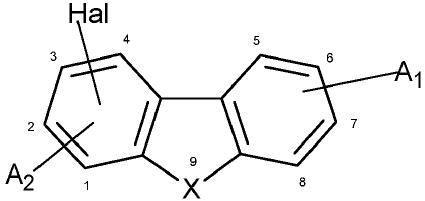 dans laquelle :
**X³** : CH-[(CH₂)ₙ-O)]ₚ-R² ;
**X⁴** : CH-R¹-{O-[(CH₂)ₙ-O)]ₚ₋R2}ₘ ;
**X⁵** : C-{[(CH₂)ₙ-O)]ₚ-R²}₂ ; et
**X⁶** : C-(R¹-{O-[(CH₂)ₙ-O)]ₚ-R²}ₘ)₂
et Hal, A¹ et A² sont tels que définis dans la formule (I) pour la préparation de composés π-conjugués, en particulier des composés π-conjugués utiles en tant que semi-conducteurs, notamment des semi-conducteurs organiques, ou pour la préparation de colorants.
pour la préparation de composés de formule (V-7) : 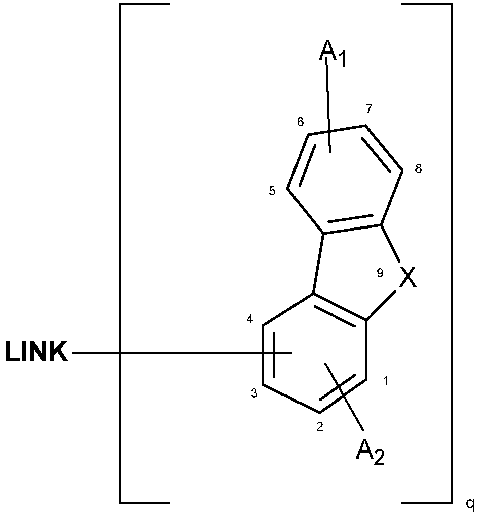 dans laquelle :
   **LINK** est tel que défini ci-dessus.

Dans un **sixième aspect**, les composés de formule (I) sont des carbazoles et la présente description concerne l'utilisation de composés de formule (I-8) : 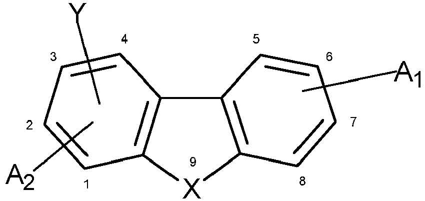 dans laquelle X représente :
**X¹** : N-[(CH₂)ₙ-O)]ₚ-R² ; ou
**X²** : N-R₁-{O-[(CH₂)ₙ-O)]ₚ-R²}ₘ ;
et Y, A¹ et A² sont tels que définis dans la formule (I)
pour la préparation de composés π-conjugués, en particulier des composés π-conjugués utiles en tant que semi-conducteurs, notamment des semi-conducteurs organiques, ou pour la préparation de colorants.

La présente description concerne donc dans un mode de réalisation l'utilisation d'un composé de formule (I-8) pour la préparation de triarylamines de formule (II-8) : 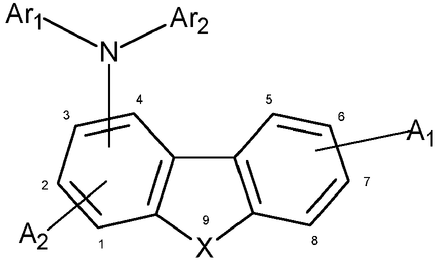 dans laquelle A¹ et A² sont tels que définis pour la formule (I) et Ar¹ et Ar² sont tels que définis dans la formule (II).

La présente description concerne également l'utilisation d'un composé de formule (I-8) pour la préparation de triarylamines de formule (III-8) : 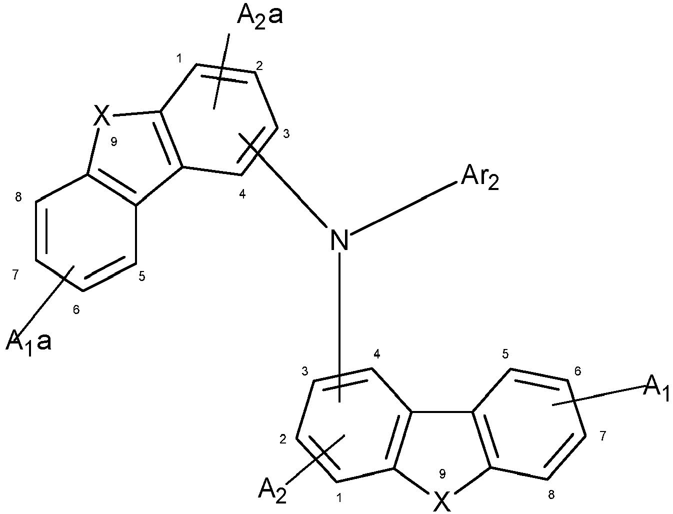 dans laquelle A¹ et A², A^{2a} et A^{1a} sont tels que définis dans la formule (I) et Ar² est tel que défini dans la formule (II).

La présente invention concerne également l'utilisation d'un composé de formule (I-8) pour la préparation de triarylamines de formule (IV-8) suivante : 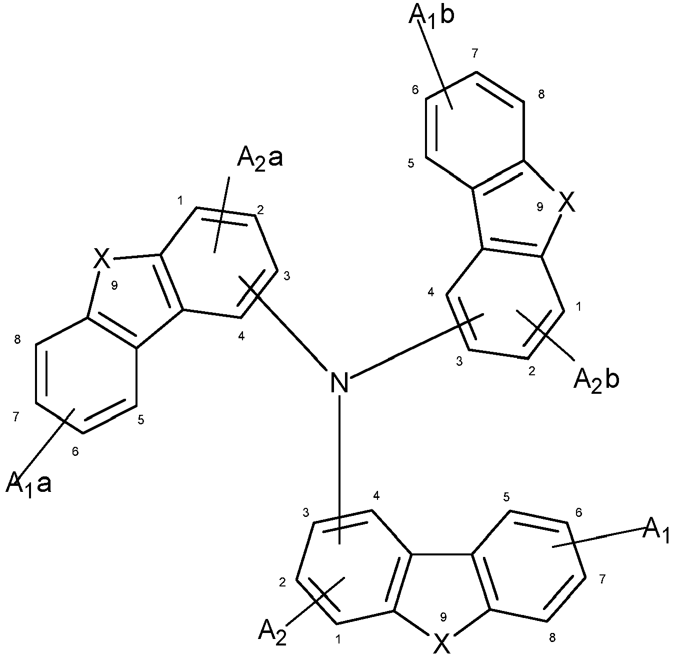 dans laquelle A¹, A², A^{1a}, A^{2a}, A^{1b} et A^{2b} sont tels que définis à la formule (I).

Dans un **septième aspect**, les composés de formule (I) sont des carbazoles et la présente description concerne l'utilisation de composés de formule (I-9) : 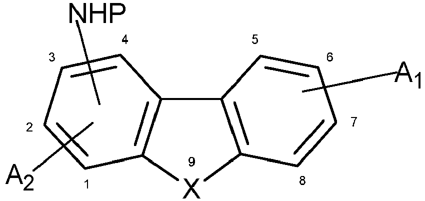 dans laquelle X représente :
**X¹** : N-[(CH₂)ₙ-O)]ₚ-R² ; ou
**X²** : N-R₁-{O-[(CH₂)ₙ-O)]ₚ-R²}ₘ ;
et Hal, A¹ et A² sont tels que définis dans la formule (I)
pour la préparation de composés π-conjugués, en particulier des composés π-conjugués utiles en tant que semi-conducteurs, notamment des semi-conducteurs organiques, ou pour la préparation de colorants.

La présente description concerne donc dans un mode de réalisation l'utilisation d'un composé de formule (I-9) pour la préparation de triarylamines de formule (II-9) : 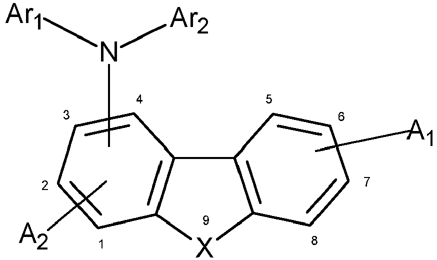 dans laquelle A¹ et A² sont tels que définis pour la formule (I) et Ar¹ et Ar² sont tels que définis dans la formule (II).

La présente description concerne également l'utilisation d'un composé de formule (I-9) pour la préparation de triarylamines de formule (III-9) : 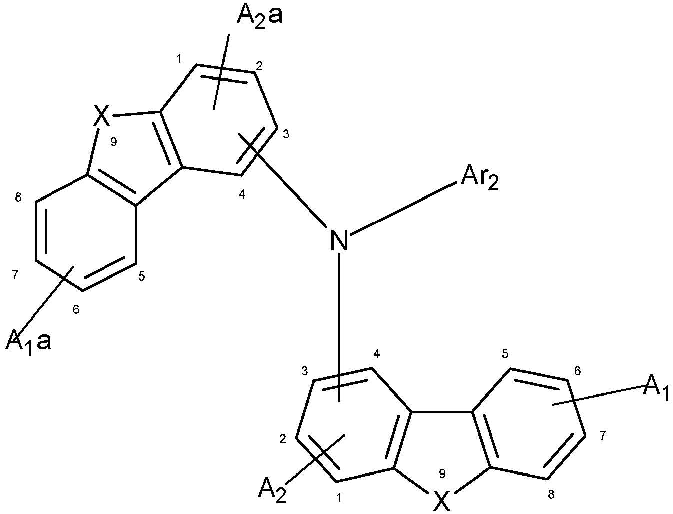 dans laquelle A¹ et A², A^{2a} et A^{1a} sont tels que définis dans la formule (I) et Ar² est tel que défini dans la formule (II).

La présente invention concerne également l'utilisation d'un composé de formule (I-9) pour la préparation de triarylamines de formule (IV-9) suivante : 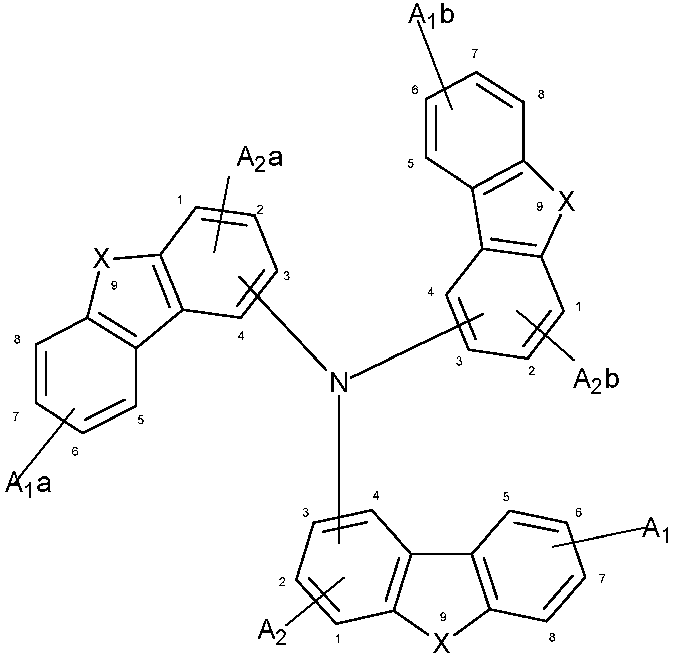 dans laquelle A¹, A², A^{1a}, A^{2a}, A^{1b} et A^{2b} sont tels que définis à la formule (I).

Dans un **huitième aspect**, les composés de formule (I) sont des carbazoles et la présente description concerne l'utilisation de composés de formule (I-10) : 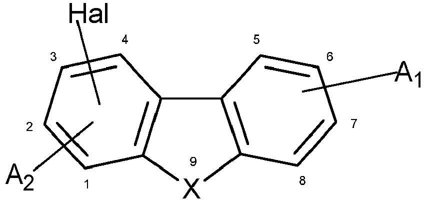 dans laquelle X représente :
**X¹** : N-[(CH₂)ₙ-O)]ₚ-R² ; ou
**X²** : N-R₁-{O-[(CH₂)ₙ-O)]ₚ-R²}ₘ ;
et Hal, A¹ et A² sont tels que définis dans la formule (I),
pour la préparation de composés π-conjugués, en particulier des composés π-conjugués utiles en tant que semi-conducteurs, notamment des semi-conducteurs organiques, ou pour la préparation de colorants.

La présente description concerne donc dans un mode de réalisation l'utilisation d'un composé de formule (I-10) pour la préparation de triarylamines de formule (II-10) : 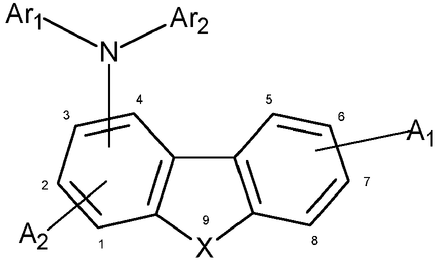 dans laquelle :
dans laquelle X représente X¹ ou X² tels que définis à la formule (I) et A¹ et A², A^{2a} et A^{1a} sont tels que définis dans la formule (I) et Ar¹ et Ar² sont tels que définis dans la formule (II).

La présente description concerne également l'utilisation d'un composé de formule (I-10) pour la préparation de triarylamines de formule (III-10) : 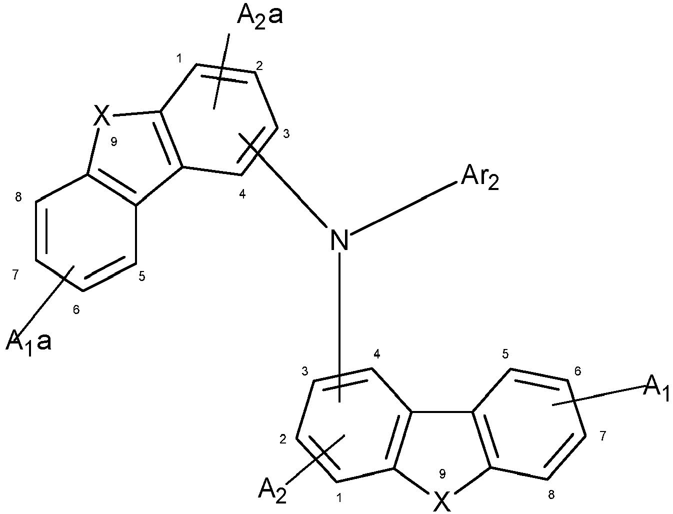 dans laquelle A¹, A², A^{2a} et A^{1a} sont tels que définis dans la formule (I) et Ar² est tel que défini dans la formule (II).

La présente invention concerne également l'utilisation d'un composé de formule (I-10) pour la préparation de triarylamines de formule (IV-10) suivante :

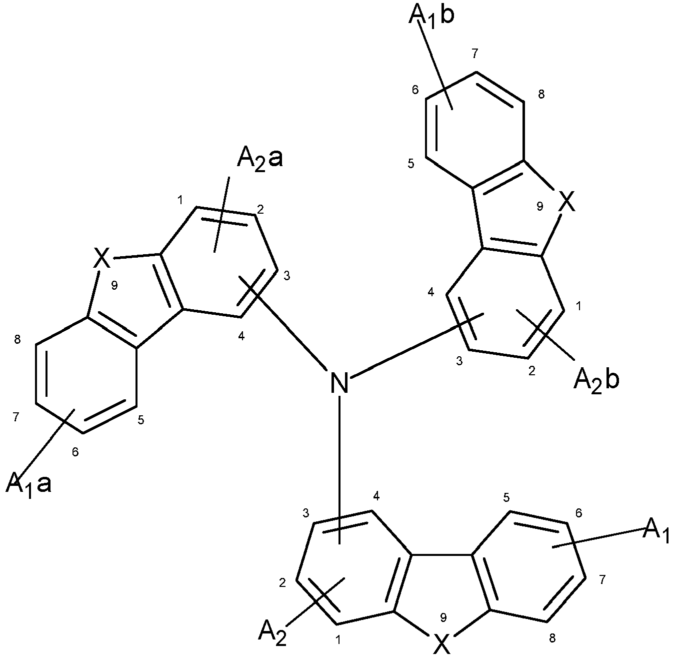

Dans un **neuvième aspect**, les composés de formule (I) sont des carbazoles et la présente description concerne l'utilisation de composés de formule (I-10) : 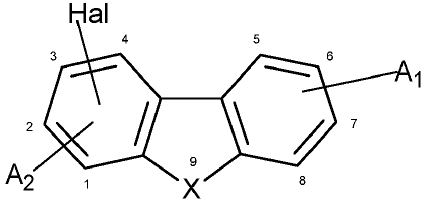 dans laquelle X représente :
**X¹** : N-[(CH₂)ₙ-O)]ₚ-R² ; ou
**X²** : N-R₁-{O-[(CH₂)ₙ-O)]ₚ-R²}ₘ ;
et Hal, A¹ et A² sont tels que définis dans la formule (I),
pour la préparation de composés de formule (V-2) : 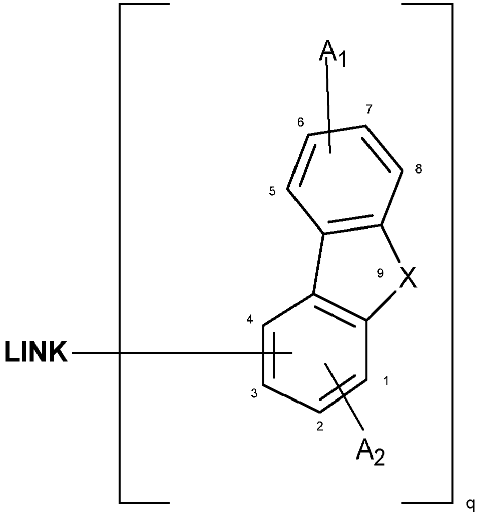 dans laquelle :
   LINK est tel que défini ci-dessus.

La présente description concerne également des composés de formule (I) : 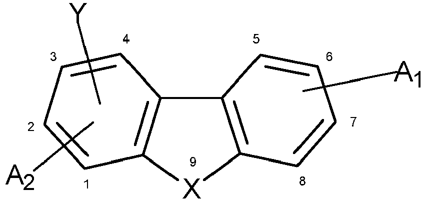 dans laquelle :
X est choisi dans le groupe constitué de :
**X¹** : N-[(CH₂)ₙ-O)]ₚ-R² ;
**X²** : N-R₁-{O-[(CH₂)ₙ-O)]ₚ-R²}ₘ ;
**X³** : CH-[(CH₂)ₙ-O)]ₚ-R² ;
**X⁴** : CH-R¹-{O-[(CH₂)ₙ-O)]ₚ₋R2}ₘ ;
**X⁵** : C-{[(CH₂)ₙ-O)]ₚ-R²}₂ ; et
**X⁶** : C-(R¹-{O-[(CH₂)ₙ-O)]ₚ-R²}ₘ)₂ ; où :
   R¹ est choisi dans le groupe constitué de aryle ; hétéroaryle ; alkyl-aryle ; cycloalkyle en C₃ à C₈ ; hétérocycloalkyle en C₃ à C₈; notamment aryle ou hétéroaryle, de préférence aryle ;
   n est un entier de 1 à 6, notamment 2 ou 3, en particulier 2 ;
   p = est un entier de 1 à 20 ;
   m est un entier de 1 à 6, notamment de 1 à 3, en particulier 1 ;
   et lorsque m est supérieur à 1, les valeurs de p et de n dans chaque groupement -{O-[(CH₂)ₙ-O)]ₚ- peuvent être identiques ou différentes les unes des autres.
   R² est un alkyle en C₁ à C₁₂; aryle ; hétéroaryle ; cycloalkyle en C₃ à C₈ ; hétérocycloalkyle en C₃ à C₈ ;
   A¹, situé de préférence en position 6 ou 7 du cycle, est choisi dans le groupe constitué de H ; alkyle en C₁ à C₂₀ ; alcényle en C₂ à C₂₀ ; alcynyle en C₂ à C₂₀ ; aryle, hétéroaryle ; cycloalkyle en C₃ à C₈ ; NO₂, CF₃ ; CN ; N₃ ; F ; Cl ; NR^{a}R^{b}; OR^{c} ; SR^{c} ; C(=O)R^{d} ;; ou un chromophore ; notamment H ou un chromophore ; où :
      R^{a} est choisi dans le groupe constitué de H ; alkyle en C₁ à C₂₀ ; aryle, hétéroaryle, cycloalkyle en C₃ à C₈ ; ou R^{a} et R^{b} forment ensemble un (C₃-C₇)hétérocyclyle ;
      R^{b} est choisi dans le groupe constitué de alkyle en C₁ à C₂₀ ; aryle, hétéroaryle, cycloalkyle en C₃ à C₈; ou R^{a} et R^{b} forment ensemble un (C₃-C₇)hétérocyclyle ;
      R^{c} est choisi dans le groupe constitué de alkyle en C₁ à C₂₀ ; aryle, hétéroaryle, cycloalkyle en C₃ à C₈; C(=O)-alkyle en C₁ à C₂₀ ; C(=O)-aryle, C(=O)-hétéroaryle, C(=O)-cycloalkyle en C₃ à C₈ ;
      R^{d} représente alkyle en C₁ à C₂₀ ; aryle, hétéroaryle, cycloalkyle en C₃ à C₈ ; NR^{a}R^{b}; OR^{c} ; SR^{c};
      Y est choisi dans le groupe constitué de NHP, Br ; I, B(OR^{e})₂ ; BF₃K ; Sn(Bu)₃ ; OSO₂CF₃ ; OSO₂C₄F₉ ; N₂⁺ ; où :
         Rₑ est choisi dans le groupe constitué de H, alkyle en C₁ à C₆, ou B(OR^{e})₂ est un ester boronique notamment choisi parmi l'ester pinacolique et l'ester glycolique ;
         le groupement Y est en position 2 du cycle lorsque X représente **X³** : CH-[(CH₂)ₙ-O)]ₚ-R² ; **X⁴**: CH-R¹-{O-[(CH₂)ₙ-O)]ₚ-R²}ₘ ; **X⁵** : C-{[(CH₂)ₙ-O)]ₚ-R²}₂ ; ou **X⁶** : C-(R¹-{O-[(CH₂)ₙ-O)]ₚ-R²}ₘ)₂ ; ou
         le groupement Y est en position 2 ou 3, de préférence en position 3, du cycle lorsque X représente N-[(CH₂)ₙ-O)]ₚ-R² ou N-R₁-{O-[(CH₂)-O)]ₚ₋R²}ₘ ;
         P représente H ou un groupement protecteur d'amine, notamment choisi dans le groupe constitué des benzyles, des carbamates, des amides et des imides cycliques ; notamment H ;
         A², situé de préférence en position 2 ou 3 du cycle est choisi dans le groupe constitué de H ; alkyle en C₁ à C₂₀ ; alcényle en C₂ à C₂₀ ; alcynyle en C₂ à C₂₀ ; aryle, hétéroaryle ; cycloalkyle en C₃ à C₈ ; NO₂, CF₃ ; CN ; N₃ ; F ; Cl ; NR^{a}R^{b}; OR^{c} ; SR^{c} ; C(=O)R^{d} ; ou un chromophore ; notamment H ; où :
            R^{a} est choisi dans le groupe constitué de H ; alkyle en C₁ à C₂₀ ; aryle, hétéroaryle, cycloalkyle en C₃ à C₈ ; ou R^{a} et R^{b} forment ensemble un (C₃-C₇)hétérocyclyle ;
            R^{b} est choisi dans le groupe constitué de alkyle en C₁ à C₂₀ ; aryle, hétéroaryle, cycloalkyle en C₃ à C₈; ou R^{a} et R^{b} forment ensemble un (C₃-C₇)hétérocyclyle ;
            R^{c} est choisi dans le groupe constitué de alkyle en C₁ à C₂₀ ; aryle, hétéroaryle, cycloalkyle en C₃ à C₈; C(=O)-alkyle en C₁ à C₂₀ ; C(=O)-aryle, C(=O)-hétéroaryle, C(=O)-cycloalkyle en C₃ à C₈ ;
            R^{d} représente alkyle en C₁ à C₂₀ ; aryle, hétéroaryle, cycloalkyle en C₃ à C₈ ; NR^{a}R^{b}; OR^{c} ; SR^{c};
            à l'exception des composés suivants :

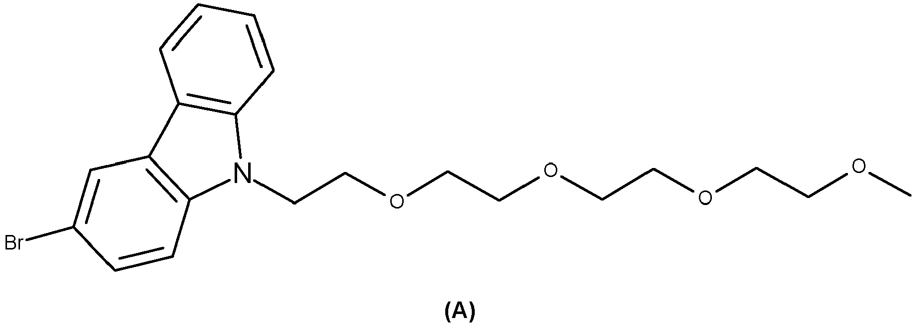

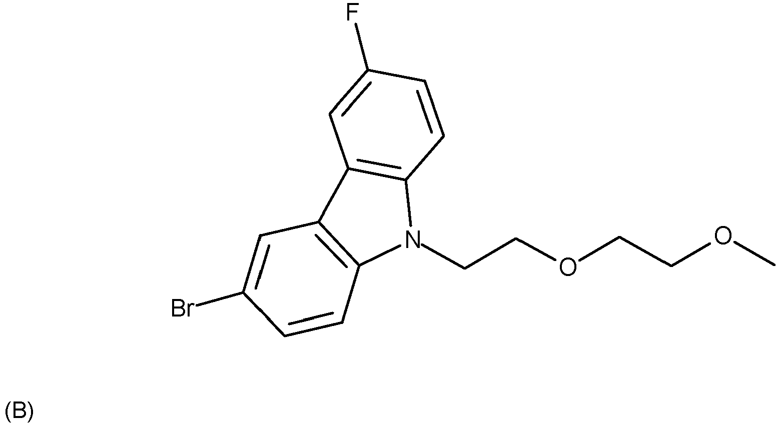

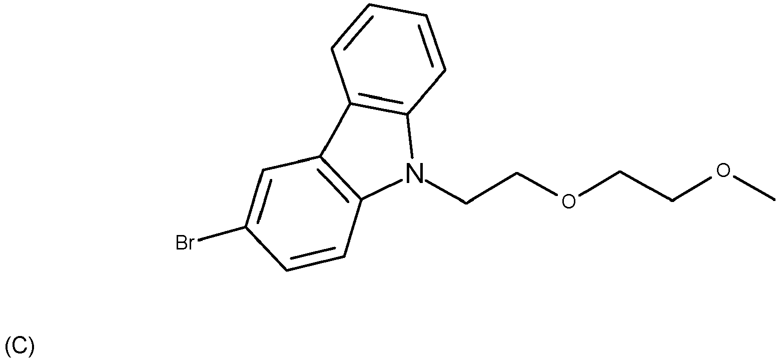

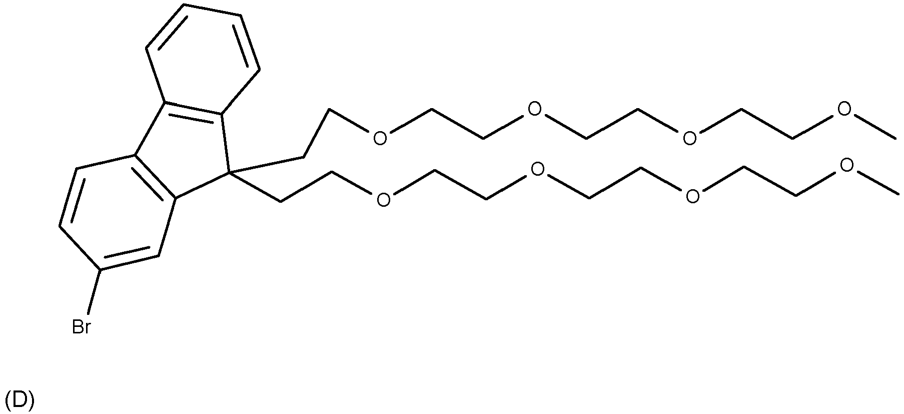

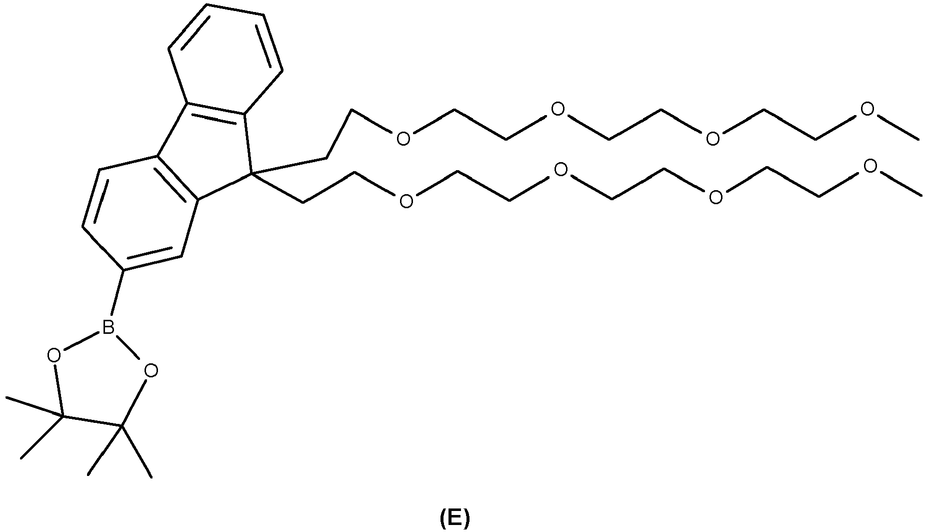

Dans un premier mode de réalisation, la présente description concerne un composé de formule (la) : 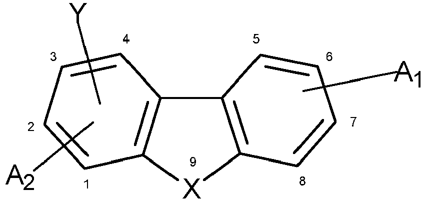 dans laquelle :
X est choisi dans le groupe constitué de :
**X³** : CH-[(CH₂)ₙ-O)]ₚ-R² ;
**X⁴** : CH-R¹-{O-[(CH₂)ₙ-O)]ₚ₋R2}ₘ ;
**X⁵** : C-{[(CH₂)ₙ-O)]ₚ-R²}₂ ; et
**X⁶** : C-(R¹-{O-[(CH₂)ₙ-O)]ₚ-R²}ₘ)₂ ; et
Y est NHP.

Dans un autre mode de réalisation, la présente description concerne un composé de formule (I), dans laquelle :
X est choisi dans le groupe constitué de :
**X³** : CH-[(CH₂)ₙ-O)]ₚ-R² ;
**X⁴** : CH-R¹-{O-[(CH₂)ₙ-O)]ₚ₋R2}ₘ ;
**X⁵** : C-{[(CH₂)ₙ-O)]ₚ-R²}₂ ; et
**X⁶** : C-(R¹-{O-[(CH₂)ₙ-O)]ₚ-R²}ₘ)₂ ; et
Y est Hal et est choisi dans le groupe constitué de Br ; I, B(OR^{e})₂ ; BF₃K; Sn(Bu)₃ ; OSO₂CF₃ ; OSO₂C₄F₉ ; N₂⁺.
à l'exception des composés (D) et (E) ci-dessus.

Dans un encore autre mode de réalisation, la présente description concerne un composé de formule (I), dans laquelle :
X est choisi dans le groupe constitué de :
**X¹** : N-[(CH₂)ₙ-O)]ₚ-R² ;
**X²** : N-R₁-{O-[(CH₂)ₙ-O)]ₚ-R²}ₘ ;
Y est NHP.

Dans un encore autre mode de réalisation, la présente description concerne un composé de formule (I), dans laquelle :
X est choisi dans le groupe constitué de :
**X¹** : N-[(CH₂)ₙ-O)]ₚ-R² ;
**X²** : N-R₁-{O-[(CH₂)ₙ-O)]ₚ-R²}ₘ ;
Y est choisi dans le groupe constitué de Br ; I, B(OR^{e})₂ ; BF₃K; Sn(Bu)₃; OSO₂CF₃ ; OSO₂C₄F₉ ; N₂⁺.
à l'exception des composés **(A)**, **(B)** et **(C)** ci-dessus.

La présente description concerne également les produits susceptibles d'être obtenus à partir des composés de formule (I) tels que définis ci-dessus.

La présente description concerne donc les composés de formule (II) : 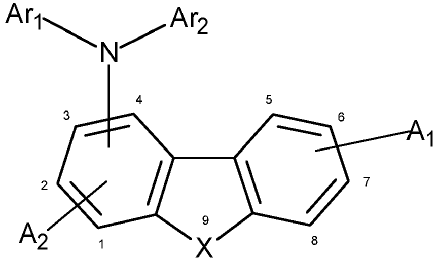 dans laquelle :
X est choisi dans le groupe constitué de :
**X¹** : N-[(CH₂)ₙ-O)]ₚ-R² ;
**X²** : N-R₁-{O-[(CH₂)ₙ-O)]ₚ-R²}ₘ ;
**X³** : CH-[(CH₂)ₙ-O)]ₚ-R² ;
**X⁴** : CH-R¹-{O-[(CH₂)ₙ-O)]ₚ₋R2}ₘ ;
**X⁵** : C-{[(CH₂)ₙ-O)]ₚ-R²}₂ ; et
**X⁶** : C-(R¹-{O-[(CH₂)ₙ-O)]ₚ-R²}ₘ)₂ ; où :
   R¹ est choisi dans le groupe constitué de aryle ; hétéroaryle ; alkyl-aryle ; cycloalkyle en C₃ à C₈ ; hétérocycloalkyle en C₃ à C₈; notamment aryle ou hétéroaryle, de préférence aryle ;
   n est un entier de 1 à 6, notamment 2 ou 3, en particulier 2 ;
   p = est un entier de 1 à 20 ;
   m est un entier de 1 à 6, notamment de 1 à 3, en particulier 1 ;
   et lorsque m est supérieur à 1, les valeurs de p et de n dans chaque groupement -{O-[(CH₂)ₙ-O)]ₚ- peuvent être identiques ou différentes les unes des autres.
   R² est un alkyle en C₁ à C₁₂; aryle ; hétéroaryle ; cycloalkyle en C₃ à C₈ ; hétérocycloalkyle en C₃ à C₈ ;
   A¹, situé de préférence en position 6 ou 7 du cycle, est choisi dans le groupe constitué de H ; alkyle en C₁ à C₂₀ ; alcényle en C₂ à C₂₀ ; alcynyle en C₂ à C₂₀ ; aryle, hétéroaryle ; cycloalkyle en C₃ à C₈ ; NO₂, CF₃ ; CN ; N₃ ; F ; Cl ; NR^{a}R^{b}; OR^{c} ; SR^{c} ; C(=O)R^{d} ;; ou un chromophore ; notamment H ou un chromophore ; où :
      R^{a} est choisi dans le groupe constitué de H ; alkyle en C₁ à C₂₀ ; aryle, hétéroaryle, cycloalkyle en C₃ à C₈ ; ou R^{a} et R^{b} forment ensemble un (C₃-C₇)hétérocyclyle ;
      R^{b} est choisi dans le groupe constitué de alkyle en C₁ à C₂₀ ; aryle, hétéroaryle, cycloalkyle en C₃ à C₈; ou R^{a} et R^{b} forment ensemble un (C₃-C₇)hétérocyclyle ;
      R^{c} est choisi dans le groupe constitué de alkyle en C₁ à C₂₀ ; aryle, hétéroaryle, cycloalkyle en C₃ à C₈; C(=O)-alkyle en C₁ à C₂₀ ; C(=O)-aryle, C(=O)-hétéroaryle, C(=O)-cycloalkyle en C₃ à C₈ ;
      R^{d} représente alkyle en C₁ à C₂₀ ; aryle, hétéroaryle, cycloalkyle en C₃ à C₈ ; NR^{a}R^{b}; OR^{c} ; SR^{c};
      le groupement Y est en position 2 du cycle lorsque X représente **X³** : CH-[(CH₂)ₙ-O)]ₚ-R² ; **X⁴**: CH-R¹-{O-[(CH₂)ₙ-O)]ₚ-R²}ₘ ; **X⁵** : C-{[(CH₂)ₙ-O)]ₚ-R²}₂ ; ou **X⁶** : C-(R¹-{O-[(CH₂)ₙ-O)]ₚ-R²}ₘ)₂ ; ou
      le groupement Y est en position 2 ou 3, de préférence en position 3, du cycle lorsque X représente N-[(CH₂)ₙ-O)]ₚ-R² ou N-R₁-{O-[(CH₂)-O)]ₚ₋R²}ₘ ;
      A², situé de préférence en position 2 ou 3 du cycle est choisi dans le groupe constitué de H ; alkyle en C₁ à C₂₀ ; alcényle en C₂ à C₂₀ ; alcynyle en C₂ à C₂₀ ; aryle, hétéroaryle ; cycloalkyle en C₃ à C₈ ; NO₂, CF₃ ; CN ; N₃ ; F ; Cl ; NR^{a}R^{b}; OR^{c} ; SR^{c} ; C(=O)R^{d} ; ou un chromophore ; notamment H ; où :
         R^{a} est choisi dans le groupe constitué de H ; alkyle en C₁ à C₂₀ ; aryle, hétéroaryle, cycloalkyle en C₃ à C₈ ; ou R^{a} et R^{b} forment ensemble un (C₃-C₇)hétérocyclyle ;
         R^{b} est choisi dans le groupe constitué de alkyle en C₁ à C₂₀ ; aryle, hétéroaryle, cycloalkyle en C₃ à C₈; ou R^{a} et R^{b} forment ensemble un (C₃-C₇)hétérocyclyle ;
         R^{c} est choisi dans le groupe constitué de alkyle en C₁ à C₂₀ ; aryle, hétéroaryle, cycloalkyle en C₃ à C₈; C(=O)-alkyle en C₁ à C₂₀ ; C(=O)-aryle, C(=O)-hétéroaryle, C(=O)-cycloalkyle en C₃ à C₈ ;
         R^{d} représente alkyle en C₁ à C₂₀ ; aryle, hétéroaryle, cycloalkyle en C₃ à C₈ ; NR^{a}R^{b}; OR^{c} ; SR^{c},
         Ar¹ et Ar², identiques ou différents, sont un aryle ou un hétéroaryle, notamment choisi parmi phényle, biphényle, naphtyle, anthracényle, pyrényle.

La présente description concerne également des produits de formule (III) : 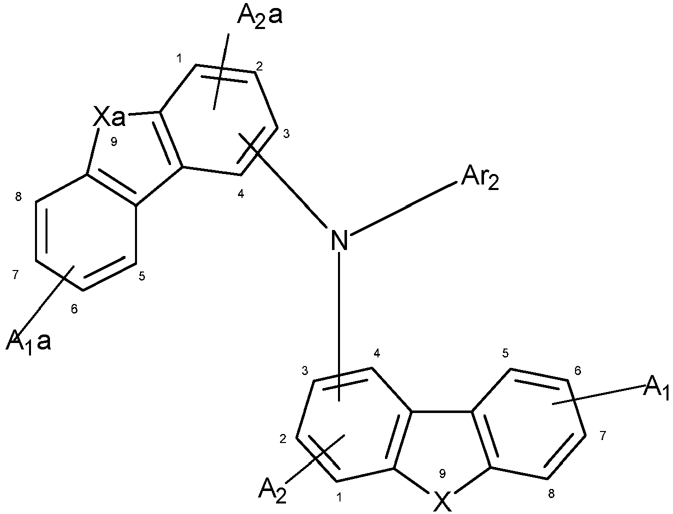 dans laquelle A^{2a} et A^{1a} ont la signification donnée pour A¹ et A² dans la revendication 1, A^{2a} et A² d'une part et A^{1a} et A¹ d'autre part pouvant être identiques ou différents l'un de l'autre et Xa et X, identiques ou différents l'un de l'autre, sont tels que définis dans la formule (I).

La présente description concerne plus particulièrement des produits de formule (III-1) : 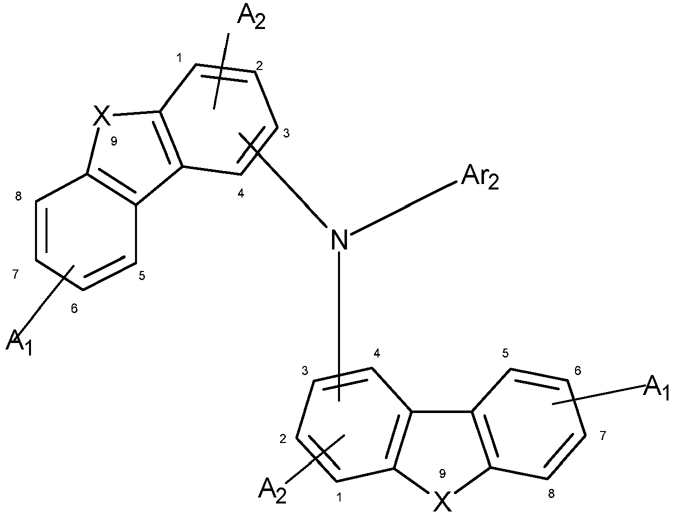 dans laquelle X, A¹ et A² sont tels que définis dans la revendication 1.

La présente invention concerne un triarylamine de formule (IV) : 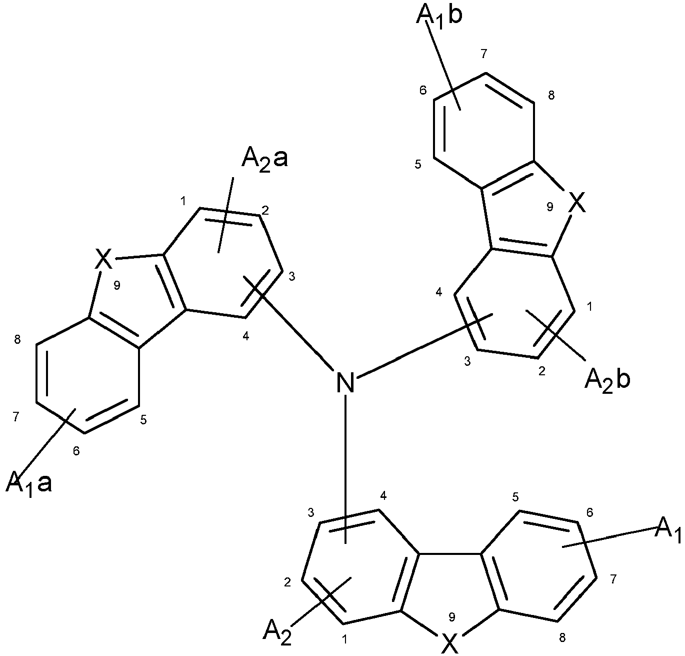 dans laquelle :
X est choisi dans le groupe constitué de :
**X¹** : N-[(CH₂)ₙ-O)]ₚ-R² ;
**X²** : N-R₁-{O-[(CH₂)ₙ-O)]ₚ-R²}ₘ ;
**X³** : CH-[(CH₂)ₙ-O)]ₚ-R² ;
**X⁴** : CH-R¹-{O-[(CH₂)ₙ-O)]ₚ-R²}ₘ ;
**X⁵** : C-{[(CH₂)ₙ-O)]ₚ-R²}₂ ; et
**X⁶** : C-**(**R¹-{O-[(CH₂)ₙ-O)]ₚ-R²}ₘ)₂ ; où :
   R¹ est choisi dans le groupe constitué d'aryle ou hétéroaryle, de préférence aryle ;
   n est un entier de 1 à 6, notamment 2 ou 3, en particulier 2 ;
   p = est un entier de 1 à 20;
   m est un entier de 1 à 6, notamment de 1 à 3, en particulier 1 ;
   et lorsque m est supérieur à 1, les valeurs de p et de n dans chaque groupement -{O-[(CH₂)ₙ-O)]ₚ- peuvent être identiques ou différentes les unes des autres.
   R² est un alkyle en C₁ à C₁₂ ; A¹, situé de préférence en position 6 ou 7 du cycle, est choisi dans le groupe constitué de H ;alkyle en C₁ à C₇ ; alcényle en C₂ à C₇ ; alcynyle en C₂ à C₇ ; aryle, hétéroaryle ; cycloalkyle en C₃ à C₈ ; NO₂, CF₃ ; CN ; N₃ ; F ; Cl ; NR^{a}R^{b} ; OR^{c} ; SR^{c} ; C(=O)R^{d} ; ou un chromophore ; notamment H ou un chromophore ; où :
      R^{a} est choisi dans le groupe constitué de H ; alkyle en C₁ à C₇ aryle, hétéroaryle, cycloalkyle en C₃ à C₈ ; ou R^{a} et R^{b} forment ensemble un (C₃-C₇)hétérocyclyle ;
      R^{b} est choisi dans le groupe constitué de alkyle en C₁ à C₇ ; aryle, hétéroaryle, cycloalkyle en C₃ à C₈ ; ou R^{a} et R^{b} forment ensemble un (C₃-C₇)hétérocyclyle ;
      R^{c} est choisi dans le groupe constitué de alkyle en C₁ à C₇ ; aryle, hétéroaryle, cycloalkyle en C₃ à C₈; C(=O)-alkyle en C₁ à C₇ ; C(=O)-aryle, C(=O)-hétéroaryle, C(=O)-cycloalkyle en C₃ à C₈ ;
      R^{d} représente alkyle en C₁ à C₇; aryle, hétéroaryle, cycloalkyle en C₃ à C₈ ; NR^{a}R^{b}; OR^{c} ; SR^{c};
      A², situé de préférence en position 2 ou 3 du cycle est choisi dans le groupe constitué de H ; alkyle en C₁ à C₇ ; alcényle en C₂ à C₇ ; alcynyle en C₂ à C₇ ; aryle, hétéroaryle ; cycloalkyle en C₃ à C₈ ; NO₂, CF₃ ; CN ; N₃ ; F ; Cl ; NR^{a}R^{b} ; OR^{c} ; SR^{c} ; C(=O)R^{d} ; ou un chromophore ; notamment H ; où :
         R^{a} est choisi dans le groupe constitué de H ; alkyle en C₁ à C₇; aryle, hétéroaryle, cycloalkyle en C₃ à C₈ ; ou R^{a} et R^{b} forment ensemble un (C₃-C₇)hétérocyclyle ;
         R^{b} est choisi dans le groupe constitué de alkyle en C₁ à C₇ ; aryle, hétéroaryle, cycloalkyle en C₃ à C₈ ; ou R^{a} et R^{b} forment ensemble un (C₃-C₇)hétérocyclyle ;
         R^{c} est choisi dans le groupe constitué de alkyle en C₁ à C₇ ; aryle, hétéroaryle, cycloalkyle en C₃ à C₈ ; C(=O)-alkyle en C₁ à C₇ ; C(=O)-aryle, C(=O)-hétéroaryle, C(=O)-cycloalkyle en C₃ à C₈ ;
         R^{d} représente alkyle en C₁ à C₇ ; aryle, hétéroaryle, cycloalkyle en C₃ à C₈ ; NR^{a}R^{b} ; OR^{c} ; SR^{c}.
         et A^{1a}, A^{2a}, A^{1b} et A^{2b} ont la signification donnée pour A¹ et A²,
         A^{2a}, A^{2b} et A² d'une part et A^{1a}, A¹ et A^{1b} d'autre part pouvant être identiques ou différents l'un de l'autre.

Dans un autre mode de réalisation, l'invention concerne une triarylamine de formule (IV) selon l'invention telle que définie ci-dessus, dans laquelle A¹, A^{1a} et A^{1b} représentent H, un aryle ou un chromophore, notamment choisi dans le groupe constitué des structures suivantes : 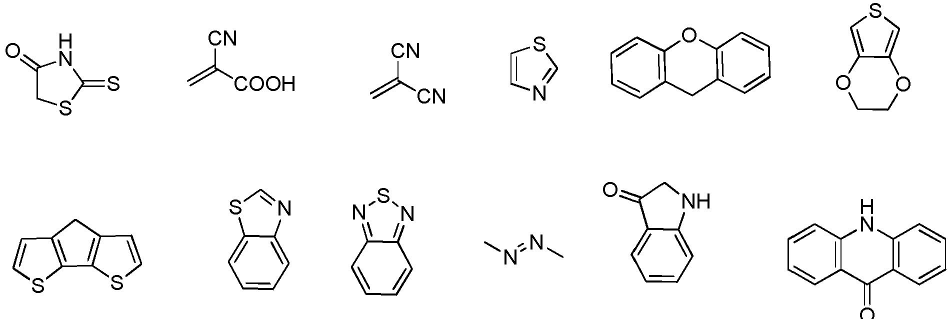 A¹, A^{1a} et A^{1b} représentant notamment H.

Dans un autre mode de réalisation, la présente invention concerne un composé de formule (IV) : 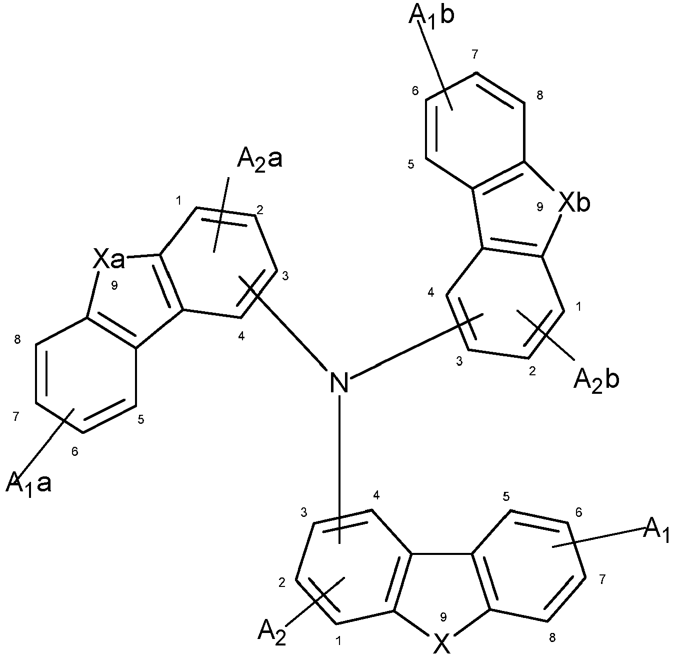 dans laquelle A^{1a}, A^{2a}, A^{1b} et A^{2b} ont la signification donnée pour A¹ et A² à la revendication 1, A^{2a}, A^{2b} et A² d'une part et A^{1a}, A¹ et A^{1b} d'autre part pouvant être identiques ou différents l'un de l'autre et X, X^{a} et X^{b}, identiques ou différents l'un de l'autre, ont la signification donnée ci-dessus.

La présente invention concerne également les composés de formule (IV-1) : 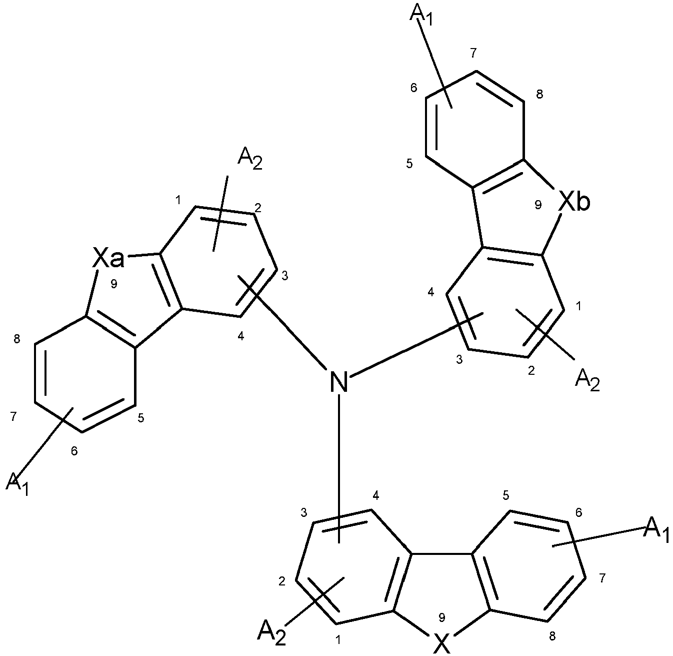 dans laquelle A1, A2 et X, X^{a} et X^{b} sont tels que définis ci-dessus.

Les composés de formule (IV-1) peuvent notamment être les composés suivants : 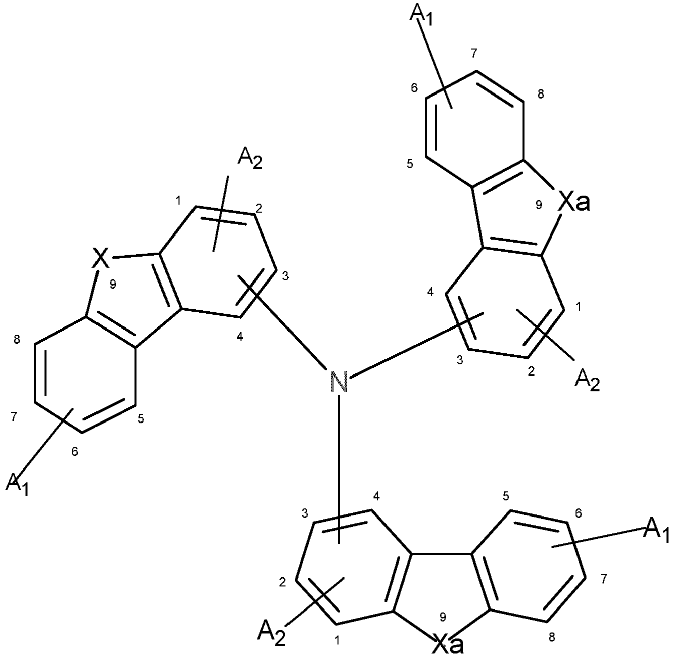
dans laquelle X représente X¹ ou X² tel que défini ci-dessus et Xa, identiques, représentent X³, X⁴, X⁵ ou X⁶ ; ou
dans laquelle X représente X³, X⁴, X⁵ ou X⁶ tel que défini ci-dessus et Xa, identiques, représentent X¹ ou X².

Les composés de formule (IV-1) peuvent notamment également être choisis parmi les composés suivants : 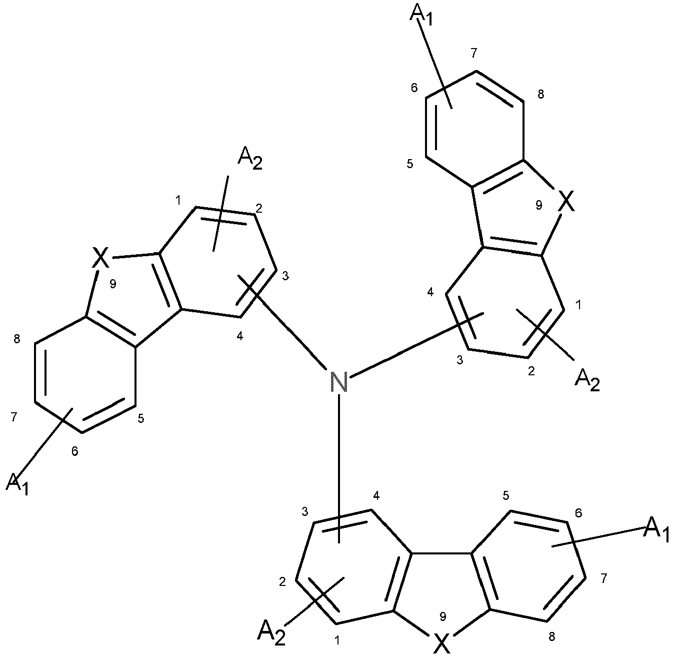 dans laquelle :
X représente X¹ ou X² tel que défini ci-dessus ; ou
X représente X³, X⁴, X⁵ ou X⁶ tel que défini ci-dessus.

La présente invention concerne donc particulièrement des composés de formule (VI) suivante : 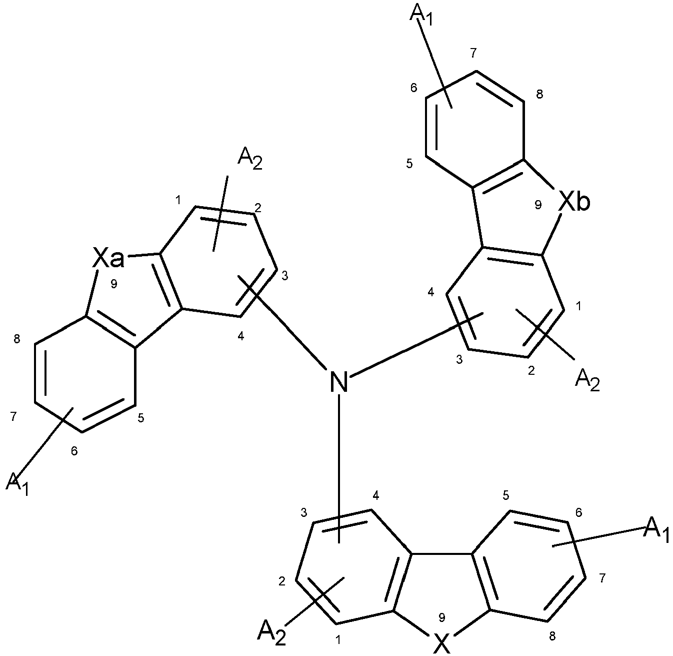 choisis parmi les composés de formule (VI) tels que définis dans le tableau 1 ci-dessous où A¹ est tel que défini ci-dessus, et est de préférence pour chaque entrée, choisi dans le groupe constitué de H, chromophore ou aryle, notamment H ou aryle, de préférence H et A² est tel que défini ci-dessus, de préférence H.

**Tableau 1 :**

| Entrée : | X | Xa | Xb |
|---|---|---|---|
| 1 | **X¹** : N-[(CH₂)ₙ-O)]ₚ-R² | **X¹** : N-[(CH₂)ₙ-O)]ₚ-R² | **X¹** : N-[(CH₂)ₙ-O)]ₚ-R² |
| 2 | **X¹** : N-[(CH₂)ₙ-O)]ₚ-R² | **X²** : N-R₁-{O-[(CH₂)ₙ-O)]ₚ-R²}ₘ ; | **X²** : N-R₁-{O-[(CH₂)ₙ-O)]ₚ-R²}ₘ |
| 3 | **X¹** : N-[(CH₂)ₙ-O)]ₚ-R² | **X³** : CH-[(CH₂)ₙ-O)]ₚ-R² | **X³** : CH-[(CH₂)ₙ-O)]ₚ-R² |
| 4 | **X¹** : N-[(CH₂)ₙ-O)]ₚ-R² | **X⁴** : CH-R¹-{O-[(CH₂)ₙ-O)]ₚ-R²}ₘ | **X⁴** : CH-R¹-{O-[(CH₂)ₙ-O)]ₚ-R²}ₘ |
| 5 | **X¹** : N-[(CH₂)ₙ-O)]ₚ-R² | **X⁵** : C-{[(CH₂)ₙ-O)]ₚ-R²}₂ | **X⁵** : C-{[(CH₂)ₙ-O)]ₚ-R²}₂ |
| 6 | **X¹** : N-[(CH₂)ₙ-O)]ₚ-R² | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| 7 | **X²** : N-R₁-{O-[(CH₂)ₙ-O)]ₚ-R²}ₘ | **X²** : N-R₁-{O-[(CH₂)ₙ-O)]ₚ-R²}ₘ | **X²** : N-R₁-{O-[(CH₂)ₙ-O)]ₚ-R²}ₘ |
| 8 | **X²** : N-R₁-{O-[(CH₂)ₙ-O)]ₚ-R²}ₘ | **X¹** : N-[(CH₂)ₙ-O)]ₚ-R² | **X¹** : N-[(CH₂)ₙ-O)]ₚ-R² |
| 9 | **X²** : N-R₁-{O-[(CH₂)ₙ-O)]ₚ-R²}ₘ | **X³** : CH-[(CH₂)ₙ-O)]ₚ-R² | **X³** : CH-[(CH₂)ₙ-O)]ₚ-R² |
| 10 | **X²** : N-R₁-{O-[(CH₂)ₙ-O)]ₚ-R²}ₘ | **X⁴** : CH-R¹-{O-[(CH₂)ₙ-O)]ₚ-R²}ₘ | **X⁴** : CH-R¹-{O-[(CH₂)ₙ-O)]ₚ-R²}ₘ |
| 11 | **X²** : N-R₁-{O-[(CH₂)ₙ-O)]ₚ-R²}ₘ | **X⁵** : C-{[(CH₂)ₙ-O)]ₚ-R²}₂ | **X⁵** : C-{[(CH₂)ₙ-O)]ₚ-R²}₂ |
| 12 | **X²** : N-R₁-{O-[(CH₂)ₙ-O)]ₚ-R²}ₘ | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| 13 | **X³** : CH-[(CH₂)ₙ-O)]ₚ-R² | **X³** : CH-[(CH₂)ₙ-O)]ₚ-R² | **X³** : CH-[(CH₂)ₙ-O)]ₚ-R² |
| 14 | **X³** : CH-[(CH₂)ₙ-O)]ₚ-R² | **X¹** : N-[(CH₂)ₙ-O)]ₚ-R² | **X¹** : N-[(CH₂)ₙ-O)]ₚ-R² |
| 15 | **X³** : CH-[(CH₂)ₙ-O)]ₚ-R² | **X²** : N-R₁-{O-[(CH₂)ₙ-O)]ₚ-R²}ₘ ; | **X²** : N-R₁-{O-[(CH₂)ₙ-O)]ₚ-R²}ₘ |
| 16 | **X³** : CH-[(CH₂)ₙ-O)]ₚ-R² | **X⁴** : CH-R¹-{O-[(CH₂)ₙ-O)]ₚ-R²}ₘ | **X⁴** : CH-R¹-{O-[(CH₂)ₙ-O)]ₚ-R²}ₘ |
| 17 | **X³** : CH-[(CH₂)ₙ-O)]ₚ-R² | **X⁵** : C-{[(CH₂)ₙ-O)]ₚ-R²}₂ | **X⁵** : C-{[(CH₂)ₙ-O)]ₚ-R²}₂ |
| 18 | **X³** : CH-[(CH₂)ₙ-O)]ₚ-R² | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| 19 | **X⁴** : CH-R¹-{O-[(CH₂)ₙ-O)]ₚ-R²}ₘ | **X⁴** : CH-R¹-{O-[(CH₂)ₙ-O)]ₚ-R²}ₘ | **X⁴** : CH-R¹-{O-[(CH₂)ₙ-O)]ₚ-R²}ₘ |
| 20 | **X⁴** : CH-R¹-{O-[(CH₂)ₙ-O)]ₚ-R²}ₘ | **X¹** : N-[(CH₂)ₙ-O)]ₚ-R² | **X¹** : N-[(CH₂)ₙ-O)]ₚ-R² |
| 21 | **X⁴** : CH-R¹-{O-[(CH₂)ₙ-O)]ₚ-R²}ₘ | **X²** : N-R₁-{O-[(CH₂)ₙ-O)]ₚ-R²}ₘ ; | **X²** : N-R₁-{O-[(CH₂)ₙ-O)]ₚ-R²}ₘ |
| 22 | **X⁴** : CH-R¹-{O-[(CH₂)ₙ-O)]ₚ-R²}ₘ | **X³** : CH-[(CH₂)ₙ-O)]ₚ-R² | **X³** : CH-[(CH₂)ₙ-O)]ₚ-R² |
| 23 | **X⁴** : CH-R¹-{O-[(CH₂)ₙ-O)]ₚ-R²}ₘ | **X⁵** : C-{[(CH₂)ₙ-O)]ₚ-R²}₂ | **X⁵** : C-{[(CH₂)ₙ-O)]ₚ-R²}₂ |
| 24 | **X⁴** : CH-R¹-{O-[(CH₂)ₙ-O)]ₚ-R²}ₘ | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| 25 | **X⁵** : C-{[(CH₂)ₙ-O)]ₚ-R²}₂ | **X⁵** : C-{[(CH₂)ₙ-O)]ₚ-R²}₂ | **X⁵** : C-{[(CH₂)ₙ-O)]ₚ-R²}₂ |
| 26 | **X⁵** : C-{[(CH₂)ₙ-O)]ₚ-R²}₂ | **X¹** : N-[(CH₂)ₙ-O)]ₚ-R² | **X¹** : N-[(CH₂)ₙ-O)]ₚ-R² |
| 27 | **X⁵** : C-{[(CH₂)ₙ-O)]ₚ-R²}₂ | **X²** : N-R₁-{O-[(CH₂)ₙ-O)]ₚ-R²}ₘ ; | **X²** : N-R₁-{O-[(CH₂)ₙ-O)]ₚ-R²}ₘ |
| 28 | **X⁵** : C-{[(CH₂)ₙ-O)]ₚ-R²}₂ | **X³** : CH-[(CH₂)ₙ-O)]ₚ-R² | **X³** : CH-[(CH₂)ₙ-O)]ₚ-R² |
| 29 | **X⁵** : C-{[(CH₂)ₙ-O)]ₚ-R²}₂ | **X⁴** : CH-R¹-{O-[(CH₂)ₙ-O)]ₚ-R²}ₘ | **X⁴** : CH-R¹-{O-[(CH₂)ₙ-O)]ₚ-R²}ₘ |
| 30 | **X⁵** : C-{[(CH₂)ₙ-O)]ₚ-R²}₂ | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| 31 | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| 32 | **X⁶** : C-**(**R¹-{O-[(CH₂)ₙ-O)]ₚ-R²}ₘ)₂ | **X¹** : N-[(CH₂)ₙ-O)]ₚ-R² | **X¹** : N-[(CH₂)ₙ-O)]ₚ-R² |
| 33 | **X⁶** : C-**(**R¹-{O-[(CH₂)ₙ-O)]ₚ-R²}ₘ)₂ | **X²** : N-R₁-{O-[(CH₂)ₙ-O)]ₚ-R²}ₘ ; | **X²** : N-R₁-{O-[(CH₂)ₙ-O)]ₚ-R²}ₘ |
| 34 | **X⁶** : C-**(**R¹-{O-[(CH₂)ₙ-O)]ₚ-R²}ₘ)₂ | **X³** : CH-[(CH₂)ₙ-O)]ₚ-R² | **X³** : CH-[(CH₂)ₙ-O)]ₚ-R² |
| 35 | **X⁶** : C-**(**R¹-{O-[(CH₂)ₙ-O)]ₚ-R²}ₘ)₂ | **X⁴** : CH-R¹-{O-[(CH₂)ₙ-O)**]**ₚ-R²}ₘ | **X⁴** : CH-R¹-{O-[(CH₂)ₙ-O)**]**ₚ-R²}ₘ |
| 36 | **X⁶** : C-**(**R¹-{O-[(CH₂)ₙ-O)]ₚ-R²}ₘ)₂ | **X⁵** : C-{[(CH₂)ₙ-O)]ₚ-R²}₂ | **X⁵** : C-{[(CH₂)ₙ-O)]ₚ-R²}₂ |

Dans un autre aspect, la présente description concerne un composé de formule (V) suivante : 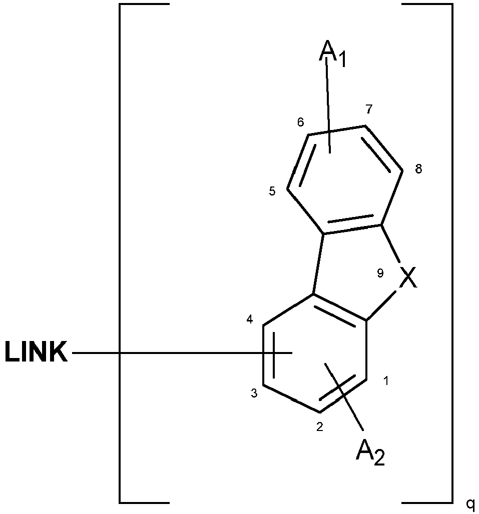 dans laquelle :
X est choisi dans le groupe constitué de :
**X¹** : N-[(CH₂)ₙ-O)]ₚ-R² ;
**X²** : N-R₁-{O-[(CH₂)ₙ-O)]ₚ-R²}ₘ ;
**X³** : CH-[(CH₂)ₙ-O)]ₚ-R² ;
**X⁴** : CH-R¹-{O-[(CH₂)ₙ-O)**]**ₚ-R²}ₘ ;
**X⁵** : C-{[(CH₂)ₙ-O)]ₚ-R²}₂ ; et
**X⁶** : C-**(**R¹-{O-[(CH₂)ₙ-O)]ₚ-R²}ₘ)₂ ; où :
   R¹ est choisi dans le groupe constitué de aryle ; hétéroaryle ; alkyl-aryle ; cycloalkyle en C₃ à C₈ ; hétérocycloalkyle en C₃ à C₈ ; notamment aryle ou hétéroaryle, de préférence aryle ;
   n est un entier de 1 à 6, notamment 2 ou 3, en particulier 2 ;
   p = est un entier de 1 à 20 ;
   m est un entier de 1 à 6, notamment de 1 à 3, en particulier 1 ;
   R² est un alkyle en C₁ à C₁₂; aryle ; hétéroaryle ; cycloalkyle en C₃ à C₈ ; hétérocycloalkyle en C₃ à C₈ ;
   A¹, situé de préférence en position 6 ou 7 du cycle, est choisi dans le groupe constitué de H ; alkyle en C₁ à C₂₀ ; alcényle en C₂ à C₂₀ ; alcynyle en C₂ à C₂₀ ; aryle, hétéroaryle ; cycloalkyle en C₃ à C₈ ; NO₂, CF₃ ; CN ; N₃ ; F ; Cl ; NR^{a}R^{b} ; OR^{c} ; SR^{c} ; C(=O)R^{d} ;; ou un chromophore ; notamment H ou un chromophore ; où :
      R^{a} est choisi dans le groupe constitué de H ; alkyle en C₁ à C₂₀ ; aryle, hétéroaryle, cycloalkyle en C₃ à C₈ ; ou R^{a} et R^{b} forment ensemble un (C₃-C₇)hétérocyclyle ;
      R^{b} est choisi dans le groupe constitué de alkyle en C₁ à C₂₀ ; aryle, hétéroaryle, cycloalkyle en C₃ à C₈ ; ou R^{a} et R^{b} forment ensemble un (C₃-C₇)hétérocyclyle ;
      R^{c} est choisi dans le groupe constitué de alkyle en C₁ à C₂₀ ; aryle, hétéroaryle, cycloalkyle en C₃ à C₈ ; C(=O)-alkyle en C₁ à C₂₀ ; C(=O)-aryle, C(=O)-hétéroaryle, C(=O)-cycloalkyle en C₃ à C₈ ;
      R^{d} représente alkyle en C₁ à C₂₀ ; aryle, hétéroaryle, cycloalkyle en C₃ à C₈ ; NR^{a}R^{b} ; OR^{c} ; SR^{c} ;
      la liaison entre le groupement LINK et le cycle est en position 2 du cycle lorsque X représente **X³** : CH-[(CH₂)ₙ-O)]ₚ-R² ; **X⁴** : CH-R¹-{O-[(CH₂)ₙ-O)**]**ₚ-R²}ₘ ; **X⁵** : C-{[(CH₂)ₙ-O)]ₚ-R²}₂ ; ou **X⁶** : C-**(**R¹-{O-[(CH₂)ₙ-O)]ₚ-R²}ₘ)₂ ;
      la liaison entre le groupement LINK et le cycle est en position 2 ou 3 du cycle lorsque X représente N-[(CH₂)ₙ-O)]ₚ-R² ou N-R₁-{O-[(CH₂)ₙ-O)]ₚ-R²}ₘ ;
      A², situé de préférence en position 2 ou 3 du cycle est choisi dans le groupe constitué de H ; alkyle en C₁ à C₂₀ ; alcényle en C₂ à C₂₀ ; alcynyle en C₂ à C₂₀ ; aryle, hétéroaryle ; cycloalkyle en C₃ à C₈ ; NO₂, CF₃ ; CN ; N₃ ; F ; Cl ; NR^{a}R^{b} ; OR^{c} ; SR^{c} ; C(=O)R^{d} ; ou un chromophore ; notamment H ; où :
         R^{a} est choisi dans le groupe constitué de H ; alkyle en C₁ à C₂₀ ; aryle, hétéroaryle, cycloalkyle en C₃ à C₈ ; ou R^{a} et R^{b} forment ensemble un (C₃-C₇)hétérocyclyle ;
         R^{b} est choisi dans le groupe constitué de alkyle en C₁ à C₂₀ ; aryle, hétéroaryle, cycloalkyle en C₃ à C₈ ; ou R^{a} et R^{b} forment ensemble un (C₃-C₇)hétérocyclyle ;
         R^{c} est choisi dans le groupe constitué de alkyle en C₁ à C₂₀ ; aryle, hétéroaryle, cycloalkyle en C₃ à C₈ ; C(=O)-alkyle en C₁ à C₂₀ ; C(=O)-aryle, C(=O)-hétéroaryle, C(=O)-cycloalkyle en C₃ à C₈ ;
         R^{d} représente alkyle en C₁ à C₂₀ ; aryle, hétéroaryle, cycloalkyle en C₃ à C₈ ; NR^{a}R^{b} ; OR^{c} ; SR^{c},
         **LINK** représente un aryle, un hétéroaryle, un polyaryle ou une triarylamine,
         q est 2,3, 4, 5 ou 6.

Dans un mode de réalisation avantageux, le groupement **LINK** est choisi dans le groupe constitué de : 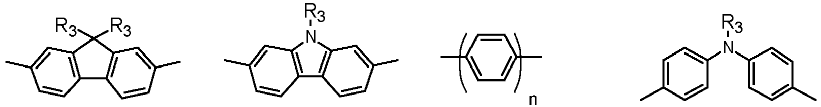 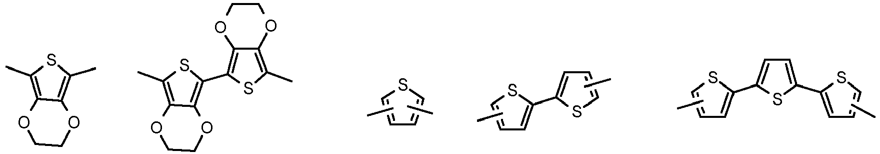 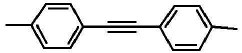 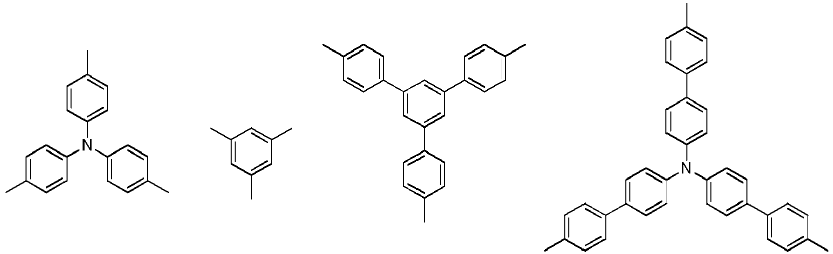 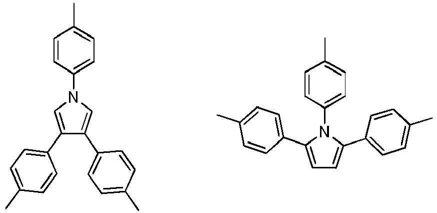 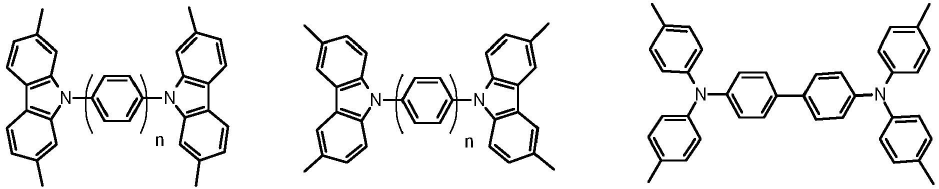 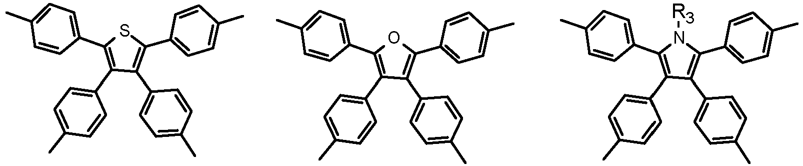 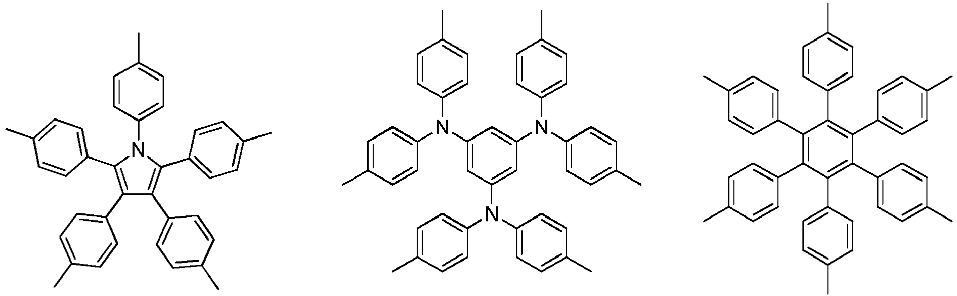 dans laquelle R³ est choisi dans le groupe constitué de alkyle en C₁ à C₂₀ ; alcényle en C₂ à C₂₀ ; alcynyle en C₂ à C₂₀ ; aryle, hétéroaryle ; cycloalkyle en C₃ à C₈ ; C(=O)R^{e} ; où R^{e} est choisi dans le groupe constitué de alkyle en C₁ à C₂₀ ; aryle, hétéroaryle, cycloalkyle en C₃ à C₈.

Dans un mode de réalisation, la présente description concerne un composé de formule (V-1) dans laquelle X est choisi dans le groupe constitué de **X¹** : N-[(CH₂)ₙ-O)]ₚ-R² ; et **X²** : N-R₁-{O-[(CH₂)ₙ-O)]ₚ-R²}ₘ.

Dans un autre mode de réalisation, la présente description concerne un composé de formule (V-1) dans laquelle X est choisi dans le groupe constitué de **X³** : CH-[(CH₂)ₙ-O)]ₚ-R² ; **X⁴** : CH-R¹-{O-[(CH₂)ₙ-O)**]**ₚ-R²}ₘ ; **X⁵** : C-{[(CH₂)ₙ-O)]ₚ-R²}₂ ; et **X⁶** : C-**(**R¹-{O-[(CH₂)ₙ-O)]ₚ-R²}ₘ**)**₂.

La présente invention concerne en outre l'utilisation d'un composé de formule (IV) ou un mélange de ces composés dans un dispositif optoélectronique tels qu'une cellule solaire photovoltaïque à colorant photosensible (DSSC), une cellule solaire photovoltaïque à quantum dot, une cellule solaire photovoltaïque hybride, une cellule solaire photovoltaïque organique, une cellule solaire photovoltaïque à pérovskite, une diode électroluminescente organique et un transistor organique à effet de champ (OFETs)..

La présente invention concerne en outre un dispositif optoélectronique tel qu'une cellule solaire photovoltaïque à colorant photosensible (DSSC), une cellule solaire photovoltaïque à quantum dot, une cellule solaire photovoltaïque hybride, une cellule solaire photovoltaïque organique, une cellule solaire photovoltaïque à pérovskite, une diode électroluminescente organique ou un transistor organique à effet de champ (OFETs) , comprenant au moins un composé de formule (IV).

Des exemples de synthons illustratifs de la présente description sont les suivants :

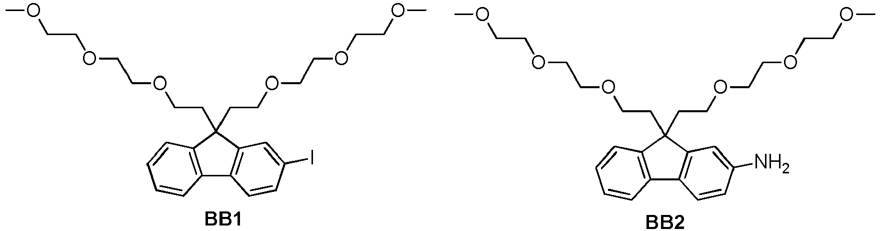

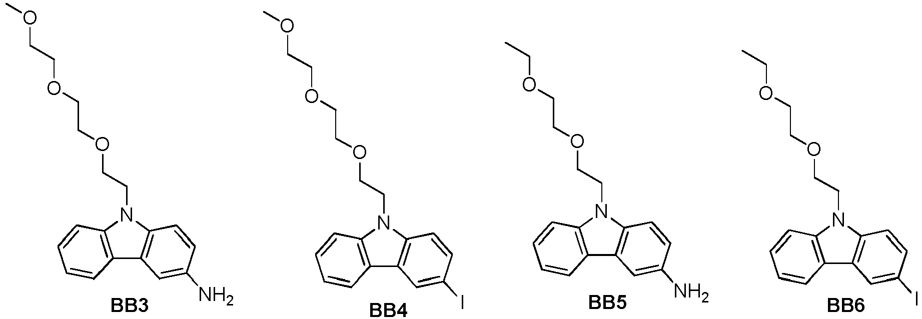

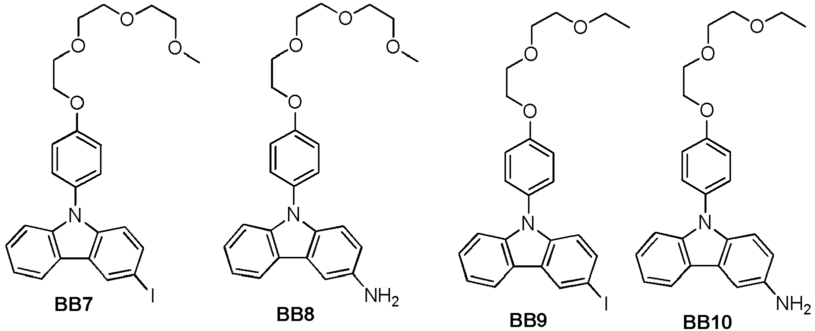

Des exemples de composés utiles en tant que matériaux pour les dispositifs électroniques organiques ou hybride tel qu'une cellule à pigment photosensible (DSSC), une cellule photovoltaïque organique (OPV), les cellules photovoltaïques polymères (PSC) les diodes électroluminescentes organiques (OLEDs), et les transistors à effet de champ organiques (OFETs), illustratifs de la présente invention sont les suivants :

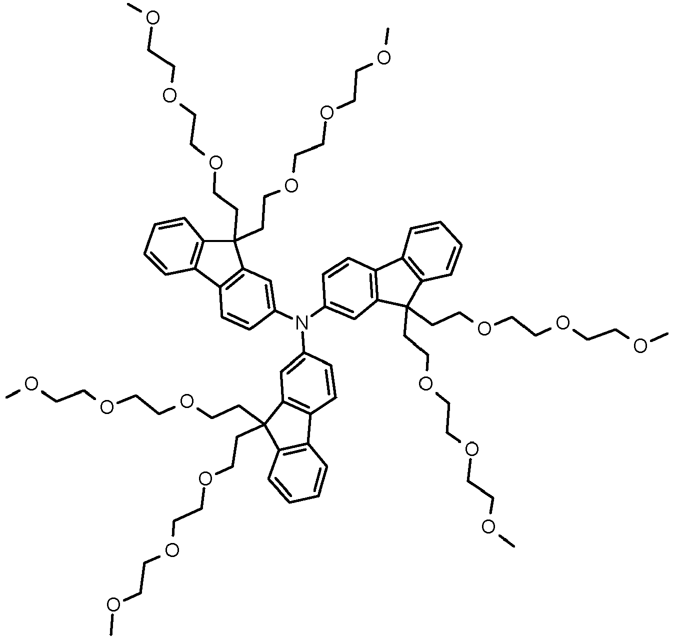

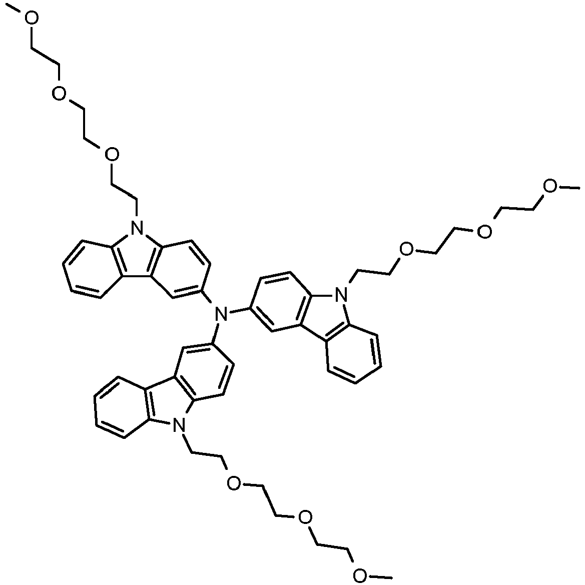

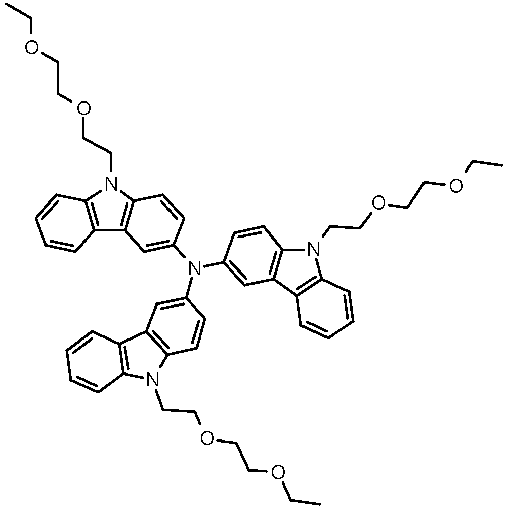

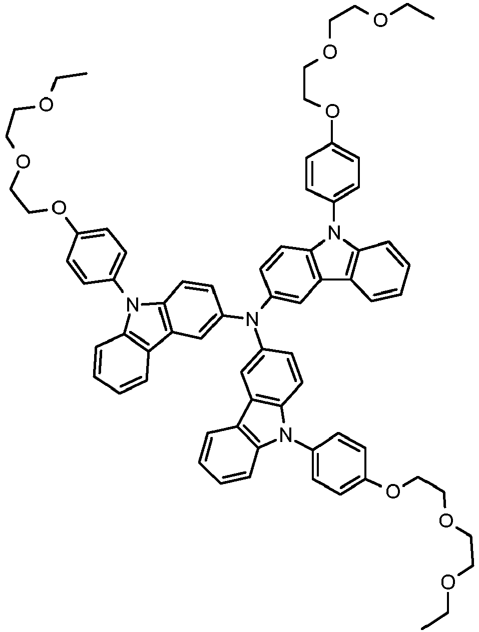

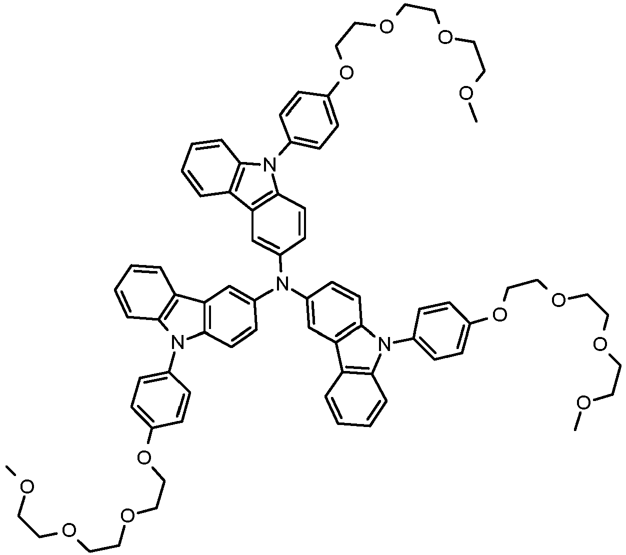

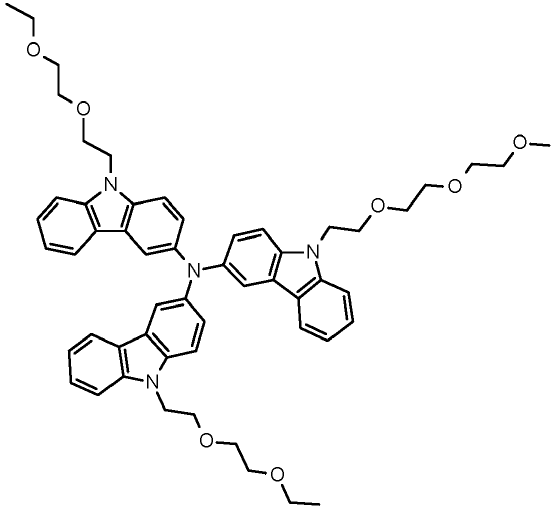

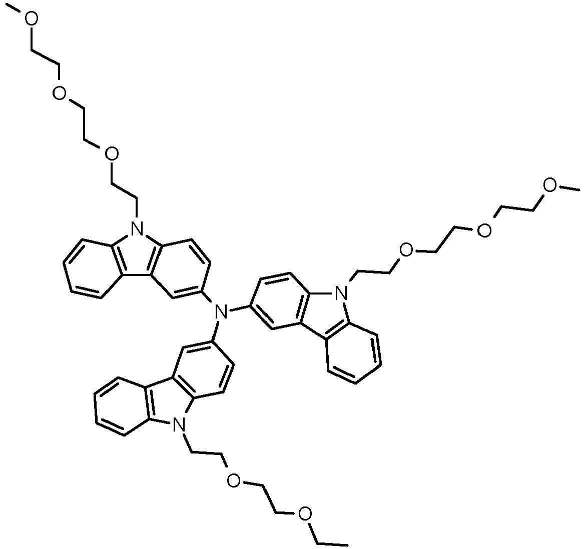

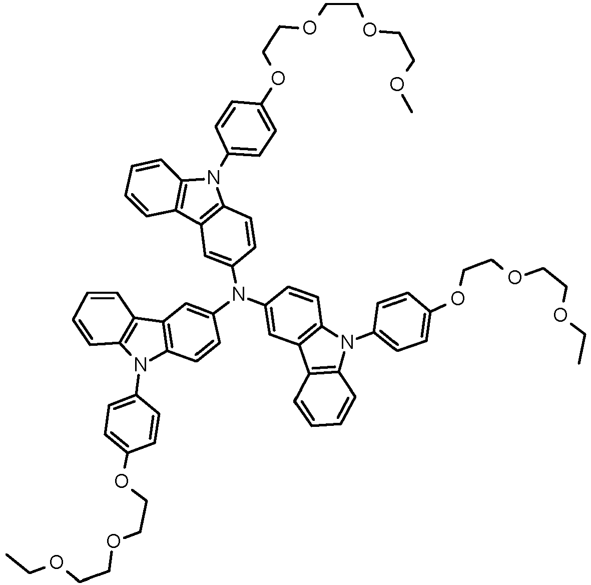

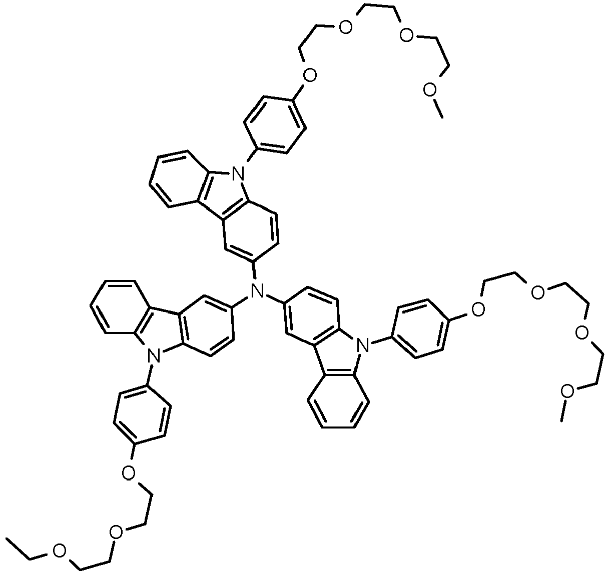

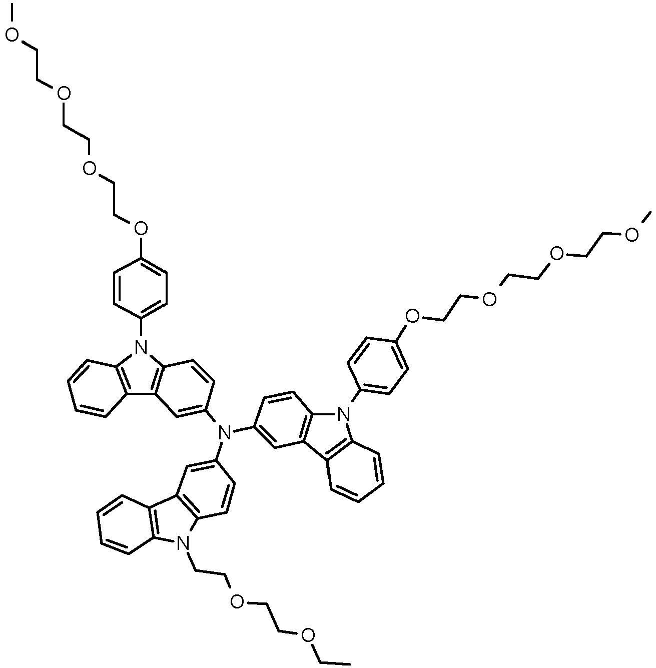

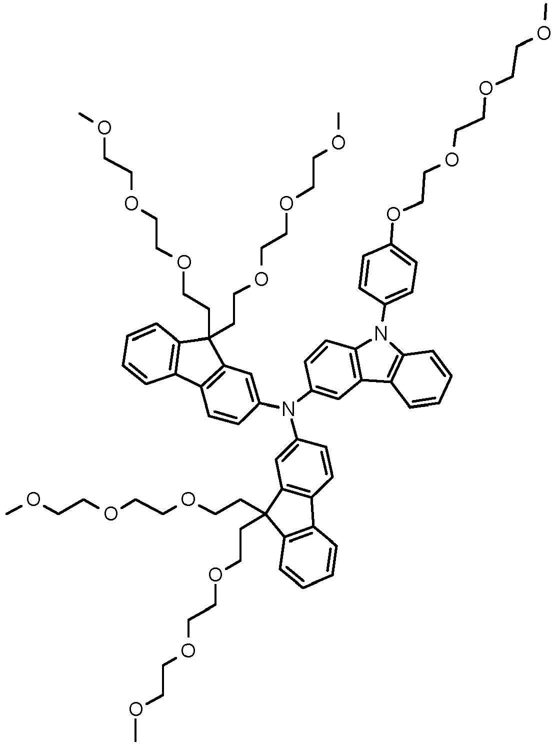

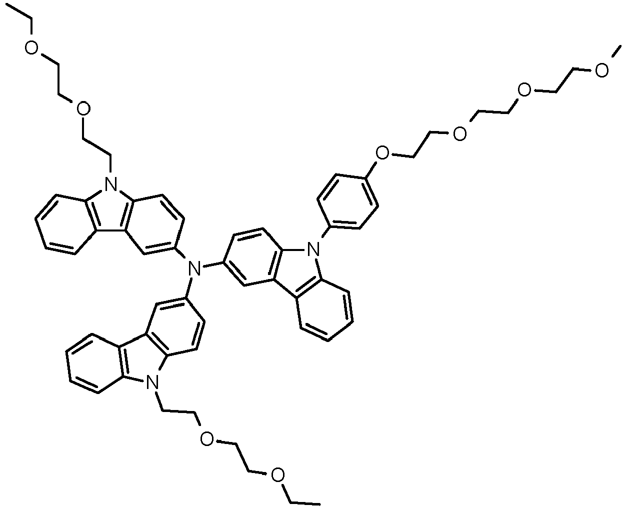

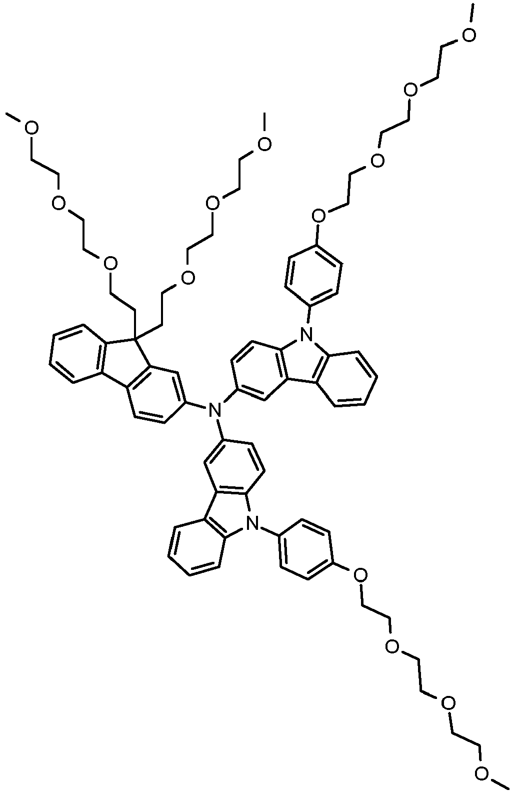

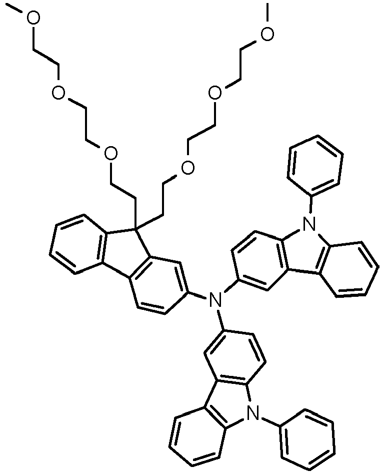

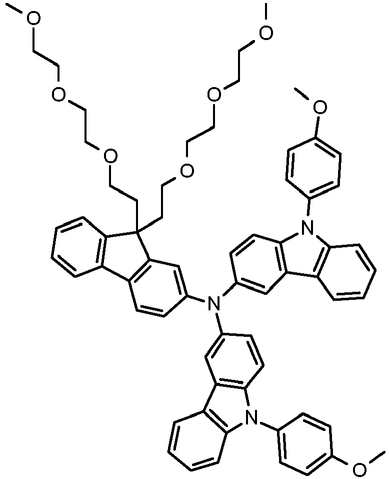

### Figures :

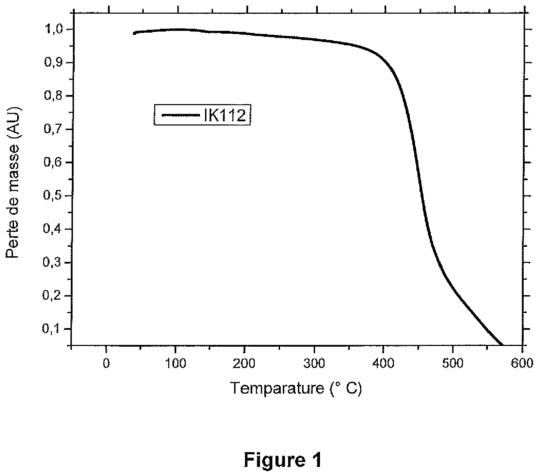

**Figure 1** **:** Thermogramme (abscisse Température (en °C) ; ordonnée Perte de masse (SU)) des mesures ATG du matériau **IK112,** balayage à 10°C/min sous atmosphère d'Azote.

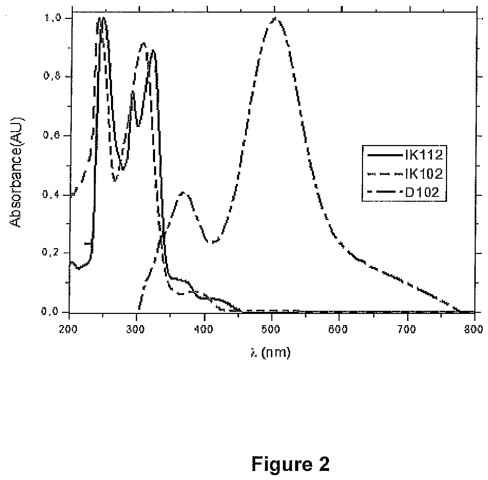

**Figure 2** **:** Spectres d'absorption UV-Visible (abscisse Longueur d'onde émise (en nm) ; ordonnée Absorbance (SU)) des composés **IK102** et **IK112** en solution à 10⁻⁵ M dans le THF comparativement au spectre du colorant utilisé (D102 en solution à 10⁻⁴ M dans l'acétonitrile).
**Figure 3** **:** Voltamogrammes (abscisse Tension (en V) ; ordonnée Intensité (en mA)) à 50 mV/s des composés **IK102** et **IK112** à 10⁻³ M dans une solution de N(Et)₄BF₄ à 0.1 M dans l'acétonitrile sur une électrode de Pt de 1cm de diamètre.
**Figure 4** : Caractéristiques J-V (abscisse Tension (en V) ; ordonnée Densité de courant (en mA.cm⁻¹)) dans l'obscurité des dispositifs utilisant les semiconducteurs synthétisés comme HTMs (**IK102** et **IK112**) en combinaison avec le colorant **D102.**
**Figure 5** : Caractéristiques J-V (abscisse Tension (en V) ; ordonnée Densité de courant (en mA.cm⁻¹)) sous illumination (AM 1.5 G) des dispositifs utilisant les semiconducteurs synthétisés comme HTMs (**IK102** et **IK112**) en combinaison avec le colorant **D102.**

### EXEMPLES :

### Exemple 1 : Mode de synthèse des composés

Certains des composés de formule I nécessaires à la synthèse des exemples de formule II, III, IV et V donnés sont produits à partir du carbazole et du fluorène commercial.

Les composés de formule I, pour les exemples donnés, dans lesquels Y représente NHP, avec P correspondant à un hydrogène, ont été produits à partir du carbazole commercial en 3 à 5 étapes comprenant principalement une étape de nitration sélective du carbazole en position 3 ou en position 2 du fluorène, une étape de fonctionnalisation de la position 9 du carbazole ou du fluorène et une étape de réduction de la fonction nitro en amine libre.

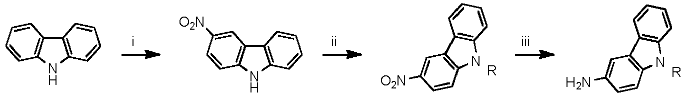

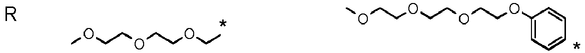

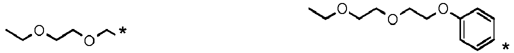

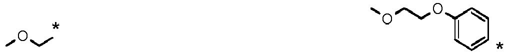

Le schéma précédent décrit la synthèse de composés de formule I avec Y = NH₂ (carbazoles) : (i) HNO₃, AcOH; (ii) NaH, RX (X = Br ou OTs) / Cu, K2CO3, 18-crown-6, o-DCB; (iii) SnCl₂, EtOH

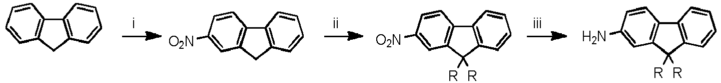

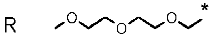

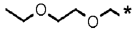

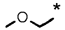

Le schéma précédent décrit la synthèse de composés de formule I avec Y = NH₂ (fluorène) : i) HNO₃, AcOH; ii) NaH, RX (X = Br ou OTs) iii) SnCl₂, EtOH

Les exemples de formule I dans laquelle Y représente un halogène, ont été produits à partir du carbazole commercial en 2 à 3 étapes comprenant principalement une étape de nitration sélective du carbazole en position 3, une étape de fonctionnalisation de la position 9 du carbazole par couplage C-N (groupements aromatiques) ou par substitution nucléophile (chaines aliphatiques) et une étape de réduction de la fonction nitro en amine libre.

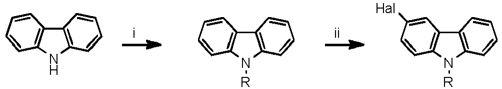

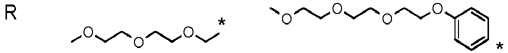

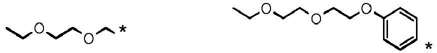

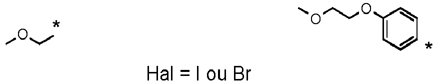

Le schéma précédent décrit la synthèse de composés carbazoles de formule I avec : i) NaH, RX (X = Br ou OTs) Il RX (X = Br ou OTs), Cu, K₂CO₃, 18-crown-6, o-DCB. ii) NBS, CHCl₃ (pour Hal = Br)// KI, KIO₃, AcOH (pour Hal = I)

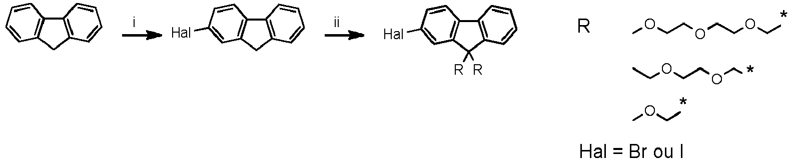

Le schéma précédent décrit la synthèse de composés fluorène de formule I : i) NBS, CHCl₃ (pour Hal = Br)// KI, KIO₃, AcOH (pour Hal = I); ii) NaH, RX (X = Br ou OTs)

Les groupements R aliphatiques ont été obtenus à partir de dérivés de glycols par traitement de ces derniers par le tribromure de phosphore selon la formule :

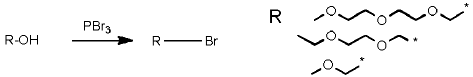

Les groupements R aromatiques ont été obtenus à partir du 4-iodo ou 4-bromophénol et de dérivés de glycols en deux étapes : une première étape de tosylation ou de bromation de l'alcool correspondant, suivi d'une seconde étape correspondant à la substitution nucléophile du groupement partant tosyle ou brome par le dérivé de phénol correspondant.

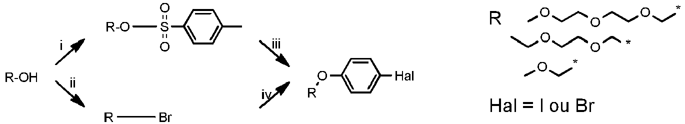

Le schéma précédent décrit la synthèse de groupements aromatiques : (i) NaOH, TsCI, THF ; (ii) PBr₃ ; (iii) t-BuOK, 4-halophenol, DMF ; (iv) KOH, 4-halophenol, DMF ou THF.

Les composés de formule II, III, IV et V ont été obtenus par une réaction de couplage C-N entre un composé de formule I dans laquelle Y représente NHP, avec P correspondant à un hydrogène ou à un groupement quelconque, et un composé de formule (I) dans lequel Y a été choisi dans le groupe constitué groupes partants comme Br ; I, B(OR)₂ ; BF3K ; Sn(Bu)₃ ; OSO2R et N₂⁺, préférentiellement de Br ; I et OSO2PhMe (OTs)

La méthode de couplage en présence de cuivre décrite dans J. Power Sources 2013, 233, 86-92 ainsi que la méthode de couplage au palladium décrite dans Polymlnt 2014; 63: 1387-1393 peuvent être préférentiellement appliquées pour effectuer le couplage C-N permettant la synthèse des composés de la présente invention. Avec X, A, Ar et Y définis comme précédemment.

Selon ces deux familles de couplages CN, les composés de formule Il ont été obtenus en une étape. Les composés de formule III et IV peuvent être obtenus en une ou deux étapes en fonction du groupement P et de la symétrie de la triarylamine.

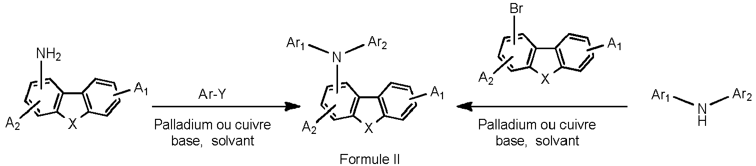

### Le schéma précédent décrit la synthèse des exemples de formule Il (exemples comparatifs)

On peut citer les exemples de formule Il suivants :

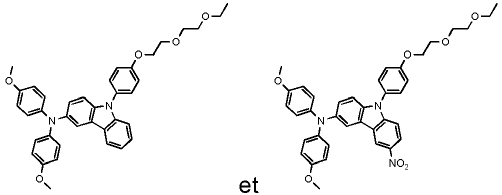

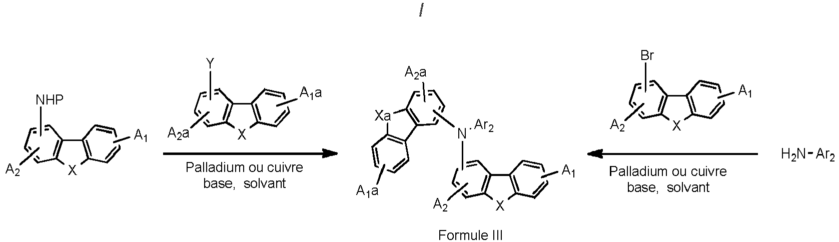

### Le schéma précédent décrit la synthèse des exemples de formule III (exemples comparatifs)

On peut citer en les exemples de formule III suivants :

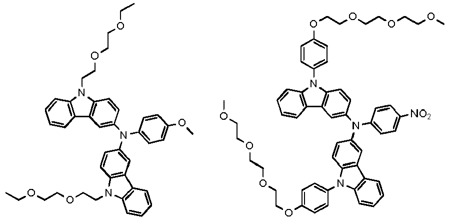

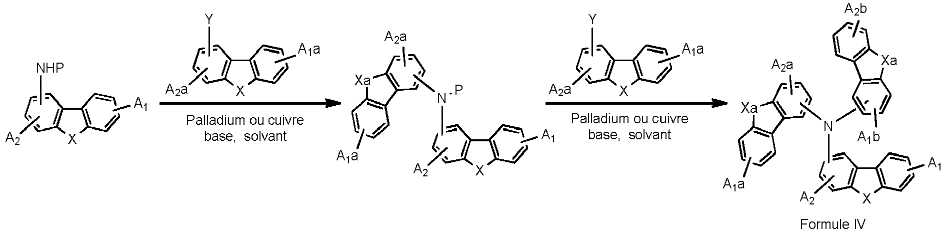

### Le schéma précédent décrit la synthèse des exemples de formule IV

On peut citer les exemples de formule IV suivants :

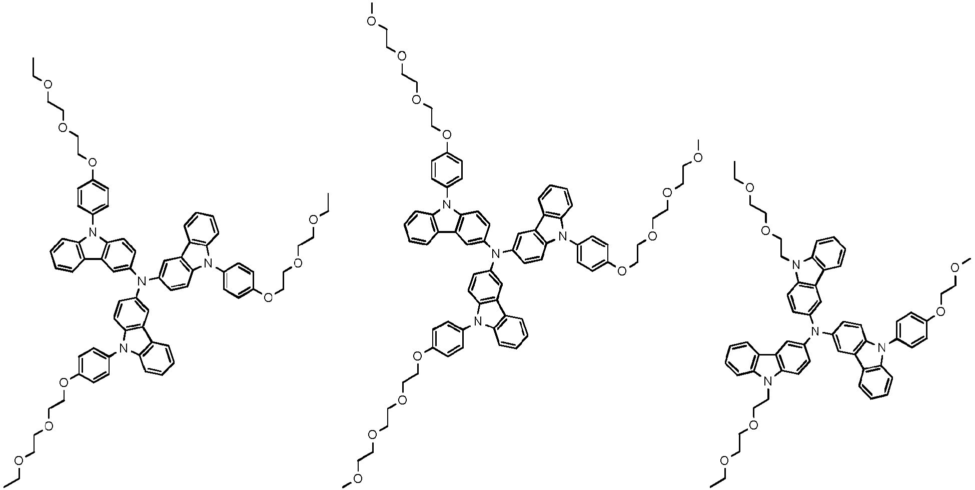

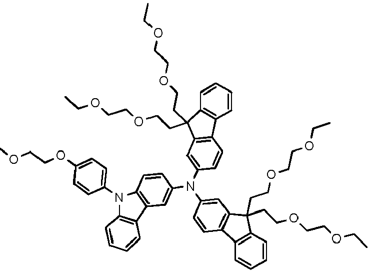

On peut citer l'exemple de formule V suivant (exemple comparatif) :

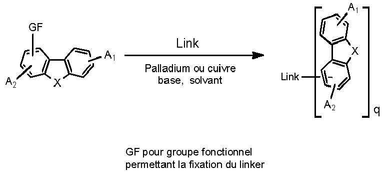

Le schéma précédent décrit la synthèse des exemples de formule V, comme par exemple le composé suivant :

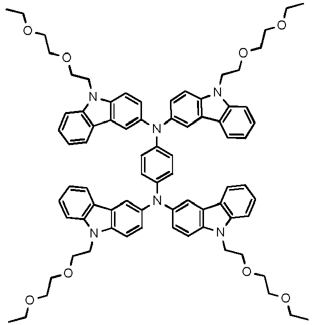

Lorsque la méthode au cuivre est employée, la base préférentiellement utilisée est une base inorganique telle que les carbonates, par exemple. Préférentiellement, le K₂CO₃ est employé. Le solvant peut être un solvant organique polaire ou non tel que le DMF, le DMSO, le benzène et ses dérivés ou un dérivé de glycol. Préférentiellement, l'ortho-dichlorobezène est utilisé. La réaction peut être menée à des températures comprises entre 100 et 400°C.

Lorsque la méthode au palladium est employée, la base préférentiellement utilisée est une base organique. Préférentiellement les tert butoxylates de sodium ou de potassium sont employés. Le solvant peut être un solvant organique polaire ou non tel que le DMF, le DMSO, le benzène et ses dérivés ou un dérivé de glycol. Préférentiellement, le toluène est utilisé. La réaction peut être menée à des températures comprises entre 50 et 200°C.

### Exemple 2 : Synthèses des composées

### Synthèse du PEG1Ts : 2-(2-(2-Méthoxyéthoxy)éthoxy)éthyl 4-méthylbenzènesulfonate

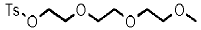

Dans un ballon monocol, du 2-(2-(2-méthoxyéthoxy)éthoxy)éthanol (32,8 g ; 200 mmoles), du chlorure de tosyle (48 g ; 1,5 eq) et du tétrahydrofurane (200 mL) ont été insérés. Le mélange a été agité jusqu'à dissolution complète des solutés puis la température a été portée à 0°C dans un mélange glace-sel. Une solution de d'hydroxyde de potassium (37,7 mL solution 16 mol.L-1 ; 3 eq) a été ensuite ajoutée goutte à goutte au mélange en faisant attention à rester en dessous de 5°C. À la fin de l'addition, le mélange a été laissé à l'agitation à température ambiante et des chromatographies sur couche mince ont été faites pour suivre l'évolution de la réaction. À la fin de la réaction 100 mL d'eau distillée ont été ajoutés et le mélange a été extrait avec de l'acétate d'éthyle (4x100mL). Les phases organiques ont été réunies et lavées avec une solution de chlorure de sodium saturée (3x60 mL), à l'eau distillée (100 mL) puis séchées et évaporées. Le composé a été obtenu sous la forme d'un liquide fluide légèrement jaune avec un rendement de 89%. Ce produit est suffisamment pur pour les opérations ultérieures.

RMN 1H (300 MHz, ppm, CDCl3) δ 2,44 (s, 3H) ; 3,36 (s, 3H); 3,49-3,55 (m, 2H); 3,57-3,63 (m, ,2H); 3,58 (s, 4H); 3,64-3,71 (m, 2H); 4,12-4,19 (m, 2H); 7,33 (d, 2H, J = 8,3 Hz); 7,79 (d, 2H, J = 8,3 Hz).

### Synthèse du PEG2Ts : 2-(2-Ethoxyéthoxy)éthyl 4-méthylbenzènesulfonate

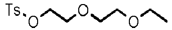

Dans un ballon monocol, du 2-(2-éthoxyéthoxy)éthanol (67,1 g ; 500 mmoles), du chlorure de tosyle (143 g ; 1,5 eq) et du tétrahydrofurane (300 mL) ont été insérés. Le mélange a été agité jusqu'à dissolution complète des solutés puis la température a été portée à 0°C dans un mélange glace-sel. Une solution de d'hydroxyde de potassium (90 mL solution 16 mol.L⁻¹ ; 3 eq) a été ensuite ajoutée goutte à goutte au mélange en faisant attention à rester en dessous de 5°C. À la fin de l'addition, le mélange a été laissé à l'agitation à température ambiante et des chromatographies sur couche mince ont été faites pour suivre l'évolution de la réaction. À la fin de la réaction 200 mL d'eau distillée ont été ajoutés et le mélange a été extrait avec de l'acétate d'éthyle (4x125mL). Les phases organiques ont été réunies et lavées avec une solution de chlorure de sodium saturée (3x150 mL), à l'eau distillée (200 mL) puis séchées sur sulfate de magnésium et évaporées. Le composé a été obtenu sous forme d'un liquide fluide légèrement jaune avec un rendement de 97%. Ce produit est suffisamment pur pour les opérations ultérieures.

RMN 1H (300 MHz, ppm, CDCl3) δ 1,16 (t, 3H, J = 7,1 Hz) ; 2,41 (s, 3H) ; 3,46 (q, 2H, J = 7,1 Hz) ; 3,46-3,51 (m, 2H) ; 3,51-3,58 (m, 2H) ; 3,62-3,69 (m, 2H) ; 4,10-4,17 (m, 2H) ; 7,27-7,35 (m, 2H) ; 7,73-7,80 (m, 2H).

### Synthèse du PEG3Ts : 2-Méthoxyéthyl 4-méthylbenzènesulfonate

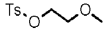

Dans un ballon monocol, du 2-méthoxyéthanol (30 g ; 400 mmoles), du chlorure de tosyle (97,15 g ; 1,25 eq) et du tétrahydrofurane (400 mL) ont été insérés. Le mélange a été agité jusqu'à dissolution complète des solutés puis la température a été portée à 0°C dans un mélange glace-sel. Une solution de d'hydroxyde de potassium (75 mL solution 16 mol.L⁻¹ ; 3 eq) a été ensuite ajoutée goutte à goutte au mélange en faisant attention à rester en dessous de 5°C. À la fin de l'addition, le mélange a été laissé à l'agitation à température ambiante et des chromatographies sur couche mince ont été faites pour suivre l'évolution de la réaction. À la fin de la réaction 160 mL d'eau distillée ont été ajoutés et le mélange a été extrait avec de l'acétate d'éthyle (4x200mL). Les phases organiques ont été réunies et lavées avec une solution de chlorure de sodium saturée (3x120 mL), à l'eau distillée (160 mL) puis séchées sur sulfate de magnésium et évaporées. Le composé a été obtenu sous forme d'un liquide fluide légèrement jaune avec un rendement de 97%. Ce produit est suffisamment pur pour les opérations ultérieures.

RMN 1H (300 MHz, ppm, CDCl3) δ 2,42 (s, 3H) ; 3,28 (s, 3H) ; 3,52-3,59 (m, 2H); 4,01-4,20 (m, 2H); 7,32 (d, 2H, J = 8,3 Hz); 7,78 (d, 2H, J = 8,3 Hz).

### Synthèse du 3-iodocarbazole

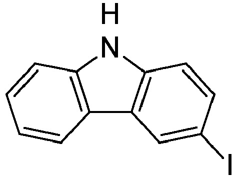

Dans un ballon du carbazole (5,6 g, 33.3 mmol, 1eq) et du KI (3,7 g, 22.2 mmol, 0,66 eq) ont été mis en solution dans l'acide acétique glacial (100 mL) à 100°C sous agitation pendant une heure. Le KIO₃ (3.6 g, 16.7 mmol, 0.5 eq) a été ensuite ajouté par petite portion et le mélange réactionnel a été laissé sous agitation à 100°C pendant deux heures supplémentaires. Une solution de thiosulfate de sodium a été alors ajouté et le solide formé a été récupéré par filtration puis recristallisé dans le dichlorométhane pour obtenir des paillettes avec un rendement de 30%.

¹H NMR (300 MHz, Acetone) δ 10,52 (m, 1H), 8,49 (s, 1H), 8,15 (d, *J =* 7,9 Hz, 1H), 7,71-7,38 (m, 4H), 7,23 - 7,18 (m, 1H).

### Synthèse du 1-lodo-4-(2-(2-(2-méthoxyéthoxy)éthoxy)éthoxy)benzène

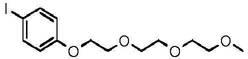

Dans un ballon, du 4-iodophénol (8,99 g ; 40 mmoles), du tert-butoxyde de potassium (6,7 g ; 1,5 eq) et du diméthylformamide (100 mL) ont été insérés. Le mélange a été agité jusqu'à dissolution complète des solutés puis le 2-(2-(2-méthoxyéthoxy)éthoxy)éthyl 4-méthylbenzènesulfonate (25,5 g ; 2 eq) a été ajouté. Le mélange a été laissé à l'agitation à température ambiante et des chromatographies sur couche mince ont été faites pour suivre l'évolution de la réaction. À la fin de la réaction 200 mL d'eau distillée ont été ajoutés et le mélange a été extrait avec de l'acétate d'éthyle (4x150mL). Les phases organiques ont été réunies et lavées à l'eau distillée (5x200 mL) puis séchées sur sulfate de magnésium et évaporées. Le mélange obtenu a été chromatographié sur colonne silice. Un liquide visqueux jaunâtre a été obtenu avec un rendement de 93%.

RMN 1H (300 MHz, ppm, CDCl3) δ 3,34 (s, 3H) ; 3,47-3,55 (m, 2H); 3,59-3,66 (m, 4H) ; 3,67-3,72 (m, 2H); 3,77-3,85 (m, 2H); 4,01-4,08 (m, 2H); 6,62-6,71 (m, 2H); 7,46-7,55 (m, 2H).

### Synthèse du 1-(2-(2-Ethoxyéthoxy)éthoxy)-4-iodobenzène

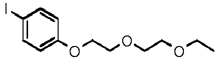

Dans un ballon, du 4-iodophénol (8,99 g ; 40 mmoles), du tert-butoxyde de potassium (6,7 g ; 1,5 eq) et du diméthylformamide (100 mL) ont été insérés. Le mélange a été agité jusqu'à dissolution complète des solutés puis le 2-(2-éthoxyéthoxy)éthyl 4-méthylbenzènesulfonate (23,2 g ; 2 eq) a été ajouté. Le mélange a été laissé à l'agitation à température ambiante et des chromatographies sur couche mince ont été faites pour suivre l'évolution de la réaction. À la fin de la réaction 200 mL d'eau distillée ont été ajoutés et le mélange a été extrait avec de l'acétate d'éthyle (4x150mL). Les phases organiques ont été réunies et lavées à l'eau distillée (5x200 mL) puis séchées sur sulfate de magnésium et évaporées. Le mélange obtenu a été chromatographié sur colonne silice. Un liquide visqueux jaunâtre a été obtenu avec un rendement de 94%.

RMN 1H (300 MHz, ppm, CDCl3) δ 1,21 (t, 3H, J = 7,1) ; 3,53 (q, 2H, J = 7,1) ; 3,57-3,63 (m, 2H) ; 3,68-3,73 (m, 2H) ; 3,81-3,87 (m, 2H) ; 4,05-4,12 (m, 2H) ; 6,65-6,72 (m, 2H) ; 7,49-7,57 (m, 2H).

### Synthèse du 1-lodo-4-(2-méthoxyéthoxy)benzène

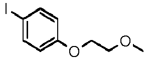

Dans un ballon, du 4-iodophénol (8,99 g ; 40 mmoles), du tert-butoxyde de potassium (6,7 g ; 1,5 eq) et du diméthylformamide (100 mL) ont été insérés. Le mélange a été agité jusqu'à dissolution complète des solutés puis le 2-méthoxyéthyl 4-méthylbenzènesulfonate (18,4 g ; 2 eq) a été ajouté. Le mélange a été laissé à l'agitation à température ambiante et des chromatographies sur couche mince ont été faites pour suivre l'évolution de la réaction. À la fin de la réaction 200 mL d'eau distillée ont été ajoutés et le mélange a été extrait avec de l'acétate d'éthyle (4x150mL). Les phases organiques ont été réunies et lavées à l'eau distillée (5x200 mL) puis séchées sur sulfate de magnésium et évaporées. Le mélange obtenu a été chromatographié sur colonne silice. Un liquide visqueux jaunâtre a été obtenu avec un rendement de 86%.

RMN 1H (300 MHz, ppm, CDCl3) δ 2,39 (s, 3H) ; 3,25 (s, 3H); 3,46-3,58 (m, 2H); 4,05-4,16 (m, 2H); 7,25-7,35 (m, 2H); 7,70-7,80 (m, 2H).

### Synthèse du 3-Nitro-9H-carbazole

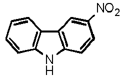

Dans un ballon bicol adapté à un réfrigérant, du carbazole (18,3 g, 0,11 mole) et 300 mL d'acide acétique ont été insérés. La température a été augmentée à 60°C. Après dissolution complète du carbazole, de l'acide nitrique (7,8 mL à 65% dans 36 mL d'acide acétique, 1,05 eq) a été ajouté goutte à goutte. Des chromatographies sur couche mince ont été faites pour suivre l'évolution de la réaction (éluant n-pentane/acétate d'éthyle 8 :2). À la fin de la réaction, le mélange a été laissé au repos pendant 24h puis filtré sur un entonnoir filtrant. La phase solide a été lavée avec 3x50 mL d'acide acétique. Le filtrat a été précipité dans 1 L d'eau à -5°C et le mélange a été laissé au repos pendant 30 minutes. Après filtration, un cake jaune a été récupéré puis lavé plusieurs fois à l'eau (5x200mL) et au méthanol (2x50 mL). Le cake a été séché à 50°C sous vide pendant 24h puis a été purifié par chromatographie sur colonne de silice. Le produit recherché a été obtenu sous la forme d'un solide jaune avec un rendement de 44%.

RMN 1H (300 MHz, ppm, THF-d8) δ 7,22-7,30 (m, 1H) ; 7,41-7,55 (m, 2H) ; 7,50 (d, 1H, J = 8,9) 8,23 (d, 1H, J = 7,9) ; 8,28 (dd, 1H, J = 7,9/2,3) ; 9,05 (d, 1H, J = 2,3) ; 11,06 (s, 1H).

### Synthèse du PEG1-Cz-I

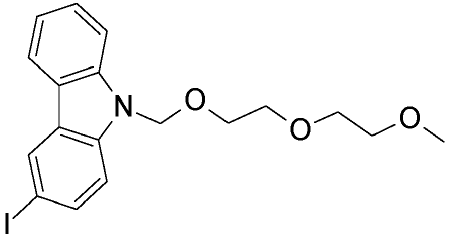

Dans un ballon, le 3-iodocarbazole (600 mg, 2 mmol, 1 eq) et le NaH 60% (140 mg, 4 mmol, 2 eq) ont été mis en solution dans le THF (30 mL). Après agitation pendant 20 minutes environ à température ambiante, le dérivé de tosyle PEG1-Ts (955 mg, 3 mmol, 1,5 eq) a été ajouté et le mélange réactionnel a été laissé sous agitation pendant une nuit. A la fin de la réaction, environ 100 mL d'eau a été ajouté au mélange et le brut a été extrait avec de l'acétate d'éthyle. Les phases organiques rassemblées ont été lavées avec une solution saturée de NaCl et séchées avec du MgSO₄. Après purification par colonne chromatographique avec un mélange éther de pétrole acétate d'éthyle, un liquide visqueux a été obtenu (rendement 76%).

¹H NMR (300 MHz, Acétone) δ 8.48 (s, 1H), 8.15 (d, *J* = 7.8 , 1H), 7.71 (dd, *J* = 8.2, 1.7 , 1H), 7.58 (d, *J* = 8.2 , 1H), 7.50 - 7.43 (m, 2H), 7.26 - 7.20 (m, 1H), 4.51 (t, *J* = 5.4 , 2H), 3.84 (t, *J* = 5.4 , 2H), 3.51 - 3.25 (m, 8H), 3.23 (s, 3H).

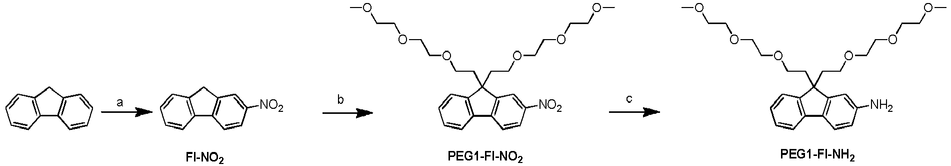

### (a) DCE, HNO₃, 0°C (b) KOH, DMSO, RT (c) SnCl₂, AcOH, HCl

### Synthèse du FI-NO₂

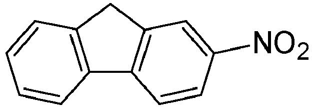

Dans un ballon le fluorène (4 g, 24 mmol, 1eq) a été mis en solution dans le dichloroéthane (50 mL) à température ambiante. Après dissolution complète du fluorène, la température a été ramenée à 0°C. Le HNO₃ (65%) a été alors ajouté (10 mL, 163 mmol, 6.8 eq) par petit volume puis le mélange réactionnel a été laissé sous agitation à 0°C pendant 10 min environ. Un précipité jaune se forme. Le solide obtenu a été récupéré par filtration puis lavé à l'éthanol et recristallisé dans ce même solvant. Rendement (63%)

¹H NMR (300 MHz, Acetone) δ 8.45 (s, 1H), 8.31 (dd, *J* = 8.4, 2.1 , 1H), 8.16 - 7.92 (m, 2H), 7.77 - 7.56 (m, 1H), 7.53 - 7.41 (m, 2H), 4.11 (s, 3H).

### Synthèse du PEG1-FI-NO₂ :

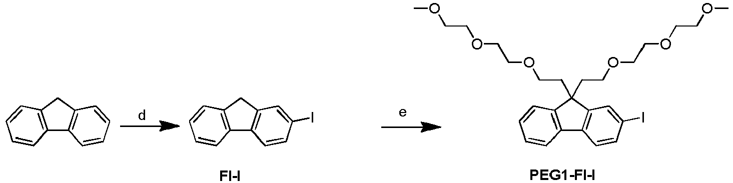

### (d) H₅IO₆, I₂, AcOH, 65°C (e) KOH, DMSO, RT

### Synthèse du 2-iodo-fluorène

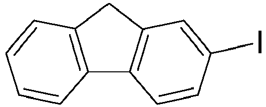

Dans un ballon le fluorène (10 g, 60 mmol, 1 eq) a été mis en solution dans 100 mL d'un mélange AcOH : Eau : H₂SO₄ (77 :20 :3). Le mélange réactionnel a été alors chauffé à 100 °C pour solubiliser le fluorène puis la température a été baissée à 65°C. Le H₅IO₆ (2.3 g, 10 mmol, 0.17 eq) le I₂ (5.1 g, 20 mmol, 0.34 eq) ont été ensuite ajoutés. Le mélange réactionnel a été alors laissé sous agitation à 65°C jusqu'à la consommation complète du I₂ (environ 3 heures). A la fin de la réaction, une solution de thiosulfate de sodium à 5% a été alors ajouté au mélange après avoir laissé la température revenir à l'ambiante. Le solide formé a été récupéré par filtration puis lavé à l'eau et recristallisé dans le n-hexane pour obtenir un solide blanc. Rendement 60%

¹H NMR (300 MHz, Acetone) δ 7.95 (s, 1H), 7.88 (d, *J =* 8.3 , 1H), 7.72 (m, 2H), 7.58 (d, *J =* 7.0 , 1H), 7.46 - 7.28 (m, 2H), 3.92 (s, 2H).

### Synthèse du 9-(4-(2-Méthoxyéthoxy)phényl)-9H-carbazole

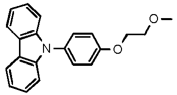

Dans un ballon bicol adapté à un réfrigérant, du carbazole (20 mmol ; 3,4 g), de la poudre de cuivre (5,10 g ; 4 eq), du carbonate de potassium (22,5 g ; 8 eq), du 18-Crown-6 (0,32 g ; 0,06 eq) et du 1-iodo-4-(2-méthoxyéthoxy)benzène (6,7 g ; 1,3 eq) ont été insérés. Le milieu réactionnel a été dégazé, placé sous atmosphère inerte et 50 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice. Un solide blanchâtre a été obtenu avec un rendement de 78%.

**RMN ¹H** (300 MHz, ppm, CDCl₃) δ 3,51 (s, 3H) ; 3,78-3,89 (m, 2H); 4,17-4,28 (m, 2H); 7,09-7,19 (m, 2H); 7,25-7,49 (m, 8H); 8,10-8,20 (m, 2H).

### Synthèse du 9-(4-(2-(2-éthoxyéthoxy)éthoxy)phényl)-9H-carbazole

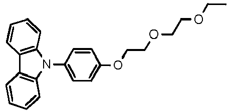

Dans un ballon bicol adapté à un réfrigérant, du carbazole (20 mmol, 3,4 g), de la poudre de cuivre (5,10 g, 4 eq), du carbonate de potassium (22,5 g, 8 eq), du 18-Crown-6 (0,32 g, 0,06 eq) et du 1-(2-(2-éthoxyéthoxy)éthoxy)-4-iodobenzène (8,9 g, 1,3 eq) ont été insérés. Le milieu réactionnel a été dégazé, placé sous atmosphère inerte et 50 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice. Un solide blanchâtre a été obtenu avec un rendement de 80,3%.

**RMN ¹H** (300 MHz, ppm, CDCl₃) δ 1,24 (t, 3H, *J* = 7,1) ; 3,57 (q, 2H, *J* = 7,1) ; 3,62-3,69 (m, 2H) ; 3,74-3,81 (m, 2H) ; 3,91-3,98 (m, 2H) ; 4,21-4,29 (m, 2H) ; 7,09-7,17 (m, 2H) ; 7,22-7,48 (m, 2H) ; 8,10-8,18 (bd, 2H, *J* = 7,6).

### Synthèse du 9-(4-(2-(2-(2-Méthoxyéthoxy)éthoxy)éthoxy)phényl)-9H-carbazole

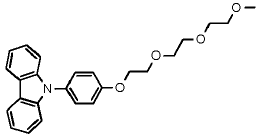

Dans un ballon bicol adapté à un réfrigérant, du carbazole (20 mmol, 3,4 g), de la poudre de cuivre (5,10 g, 4 eq), du carbonate de potassium (22,5 g, 8 eq) du 18-Crown-6 (0,32 g, 0,06 eq) et du 1-iodo-4-(2-(2-(2-méthoxyéthoxy)éthoxy)éthoxy)benzène (9,69 g, 1,3 eq) ont été insérés. Le milieu réactionnel a été dégazé, placé sous atmosphère inerte et 50 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice. Un solide blanchâtre a été obtenu avec un rendement de 84%.

**RMN ¹H** (300 MHz, ppm, CDCl₃) δ 3,40 (s, 3H) ;3,52-3,62 (m, 2H); 3,64-3,84 (m, 6H); 3,87-4,00 (m, 2H); 4,19-4,30 (m, 2H); 7,13 (d, 2H, *J* = 8,7); 7,22-7,41 (m, 6H) ; 7,44 (d, 2H, *J* = 8,7) ; 8,14 (bd, 2H, *J* = 7,9).

### Synthèse du 9-(4-(2-Méthoxyéthoxy)phényl)-3-nitro-9H-carbazole

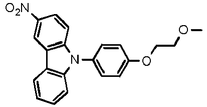

Dans un ballon bicol adapté à un réfrigérant, du 3-nitro carbazole (20 mmol ; 4,25 g), de la poudre de cuivre (5,10 g ; 4 eq), du carbonate de potassium (22,5 g ; 8 eq) du 18-Crown-6 (0,32 g ; 0,06 eq) et du 1-iodo-4-(2-méthoxyéthoxy)benzène (6,7 g ; 1,2 eq) ont été insérés. Le milieu réactionnel a été dégazé, placé sous atmosphère inerte et 50 mL d'orthodichlorobenzène ont été ajoutés. Le mélange a été porté à 180°C. Des chromatographies sur couche mince ont été faites pour suivre l'évolution de la réaction (éluant n-pentane/éther diéthylique 6:4). À la fin de la réaction, le mélange a été filtré sur Celite et le filtrat obtenu a été distillé sous pression réduite pour enlever le solvant. Le résidu a été purifié par chromatographie sur colonne de silice. Un solide jaune a été obtenu avec un rendement de 70%.

**RMN ¹H** (300 MHz, ppm, Acetone D₆) δ 3,41 (s, 3H) ; 3,76-3,84 (m, 2H); 4,25-4,33 (m, 2H); 7,25-7,33 (m, 2H); 7,34-7,43 (m, 2H); 7,43 (d, 1H, *J* = 8,9); 7,52-7,62 (m, 3H); 8,34 (dd, 1H, *J* = 8,9/2.2); 8,46 (bd, 1H, *J* = 7,6) ; 9,18 (d, 1H, *J* = 2,2).

### Synthèse du 9-(4-(2-(2-Ethoxyéthoxy)éthoxy)phényl)-3-nitro-9H-carbazole

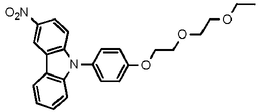

Dans un ballon bicol adapté à un réfrigérant, du 3-nitro carbazole (20 mmol ; 4,25 g), de la poudre de cuivre (5,10 g ; 4 eq), du carbonate de potassium (22,5 g ; 8 eq) du 18-Crown-6 (0,32 g ; 0,06 eq) et du 1-iodo-4-(2-(2-(2-methoxyethoxy)ethoxy)ethoxy)benzene (8,09 g, 1,2 eq) ont été insérés. Le milieu réactionnel a été dégazé, placé sous atmosphère inerte et 50 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice. Un solide jaune a été obtenu avec un rendement de 61,7%.

**RMN ¹H** (300 MHz, ppm, CDCl₃) δ 1,24 (t, 3H, *J* = 7,0) ; 3,57 (q, 2H, *J* = 7,0) ; 3,63-3,70 (m, 2H) ; 3,74-3,81 (m, 2H) ; 3,91-4,00 (m, 2H) ; 4,22-4,30 (m, 2H) ; 7,13-7,20 (m, 2H) ; 7,30 (d, 1H, *J =* 8,9) ; 7,31-7,45 (m, 4H) ; 7,46-7,54 (m, 1H) ; 7,19 (bd, 1H, *J =* 7,7) ; 8,30 (dd, 1H, *J* = 8,9/2,2) ; 9,06 (d, 1H, *J* = 2,2).

### Synthèse du 9-(4-(2-(2-(2-Méthoxyéthoxy)éthoxy)éthoxy)phényl)-3-nitro-9H-carbazole

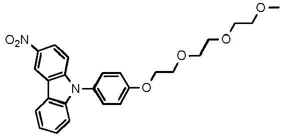

Dans un ballon bicol adapté à un réfrigérant, du 3-nitro carbazole (20 mmol ; 4,25 g), de la poudre de cuivre (5,10 g ; 4 eq), du carbonate de potassium (22,5 g ; 8 eq) du 18-Crown-6 (0,32 g ; 0,06 eq) et du 1-iodo-4-(2-(2-(2-méthoxyéthoxy)éthoxy)éthoxy)benzène (8,10 g ; 1,2 eq) ont été insérés. Le milieu réactionnel a été dégazé, placé sous atmosphère inerte et 50 mL d'ortho-dichlorobenzène ont été ajoutés. Le mélange a été porté à 180°C. Des chromatographies sur couche mince ont été faites pour suivre l'évolution de la réaction (éluant n-pentane/acétate d'éthyle 6:4). À la fin de la réaction, le mélange a été filtré sur Celite et le filtrat obtenu a été distillé sous pression réduite pour enlever le solvant. Le résidu a été purifié par chromatographie sur colonne de silice. Un solide jaune a été obtenu avec un rendement de 83,5 %.

**RMN ¹H** (300 MHz, ppm, CDCl₃) δ 3,29 (s, 3H) ; 3,42-3,76 (m, 8H) ; 3,87-394 (m, 2H) ; 4,26-4,34 (s, 2H); 7,24-7,32 (m, 2H); 7,34-7,46 (m, 3H); 7,60-7,62 (m, 3H); 8,33 (dd, 1H, *J* = 9,1/2,3); 8,42 (d, 1H, *J* = 7,9) ; 9,16 (d, 1H, *J* = 2,3).

### Synthèse du 3-Bromo-9-(4-(2-(2-éthoxyéthoxy)éthoxy)phényl)-9H-carbazole

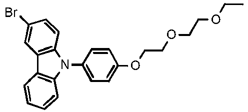

Dans un ballon bicol, du 9-(4-(2-(2-éthoxyéthoxy)éthoxy)phenyl)-9H-carbazole (5,2 mmole, 1,95 g) et 26 mL de chloroforme ont été insérés. Le mélange a été agité jusqu'à dissolution du soluté, puis du N-bromosuccinimide (0,94 g, 1 eq) a été ajouté en une fois. Des chromatographies sur couche mince ont été faites pour suivre l'évolution de la réaction. À la fin de la réaction, le solvant a été évaporé et le résidu a été chromatographié sur colonne de silice. Un liquide clair très visqueux a été obtenu avec un rendement de 92,6 %.

**RMN ¹H** (300 MHz, ppm, CDCl₃) δ 1,24 (t, 3H, *J* = 6,9) ; 3,57 (q, 2H, *J* = 7,0) ; 3,62-3,69 (m, 2H) ; 3,73-3,81 (m, 2H) ; 3,90-3,98 (m, 2H) ; 4,20-4,28 (m, 2H) ; 7,08-7,15 (m, 2H) ; 7,18 (d, 1H, *J =* 8,6) ; 7,26-7,43 (m, 5H) ; 7,46 (dd, 1H, *J =* 8,6/1,8) ; 8,08 (d, 1H, *J =* 7,7) ; 8,23 (d, 1H, *J* = 1,8).

### Synthèse du 3-Bromo-9-(4-(2-(2-(2-méthoxyéthoxy)éthoxy)éthoxy)phényl)-9H-carbazole

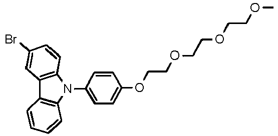

Dans un ballon bicol, du 9-(4-(2-(2-(2-méthoxyéthoxy)éthoxy)éthoxy)phényl)-9*H*-carbazole (5,20 mmole, 2,10 g) et 26 mL de chloroforme ont été insérés. Le mélange a été agité jusqu'à dissolution du soluté, puis du N-bromosuccinimide (0,94 g, 1 eq) a été ajouté en une fois. Des chromatographies sur couche mince ont été faites pour suivre l'évolution de la réaction. À la fin de la réaction, le solvant a été évaporé et le résidu a été chromatographié sur colonne de silice. Un liquide clair très visqueux a été obtenu avec un rendement de 95 %.

**RMN ¹H** (300 MHz, ppm, CDCl₃) δ 3,40 (s, 3H) ; 3,54-3,61 (m, 2H); 3,65-3,75 (m,4H); 3,76-3,82 (m, 2H); 3,89-3,97 (m, 2H); 4,90-4,27 (m, 2H); 7,08-716 (m, 2H) ; 7,18 (d, 1H, *J* = 8,7) ; 7,24-7,43 (m, 5H) ; 7,46 (dd, 1H, *J* = 8,8/2,0) ; 8,05-8,11 (m, 1H) ; 8,23 (d, 1H, *J* = 2,0).

### Synthèse du 3-lodo-9-(4-(2-méthoxyéthoxy)phényl)-9H-carbazole

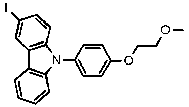

Dans un ballon bicol muni d'un réfrigérant, du 9-(4-(2-méthoxyéthoxy)phényl)-9H-carbazole (5 mmoles ; 1,6 g), de l'acide acétique (50 mL) et de l'iodate de potassium (0,65g ; 0,6 eq)sont insérés. Le mélange a été porté à 100°C pendant une heure puis de l'iodure de potassium (0,67 g ; 0,8 eq) a été ajouté. Le mélange est laissé à l'agitation à 100°C pendant deux heures supplémentaires. Le contenu du réacteur a été plongé dans 100 mL d'eau distillée à 0°C et extrait par du dichlorométhane (4x100mL). Les phases organiques ont été réunies, lavées avec une solution de carbonate de potassium jusqu'à pH légèrement basique, à l'eau (100 mL), puis séchées sur sulfate de magnésium et évaporées. Le composé obtenu avec un rendement de 98% sous forme d'une huile visqueuse marron, est suffisamment pur pour les opérations ultérieures.

**RMN ¹H** (300 MHz, ppm, CDCl₃) δ 3,51 (s, 3H); 3,79-3,87 (m, 2H); 4,17-4,26 (m, 2H); 7,08 (d, 1H, *J* = 8,6) ; 7,09-7,16 (m, 2H) ; 7,24-7,43 (m, 5H) ; 7,63 (dd, 1H, *J* = 8,6/1,6) ; 8,07 (bd, 1H, *J* = 7,7) ; 8,44 (d, 1H, *J* = 1,6).

### Synthèse du 3-lodo-9-(4-(2-(2-éthoxyéthoxy)éthoxy)phényl)-9H-carbazole

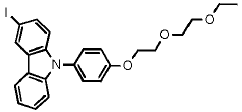

Dans un ballon bicol muni d'un réfrigérant, du 9-(4-(2-(2-éthoxyéthoxy)éthoxy)phényl)-9*H-*carbazole (4 mmoles ; 1,6 g), de l'acide acétique (50 mL) et de l'iodate de potassium (0,51 g ; 0,6 eq) ont été insérés. Le mélange a été porté à 100°C pendant une heure puis de l'iodure de potassium (0,53 g ; 0,8 eq) a été ajouté. Le mélange a été laissé à l'agitation à 100°C pendant deux heures supplémentaires. Le contenu du réacteur a été plongé dans 100 mL d'eau distillée à 0°C et extrait par du dichlorométhane (4x100mL). Les phases organiques ont été réunies, lavées avec une solution de carbonate de potassium jusqu'à pH légèrement basique, à l'eau (100 mL), puis séchées sur sulfate de magnésium et évaporées. Le composé obtenu avec un rendement de 98% sous forme d'une huile translucide, est suffisamment pur pour les opérations ultérieures.

**RMN ¹H** (300 MHz, ppm, CDCl₃) δ 1,24 (t, 3H, *J* = 6,9) ; 3,57 (q, 2H, *J* = 6,9) ; 3,62-3,70 (m, 2H) ; 3,74-3,81 (m, 2H) ; 3,89-3,98 (m, 2H) ; 4,19-4,28 (m, 2H) ; 7,09 (d, 1H, *J =* 8,7) ; 7,09-7,15 (m, 2H) ; 7,24-7,46 (m, 5H) ; 7,63 (dd, 1H, *J* = 8,7/1,7) ; 8,07 (bd, 1H, *J* = 7,7) ; 8,43 (d, 1H, *J* = 1,7).

### Synthèse du 3-Iodo-9-(4-(2-(2-(2-méthoxyéthoxy)éthoxy)éthoxy)phényl)-9H-carbazole

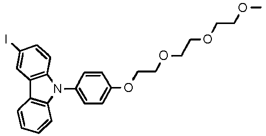

Dans un ballon bicol muni d'un réfrigérant, du 9-(4-(2-(2-(2-méthoxyéthoxy)éthoxy)éthoxy)phényl)-9*H*-carbazole (5 mmoles ; 2,03 g), de l'acide acétique (50 mL) et de l'iodate de potassium (0,64 g ; 0,6 eq) ont été insérés. Le mélange a été porté à 100°C pendant une heure puis de l'iodure de potassium (0,66 g ; 0,8 eq) a été ajouté. Le mélange a été laissé à l'agitation à 100°C pendant deux heures supplémentaires. Le contenu du réacteur a été plongé dans 100 mL d'eau distillée à 0°C et extrait par du dichlorométhane (4x100mL). Les phases organiques ont été réunies, lavées avec une solution de carbonate de potassium jusqu'à pH légèrement basique, à l'eau (100 mL), puis séchées sur sulfate de magnésium et évaporées. Le composé obtenu avec un rendement de 98% sous forme d'une huile visqueuse marron, est suffisamment pur pour les opérations ultérieures.

**RMN ¹H** (300 MHz, ppm, CDCl₃) δ 3,39 (s, 3H) ; 3,53-3,60 (m, 2H) ; 3,65-3,82 (m, 6H); 3,87-3,97 (m, 2H); 4,17-4,27 (m, 2H); 7,08 (d, 1H, *J* = 8,6); 7,09-7,15 (m, 2H) ; 7,23-7,46 (m, 5H) ;7,62 (dd, 1H, *J* = 8,6/1,7) ; 8,06 (bd, 1H, *J* = 7,8) ; 8,43 (d, 1H, *J* = 1,7).

### Synthèse du 9-(2-(2-éthoxyéthoxy)éthyl)-3-iodo-9H-carbazole

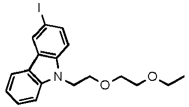

Dans un ballon bicol, ont été ajoutés du 3-iodo-carbazole (1,80 g; 6 mmoles), et du tétrahydrofurane (50 mL). Le mélange a été mis sous agitation et de l'hydrure de sodium (300 mg, 2 éq, 60% dans l'huile) a été ajouté en petites portions. À la fin du dégagement d'hydrogène, du 2-(2-éthoxyéthoxy)éthyl 4-méthylbenzènesulfonate (2,6 g ; 1,5 eq) ont été ajoutés. Le mélange a été agité à température ambiante et des chromatographies sur couche mince ont été faites pour suivre l'évolution de la réaction. Une fois la réaction terminée, le brut a été filtré avec sur Celite. Le filtrat obtenu a été purifié par chromatographie. On obtient une huile jaunâtre très visqueuse avec un rendement de 73%.

**RMN ¹H** (300 MHz, ppm, Acetone D₆) δ 1,00 (t, 3H, J = 7,00) ; 3,28 (q, 2H, J = 7,00) ; 3,34-3,40 (m, 2H) ; 3,44-3,50 (m, 2H) ; 3,88 (t, 2H, J = 5,4) ; 4,57 (t, 2H, 5,4) ; 7,18-7,27 (m, 1H) ; 7,43-7,53 (m, 2H) ; 7,62 (bd, 1H, J = 8,3) ; 7,70 (dd, 1H, J = 8,7/1,8) ; 8,14-8,20 (m, 1H) ; 8,48 (d, 1H, J = 1,8).

### Synthèse du 3-bromo-9-(4-(2-(2-éthoxyéthoxy)éthoxy)phényl)-6-nitro-9H-carbazole

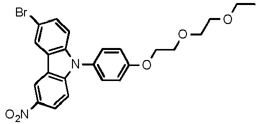

Dans un ballon bicol, du 9-(4-(2-(2-éthoxyéthoxy)éthoxy)phényl)-3-nitro-9*H*-carbazole (2,98 mmoles, 1,25 g) et 6 mL d'acide acétique glacial ont été insérés. Du dibrome (0,16 mL dans 6 mL d'acide acétique glacial, 1,05 eq) a été alors ajouté goutte à goutte. À la fin de l'addition, 5 mL d'acide acétique a été ajouté et le mélange a été laissé à l'agitation pendant 1 h. Le mélange réactionnel a été ensuite versé dans 70 mL d'eau distillée, une pâte précipite. Le mélange a été repris par 100 mL d'acétate d'éthyle, neutralisé par une solution de carbonate de sodium, lavé à l'eau (2x70 mL), séché et évaporé sous vide. Le résidu a été chromatographié sur colonne de silice. Un solide jaune a été obtenu avec un rendement de 99%.

**RMN ¹H** (300 MHz, ppm, CDCl₃) δ 1,23 (t, 3H, *J* = ) ; 3,56 (q, 2H, *J* = ) ; 3,64-3,69 (m, 2H) ; 3,74-3,80 (m, 2H) ; 3,90-3,98 (m, 2H) ; 4,22-4,30 (m, 2H) ;7,12-7,20 (m, 2H) ; 7,20 (d, 1H, *J* = 8,8) ; 7,29 (d, 1H, *J* = 9,0) ; 7,35-7,43 (m, 2H) ; 7,57 (dd, 1H, *J* = 8,8/2,0) ; 8,26-8,37 (m, 2H) ; 9,00 (d, 1H, *J* = 2,0).

### Synthèse du 6-bromo-9-(4-(2-(2-éthoxyéthoxy)éthoxy)phényl)-9H-carbazol-3-amine

La synthèse est réalisable à partir de 9-(4-(2-(2-éthoxyéthoxy)éthoxy)phényl)-9H-carbazol-3-amine et de dibrome selon la même méthode que le 3-bromo-9-(4-(2-(2-éthoxyéthoxy)éthoxy)phényl)-6-nitro-9*H*-carbazole.

### Synthèse du 3-bromo-9-(2-(2-ethoxyethoxy)ethyl)-6-nitro-9H-carbazole

La synthèse est réalisable à partir de 9-(2-(2-ethoxyethoxy)ethyl)-6-nitro-9H-carbazole et de dibrome selon la même méthode que le 3-bromo-9-(4-(2-(2-éthoxyéthoxy)éthoxy)phényl)-6-nitro-9*H*-carbazole. Le 9-(2-(2-éthoxyéthoxy)éthyl)-3-nitro-9H-carbazole étant synthétisable à partir du 3-nitro-carbazole et du 2-(2-éthoxyéthoxy)éthyl 4-méthylbenzènesulfonate de la même manière que le 9-(2-(2-éthoxyéthoxy)éthyl)-3-iodo-9H-carbazole.

### Synthèse du 3-bromo-9-(2-(2-(2-méthoxyéthoxy)éthoxy)éthyl)-9H-carbazole

La synthèse est réalisable à partir de 9-(2-(2-ethoxyethoxy)ethyl)-6-nitro-9H-carbazole et de dibrome selon la même méthode que le 3-bromo-9-(4-(2-(2-éthoxyéthoxy)éthoxy)phényl)-6-nitro-9*H*-carbazole. Le 9-(2-(2-éthoxyéthoxy)éthyl)-3-nitro-9H-carbazole étant synthétisable à partir du 3-nitro-carbazole et du 2-(2-éthoxyéthoxy)éthyl 4-méthylbenzènesulfonate de la même manière que le 9-(2-(2-éthoxyéthoxy)éthyl)-3-iodo-9H-carbazole.

### Synthèse du 9,9-bis(2-(2-éthoxyéthoxy)éthyl)-9H-fluoren-2-amine

La synthèse du **9,9-bis(2-(2-éthoxyéthoxy)éthyl)-9*H*-fluoren-2-amine est réalisée selon la même méthode que le PEG1-FI-NO₂.**

### Synthèse du 9,9-bis(2-méthoxyéthyl)-9H-fluoren-2-amine

La synthèse du 9,9-bis(2-méthoxyéthyl)-9*H*-fluoren-2-amine est réalisée selon la même méthode que le PEG1-FI-NO₂.

### Synthèse du 2-iodo-9,9-bis(2-méthoxyéthyl)-9H-fluorène

La synthèse du 2-iodo-9,9-bis(2-méthoxyéthyl)-9*H*-fluorène est réalisée selon la même méthode que le PEG1-FI-NO₂.

### Synthèse du 9,9-bis(2-méthoxyéthyl)-N²,N²-bis(4-méthoxyphényl)-9H-fluorene-2,7-diamine

La synthèse du 9,9-bis(2-méthoxyéthyl)-*N²,N²*-bis(4-méthoxyphényl)-9*H*-fluorene-2,7-diamine est réalisée selon la même méthode que le PEG1-FI-NO₂.

### Synthèse du 7-iodo-9,9-bis(2-méthoxyéthyl)-N,N-bis(4-méthoxyphényl)-9H-fluoren-2-amine

La synthèse du 7-iodo-9,9-bis(2-méthoxyéthyl)-*N,N*-bis(4-méthoxyphényl)-9*H*-fluoren-2-amine est réalisée selon la même méthode que le PEG1-FI-NO₂.

### Synthèse du 9,9-bis(2-(2-ethoxyethoxy)ethyl)-2-iodo-9H-fluorene

La synthèse du 9,9-bis(2-(2-ethoxyethoxy)ethyl)-2-iodo-9H-fluorene est réalisée selon la même méthode que le PEG1-FI-NO₂.

### Synthèse du 9-(4-(2-méthoxyéthoxy)phényl)-9H-carbazol-3-amine

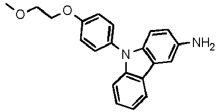

Dans un ballon bicol, du 9-(4-(2-méthoxyéthoxy)phényl)-3-nitro-9*H*-carbazole (3,4 mmoles, 1,25 g), du chlorure d'étain anhydre (4,00 g ; 6 eq) ont été insérés . Les solides ont été dégazés et le réacteur a été placé sous atmosphère inerte. De l'éthanol absolu a été ajouté et le mélange a été replacé sous atmosphère inerte par bullage de diazote pendant 5 min. La température a été ensuite augmentée jusqu'au reflux de l'éthanol. Des chromatographies sur couche mince ont été faites pour suivre l'évolution de la réaction. À la fin de la réaction, Le mélange a été laissé sous agitation jusqu'à retour à température ambiante. Le mélange a été hydrolysé pendant 3h dans une solution d'hydroxyde de sodium (20 mL de solution à 40%). Le mélange a été ensuite repris à l'acétate d'éthyle (75 mL) et filtré sur Celite avec un lavage par 75 mL d'acétate d'éthyle supplémentaire. La phase organique a été extraite, lavée abondamment à l'eau (5x50 mL), séchée sur sulfate de sodium. Le solvant a été évaporé et le résidu a été chromatographié sur colonne de silice. Un liquide violet très foncé et très visqueux a été obtenu avec un rendement de 95 %.

### Synthèse du 9-(4-(2-(2-éthoxyéthoxy)éthoxy)phényl)-9H-carbazol-3-amine

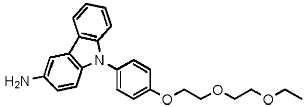

Dans un ballon bicol, du 9-(4-(2-(2-éthoxyéthoxy)éthoxy)phényl)-3-nitro-9*H*-carbazole (3,4 mmoles, 1,44 g), du chlorure d'étain anhydre (4,00 g ; 6 eq) ont été insérés. Les solides ont été dégazés et le réacteur a été placé sous atmosphère inerte. De l'éthanol absolu a été ajouté et le mélange a été replacé sous atmosphère inerte par bullage de diazote pendant 5 min. La température a été ensuite augmentée jusqu'au reflux de l'éthanol. Des chromatographies sur couche mince ont été faites pour suivre l'évolution de la réaction. À la fin de la réaction, Le mélange a été laissé sous agitation jusqu'à retour à température ambiante. Le mélange a été hydrolysé pendant 3h dans une solution d'hydroxyde de sodium (20 mL de solution à 40%). Le mélange a été ensuite repris à l'acétate d'éthyle (75 mL) et filtré sur Celite avec un lavage par 75 mL d'acétate d'éthyle supplémentaire. La phase organique a été extraite, lavée abondamment à l'eau (5x50 mL), séchée sur sulfate de sodium. Le solvant a été évaporé et le résidu a été chromatographié sur colonne de silice. Un liquide violet très foncé et très visqueux a été obtenu avec un rendement de 99 %.

**RMN ¹H** (300 MHz, ppm, CDCl₃) δ 1,24 (t, 3H, *J* = 6,9) ; 3,56 (q, 2H, *J* = 6,9) ; 3,63-3,69 (m, 2H) ;3,74-3,81 (m, 2H) ; 3,90-3,97 (m, 2H) ; 4,19-4,26 (m, 2H) ; 7,00 (dd, 1H, *J* = 8,6/2,0) ; 7,05-7,13 (m, 2H) ; 7,17 (d, 1H, *J* = 8,6) ; 7,16-7,24 (m, 1H) ; 7,27-7,43 (m,4H) ; 7,64 (d, 1H, *J* = 2,0) ;8 ;00-8,10 (m, 1H).

### Synthèse du 9-(4-(2-(2-(2-méthoxyéthoxy)éthoxy)éthoxy)phényl)-9H-carbazol-3-amine

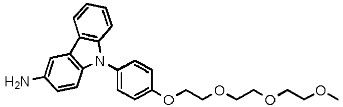

Dans un ballon bicol, du 9-(4-(2-(2-(2-méthoxyéthoxy)éthoxy)éthoxy)phényl)-3-nitro-9*H-*carbazole (3,4 mmoles, 1,55 g), du chlorure d'étain anhydre (4,00 g ; 6 eq) ont été insérés. Les solides ont été dégazés et le réacteur a été placé sous atmosphère inerte. De l'éthanol absolu a été ajouté et le mélange a été replacé sous atmosphère inerte par bullage de diazote pendant 5 min. La température a été ensuite augmentée jusqu'au reflux de l'éthanol. Des chromatographies sur couche mince ont été faites pour suivre l'évolution de la réaction. À la fin de la réaction, Le mélange a été laissé sous agitation jusqu'à retour à température ambiante. Le mélange a été hydrolysé pendant 3h dans une solution d'hydroxyde de sodium (20 mL de solution à 40%). Le mélange a été ensuite repris à l'acétate d'éthyle (75 mL) et filtré sur Celite avec un lavage par 75 mL d'acétate d'éthyle supplémentaire. La phase organique a été extraite, lavée abondamment à l'eau (5x50 mL), séchée sur sulfate de sodium. Le solvant a été évaporé et le résidu a été chromatographié sur colonne de silice. Un liquide violet très foncé et très visqueux a été obtenu avec un rendement de 99 %.

**RMN ¹H** (300 MHz, ppm, CDCl₃) δ 3,39 (s, 3H) ; 3,54-3,61 (m, 2H); 3,65-3,75 (m,4H); 3,76-3,82 (m, 2H); 3,88-3,96 (m, 2H);4,15-4,24 (m, 2H) ; 6,99 (dd, 1H, *J* = 8,7/2,1) ; 7,02-7,09 (m, 2H) ; 7,15 (d, 1H, *J* = 8,7) ; 7,16-7,23 (m, 2H) ; 7,23-7,40 (m, 4H) ; 7,63 (d, 1H, *J* = 2,1) ; 7,99-8,06 (m, 1H).

### Synthèse du 9,9-bis(2-(2-éthoxyéthoxy)éthyl)-2-iodo-9H-fluorène

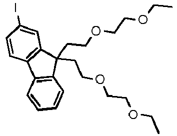

Dans un ballon bicol ont été ajoutés du 2-iodo-fluorène (4,20 g ; 14,3 mmoles), du bromure de tétrabutylammonium (1,48 g, 4,58 mmol) et du 2-(2-éthoxyéthoxy)éthyl 4-méthylbenzènesulfonate (13,76 g, 43 mmol) dans le toluène (35 ml). On ajoute du NaOH (24 g dans 24 ml d'eau). Le mélange a été agité à 75 °C pendant 8 h, puis refroidi et dilué avec du dichlorométhane. La phase organique a été séparée et lavée avec une solution d'acide chlorhydrique, puis avec une solution de chlorure de sodium et séchée sur sulfate de magnésium. Après évaporation des solvants, le résidu a été purifié par chromatographie pour obtenir une huile claire avec un rendement de 60%.

**RMN ¹H** (300 MHz, ppm, Acétone D₆) δ 1.04 (t, 6H, J = 7,0) ; 2,30-2,47 (m, 4H) ; 2,70-2,83 (m, 4H) ; 3,08-3,21 (m, 4H) ; 3,22-3,28 (m, 4H) ; 3,33 (q, 4H, J = 7,0) ; 7,33-7,42 (m, 2H) ; 7,52-7,60 (m, 1H) ; 7,62 (d, 1H, J = 8,0) ; 7,73 (dd, 1H, J = 8,0/1,6) ; 7,78-7,85 (m, 1H) ; 7,97 (d, 1H, J = 1,6).

### Synthèse du 9-(4-(2-(2-Ethoxyéthoxy)éthoxy)phényl)-N,N-bis(4-méthoxyphényl)-9H-carbazol-3-amine (Exemple comparatif)

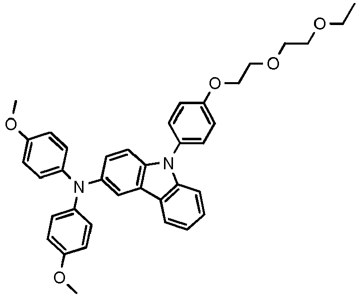

Dans un ballon bicol adapté à un réfrigérant, du 9-(4-(2-(2-éthoxyéthoxy)éthoxy)phényl)-9*H-*carbazol-3-amine (11,5 ml de solution à 0,174 mol.L⁻¹ dans le toluène; 2 mmoles), de la poudre de cuivre (510 mg ; 4 eq), du carbonate de potassium (2,25 g ; 8 eq) du 18-Crown-6 (32 mg ; 0,06 eq) et du 4-iodoanisole (1,43 g, 3 eq) ont été insérés. Le milieu réactionnel a été dégazé, placé sous atmosphère inerte et 5 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice. Une huile marron très visqueuse a été obtenue avec un rendement de 80 %.

**RMN ¹H** (300 MHz, ppm, Acétone-D₆) δ 1,14 (t, 3H, *J* = 7,0) ; 3,49 (q, 2H, *J* = 7,0) ; 3,54-3,61 (m, 2H) ; 3,65-3,72 (m, 2H) ; 3,76 (s, 6H) ; 3,84-3,92 (m, 2H) ; 4,22-4,30 (m, 2H) ; 6,80-6,89 (m, 4H) ; 6,92-7,02 (m, 4H) ; 7,13 (dd, 1H, *J* = 8,8/2,1)7,15-7,32 (m, 6H) ; 7,33-7,42 (m, 1H) ; 7,45-7,54 (m, 2H) ; 7,84 (d, 1H, *J* = 2,1) ; 8,05 (bd, 1H, *J* = 7,6).

### Synthèse du 9-(4-(2-(2-(2-méthoxyéthoxy)éthoxy)éthoxy)phényl)-N,N-bis(4-(2-méthoxyéthoxy)phényl)-9H-carbazol-3-amine (Exemple comparatif)

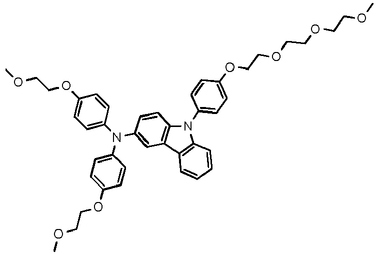

Dans un ballon bicol adapté à un réfrigérant, du 9-(4-(2-(2-(2-méthoxyéthoxy)éthoxy)éthoxy)phényl)-9H-carbazol-3-amine (420 mg; 1,00 mmole), de la poudre de cuivre (510 mg ; 8 eq), du carbonate de potassium (2,21 g ; 16 eq) du 18-Crown-6 (32 mg ; 0,06 eq) et du 1-iodo-4-(2-méthoxyéthoxy)benzène (700 mg, 2,5 eq) ont été insérés. Le milieu réactionnel a été dégazé, placé sous atmosphère inerte et 10 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice.

### Synthèse du 9-(4-(2-(2-Ethoxyéthoxy)éthoxy)phényl)-N,N-bis(4-méthoxyphényl)-6-nitro-9H-carbazol-3-amine (Exemple comparatif)

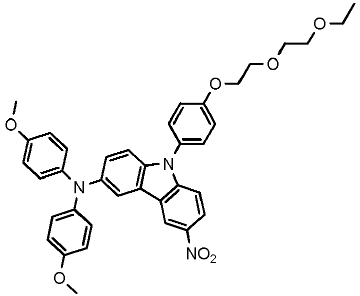

Dans un ballon de Schlenk ont été ajoutés du 6-bromo-9-(4-(2-(2-éthoxyéthoxy)éthoxy)phényl)-9*H*-carbazol-3-amine (2,8 mmoles; 1,40 g), de la bis(4-méthoxyphényl)amine (720 mg ; 1,1 eq), de la tris-terbutylphosphine (115 µL, 0,161 eq) et de l'acétate de palladium (25 mg, 0,04 eq). Après avoir adapté le réacteur à un réfrigérant, le milieu réactionnel a été dégazé et placé sous atmosphère inerte. Du toluène sec (45 mL) a été alors ajouté au mélange de réactifs. Après agitation pendant 15 minutes, à température ambiante, du tert-butoxyde de sodium (1,35 g, 5 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Célite. Le filtrat obtenu a été purifié par colonne chromatographie. Une huile jaunâtre très visqueuse a été obtenue avec un rendement de 11%.

**RMN ¹H** (300 MHz, ppm, Acétone-D₆) δ 1,14 (t, 3H, *J* = 7,0) ; 3,49 (q, 2H, *J* = 7,0) ; 355-3,60 (m, 2H) ; 3,66-3,71 (m,2H) ; 3,78 (s, 6H) ; 3,88-3,92 (m, 2H) ; 4,25-4,32 (m, 2H) ; 6,83-6,92 (m, 4H) ; 6,98-7,07 (m, 4H) ; 7,22 (dd, 1H, *J* = 8,9/2,2) ; 7,22-7,32 (m, 3H) 7,39 (d, 1H, *J =* 9,0); 7,52-7,59 (m, 2H) ; 8,03 (d, 1H, *J* = 2,2) ; 8,28 (dd, 1H, *J* = 9,2/2,2) ; 9,05 (d, 1H, *J =* 2,2).

### Synthèse du 9-(4-(2-(2-(2-méthoxyéthoxy)éthoxy)éthoxy)phényl)-N,N-di(naphthalen-2-yl)-9H-carbazol-3-amine (Exemple comparatif)

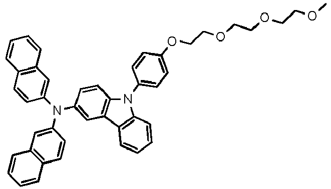

Dans un ballon de Schlenk sont ajoutés de la 9-(4-(2-(2-(2-méthoxyéthoxy)éthoxy)éthoxy)phényl)-9H-carbazol-3-amine (420 mg; 1,00 mmole), du 2-bromonaphthalène (520 mg ; 2,5 eq), de la tris-terbutylphosphine (41 µL ; 0,161 eq) et de l'acétate de palladium (18 mg, 0,08 eq). Après avoir adapté le réacteur à un réfrigérant, le milieu réactionnel est dégazé et placé sous atmosphère inerte. Du toluène sec (16 mL) est alors ajouté au mélange de réactifs. Après agitation pendant 15 minutes, à température ambiante, du tert-butoxyde de sodium (960 mg, 10 eq) est ajouté au mélange réactionnel et celui-ci est porté au reflux du toluène. Des chromatographies sur couche mince sont faites pour suivre l'évolution de la réaction. Une fois la réaction terminée, le brut est filtré avec de la Celite. Le filtrat obtenu est purifié par colonne chromatographie.

### Synthèse du 9-(4-(2-(2-éthoxyéthoxy)éthoxy)phényl)-N-(9-(4-(2-(2-éthoxyéthoxy)éthoxy)phényl)-9H-carbazol-3-yl)-N-(4-(2-(2-(2-méthoxyéthoxy)éthoxy)éthoxy)phényl)-9H-carbazol-3-amine

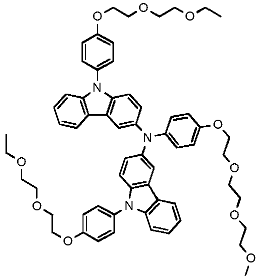

Dans un ballon bicol adapté à un réfrigérant, du (9-(4-(2-(2-éthoxyéthoxy)éthoxy)phényl)-9H-carbazol-3-yl)amine (0,60 g; 0,81 mmole), de la poudre de cuivre (210 mg ; 4 eq), du carbonate de potassium (890 mg ; 8 eq) du 18-Crown-6 (13 mg ; 0,06 eq) et du 1-iodo-4-(2-(2-(2-méthoxyéthoxy)éthoxy)éthoxy)benzène (350 mg, 3 eq) ont été insérés. Le milieu réactionnel a été dégazé, placé sous atmosphère inerte et 2 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice.

### Synthèse du 9-(4-(2-(2-éthoxyéthoxy)éthoxy)phényl)-N-(9-(4-(2-(2-éthoxyéthoxy)éthoxy)phényl)-6-nitro-9H-carbazol-3-yl)-N-(4-méthoxyphényl)-6-nitro-9H-carbazol-3-amine

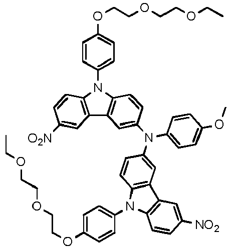

Dans un ballon de Schlenk, de la 4-méthoxyaniline (0,90 mg; 0,73 mmole), du 3-bromo-9-(4-(2-(2-éthoxyéthoxy)éthoxy)phényl)-6-nitro-9H-carbazole (910 mg ; 2,5 eq), de la tris-terbutylphosphine (30 µL ; 0,161 eq) et de l'acétate de palladium (7 mg, 0,08 eq) ont été insérés. Après avoir adapté le réacteur à un réfrigérant, le milieu réactionnel a été dégazé et placé sous atmosphère inerte. Du toluène sec (12 mL) a été alors ajouté au mélange de réactifs. Après agitation pendant 15 minutes, à température ambiante, du tert-butoxyde de sodium (700 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Celite. Le filtrat obtenu a été purifié par colonne chromatographie.

### Synthèse du 9-(4-(2-(2-(2-méthoxyéthoxy)éthoxy)éthoxy)phényl)-N-(9-(4-(2-(2-(2-méthoxyéthoxy)éthoxy)éthoxy)phényl)-9H-carbazol-3-yl)-N-(4-nitrophényl)-9H-carbazol-3-amine(Exemple comparatif)

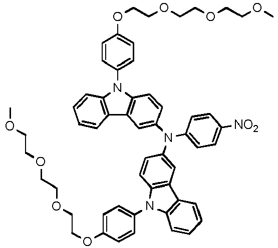

Dans un ballon de Schlenk ont été ajoutés de la 4-nitroaniline (100 mg ; 0,72 mmole), du 3-bromo-9-(4-(2-(2-(2-méthoxyéthoxy)éthoxy)éthoxy)phényl)-9H-carbazole (880 mg ; 2,5 eq), de la tris-terbutylphosphine (30 µL ; 0,161 eq) et de l'acétate de palladium (13 mg, 0,08 eq). Après avoir adapté le réacteur à un réfrigérant, le milieu réactionnel a été dégazé et placé sous atmosphère inerte. Du toluène sec (12 mL) a été alors ajouté au mélange de réactifs. Après agitation pendant 15 minutes, à température ambiante, du tert-butoxyde de sodium (700 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Celite. Le filtrat obtenu a été purifié par colonne chromatographie.

### Synthèse du 9-(2-(2-ethoxyethoxy)ethyl)-N-(9-(2-(2-ethoxyethoxy)ethyl)-6-nitro-9H-carbazol-3-yl)-N-(4-methoxyphenyl)-6-nitro-9H-carbazol-3-amine (Exemple comparatif)

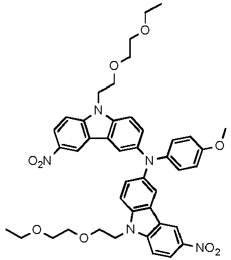

Dans un ballon de Schlenk de la 4-methoxyaniline (94 mg ; 0,76 mmole), du 3-bromo-9-(2-(2-ethoxyethoxy)ethyl)-6-nitro-9H-carbazole (770 mg ; 2,5 eq), de la tris-terbutylphosphine (31 µL ; 0,161 eq) et de l'acétate de palladium (14 mg, 0,08 eq) ont été ajoutés. Après avoir adapté le réacteur à un réfrigérant, le milieu réactionnel a été dégazé et placé sous atmosphère inerte. Du toluène sec (12 mL) a été alors ajouté au mélange de réactifs. Après agitation pendant 15 minutes, à température ambiante, du tert-butoxyde de sodium (730 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Celite. Le filtrat obtenu a été purifié par colonne chromatographie.

### Synthèse du 9-(2-(2-(2-méthoxyéthoxy)éthoxy)éthyl)-N-(9-(2-(2-(2-méthoxyéthoxy)éthoxy)éthyl)-9H-carbazol-3-yl)-N-(4-nitrophényl)-9H-carbazol-3-amine (Exemple comparatif)

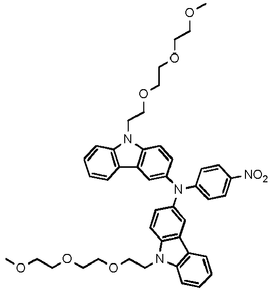

Dans un ballon de Schlenk ont été ajoutés de la 4-nitroaniline (100 mg ; 0,72 mmole), du 3-bromo-9-(2-(2-(2-méthoxyéthoxy)éthoxy)éthyl)-9H-carbazole (710 mg ; 2,5 eq), de la tris-terbutylphosphine (30 µL ; 0,161 eq) et de l'acétate de palladium (13 mg ; 0,08 eq) ont été ajouté. Après avoir adapté le réacteur à un réfrigérant, le milieu réactionnel a été dégazé et placé sous atmosphère inerte. Du toluène sec (12 mL) a été alors ajouté au mélange de réactifs. Après agitation pendant 15 minutes, à température ambiante, du tert-butoxyde de sodium (690 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Celite. Le filtrat obtenu a été purifié par colonne chromatographie.

### Synthèse du 9-(2-(2-éthoxyéthoxy)éthyl)-N-(9-(2-(2-éthoxyéthoxy)éthyl)-6-nitro-9H-carbazol-3-yl)-N-(4-méthoxyphényl)-6-nitro-9H-carbazol-3-amine (Exemple comparatif)

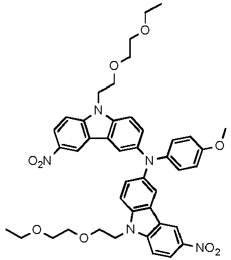

Dans un ballon de Schlenk sont ajoutés de la 4-methoxyaniline (94 mg ; 0,76 mmole), du 3-bromo-9-(2-(2-ethoxyethoxy)ethyl)-6-nitro-9H-carbazole (770 mg ; 2,5 eq), de la tris-terbutylphosphine (31 µL ; 0,161 eq) et de l'acétate de palladium (14 mg, 0,08 eq). Après avoir adapté le réacteur à un réfrigérant, le milieu réactionnel est dégazé et placé sous atmosphère inerte. Du toluène sec (12 mL) est alors ajouté au mélange de réactifs. Après agitation pendant 15 minutes, à température ambiante, du tert-butoxyde de sodium (730 mg, 10 eq) est ajouté au mélange réactionnel et celui-ci est porté au reflux du toluène. Des chromatographies sur couche mince sont faites pour suivre l'évolution de la réaction. Une fois la réaction terminée, le brut est filtré avec de la Celite. Le filtrat obtenu est purifié par colonne chromatographie.

### Synthèse du bis(9-(4-(2-(2-(2-méthoxyéthoxy)éthoxy)éthoxy)phényl)-9H-carbazol-3-yl)amine (Exemple comparatif)

La synthèse du bis(9-(4-(2-(2-(2-méthoxyéthoxy)éthoxy)éthoxy)phényl)-9H-carbazol-3-yl)amine est telle que la synthèse du 9-(4-(2-(2-(2-méthoxyéthoxy)éthoxy)éthoxy)phényl)-N-(9-(4-(2-(2-(2-méthoxyéthoxy)éthoxy)éthoxy)phényl)-9H-carbazol-3-yl)-N-(4-nitrophényl)-9H-carbazol-3-amine sans ajout de 4-nitroaniline.

### Synthèse du 9-(4-(2-(2-éthoxyéthoxy)éthoxy)phényl)-N³,N³-bis(4-méthoxyphényl)-9H-carbazole-3,6-diamine (Exemple comparatif)

La synthèse du 9-(4-(2-(2-éthoxyéthoxy)éthoxy)phényl)-N³,N³-bis(4-méthoxyphényl)-9H-carbazole-3,6-diamine est telle que la synthèse du 9-(4-(2-(2-(2-méthoxyéthoxy)éthoxy)éthoxy)phényl)-N-(9-(4-(2-(2-(2-méthoxyéthoxy)éthoxy)éthoxy)phényl)-9H-carbazol-3-yl)-N-(4-nitrophényl)-9H-carbazol-3-amine sans ajout de 4-nitroaniline et avec deux précurseurs carbazoles différents

### Synthèse du 6-bromo-9-(4-(2-(2-éthoxyéthoxy)éthoxy)phényl)-N,N-bis(4-méthoxyphényl)-9H-carbazol-3-amine (Exemple comparatif)

La synthèse du 6-bromo-9-(4-(2-(2-éthoxyéthoxy)éthoxy)phényl)-N,N-bis(4-méthoxyphényl)-9H-carbazol-3-amine est telle que la synthèse du 9-(4-(2-(2-(2-méthoxyéthoxy)éthoxy)éthoxy)phényl)-N-(9-(4-(2-(2-(2-méthoxyéthoxy)éthoxy)éthoxy)phényl)-9H-carbazol-3-yl)-N-(4-nitrophényl)-9H-carbazol-3-amine sans ajout de 4-nitroaniline et avec deux précurseurs carbazoles différents

### Synthèse de la tris(9-(4-(2-(2-éthoxyéthoxy)éthoxy)phényl)-9H-carbazol-3-yl)amine

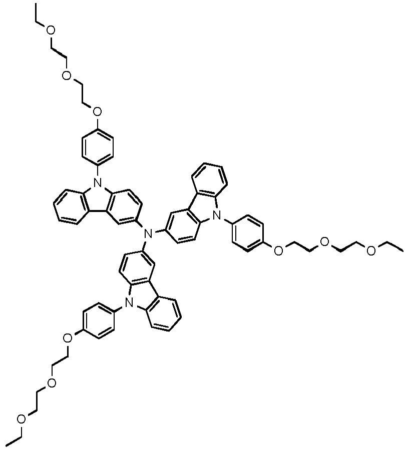

### Méthode 1

Dans un ballon de Schlenk ont été ajoutés de la 9-(4-(2-(2-éthoxyéthoxy)éthoxy)phényl)-9*H-*carbazol-3-amine (1,7 ml de solution à 3,64.10⁻⁴ mol.L⁻¹ dans le toluène; 0,62 mmole), du 3-bromo-9-(4-(2-(2-éthoxyéthoxy)éthoxy)phényl)-9*H*-carbazole (3,20 ml de solution 4,93.10⁻⁴ mol.L⁻¹ dans le toluène; 2,5 eq), de la tris-terbutylphosphine (25 µL ; 0,161 eq) et de l'acétate de palladium (11 mg, 0,08 eq). Après avoir adapté le réacteur à un réfrigérant, le milieu réactionnel a été dégazé et placé sous atmosphère inerte. Du toluène sec (21 mL) a été alors ajouté au mélange de réactifs. Après agitation pendant 15 minutes, à température ambiante, du tert-butoxyde de sodium (590 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Célite. Le filtrat obtenu a été purifié par colonne chromatographie. Un composé solide vert très sombre a été obtenu avec un rendement de 32%.

### Méthode 2

Dans un ballon bicol adapté à un réfrigérant, de la 9-(4-(2-(2-éthoxyéthoxy)éthoxy)phényl)-9*H*-carbazol-3-amine (1,7 ml de solution à 3,64.10⁻⁴ mol.L⁻¹ dans le toluène; 0,62 mmole), de la poudre de cuivre (160 mg ; 4 eq), du carbonate de potassium K₂CO₃ (680 mg ; 8 eq), du 18-Crown-6 (10 mg ; 0,06 eq) et du 3-iodo-9-(4-(2-(2-éthoxyéthoxy)éthoxy)phényl)-9*H-*carbazole (780 mg, 2,5 eq) ont été insérés. Le milieu réactionnel a été dégazé, placé sous atmosphère inerte et mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice. Un composé verdâtre a été obtenu avec un rendement de 65 %.

**RMN ¹H** (300 MHz, ppm, Acétone-D₆) δ 1,12 (t, 9H, *J* =7,0) ; 3,47 (q, 6H, *J* = 7,0) ; 3,52-3,59 (m, 6H) ; 3,63-3,70 (m, 6H) ; 3,82-3,90 (m, 6H) ; 4,18-4,26 (m, 6H) ; 7,05-7,14 (m, 4H) ; 7,26-7,38 (m, 20H) ; 7,44-7,53 (m, 6H) ; 7,92 (bd, 3H, *J* = 7,8) ; 7,96 (bs, 3H).

### Synthèse de la tris(9-(4-(2-(2-(2-méthoxyéthoxy)éthoxy)éthoxy)phényl)-9H-carbazol-3-yl)amine

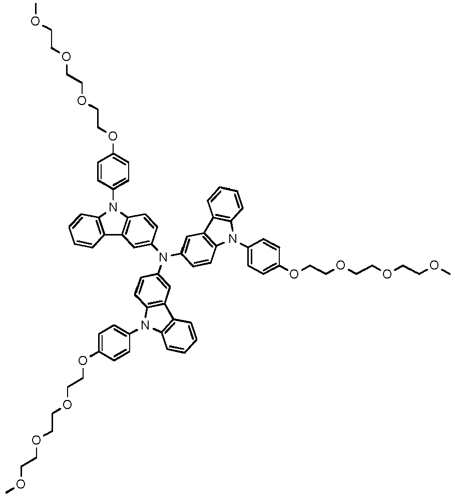

Dans un ballon de Schlenk ont été ajoutés de la 9-(4-(2-(2-(2-méthoxyéthoxy)éthoxy)éthoxy)phényl)-9H-carbazol-3-amine (473 µl de solution à 13,1.10⁻³ mol.L⁻¹ dans le toluène; 0,62 mmole), du 3-bromo-9-(4-(2-(2-éthoxyéthoxy)éthoxy)phényl)-9*H*-carbazole (1,81 ml de solution 1,05.10⁻³ mol.L⁻¹ dans le toluène; 3 eq), de la tris-terbutylphosphine (25 µL ; 0,161 eq) et de l'acétate de palladium (11 mg, 0,08 eq). Après avoir adapté le réacteur à un réfrigérant, le milieu réactionnel a été dégazé et placé sous atmosphère inerte. Du toluène sec (21 mL) a été alors ajouté au mélange de réactifs. Après agitation pendant 15 minutes, à température ambiante, du tert-butoxyde de sodium (590 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Célite. Le filtrat obtenu a été purifié par colonne chromatographie. Un composé jaune verdâtre a été obtenu avec un rendement de 18%.

**RMN ¹H** (300 MHz, ppm, CDCl₃) δ 3,26 (s, 9H); 3,42-3,48 (m, 2H); 3,53-3,63 (m, 12H); 3,63-3,70 (m, 6H); 3,80-3,89 (m, 6H); 4,15-4,24 (m, 6H); 7,34-7,38 (m ; 22H) ; 7,39-7,51 (m, 8H) ; 7,87 (d, 3H, *J* = 7,6).

### Synthèse du 9-(2-(2-éthoxyéthoxy)éthyl)-N-(9-(2-(2-éthoxyéthoxy)éthyl)-9H-carbazol-3-yl)-N-(9-(4-(2-méthoxyéthoxy)phényl)-9H-carbazol-3-yl)-9H-carbazol-3-amine

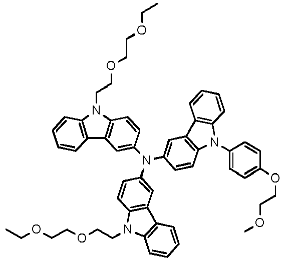

Dans un ballon bicol adapté à un réfrigérant, on insère du 9-(4-(2-méthoxyéthoxy)phényl)-9*H*-carbazol-3-amine (200 mg ; 0,6 mmole), de la poudre de cuivre (310 mg ; 8 eq), du carbonate de potassium (1,40 g ; 16 eq), du 18-Crown-6 (30 mg ; 0,12 eq) et du 9-(2-(2-éthoxyéthoxy)éthyl)-3-iodo-9*H*-carbazole (1,10 g ; 4 eq). Le milieu réactionnel a été dégazé, placé sous atmosphère inerte et 25 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice. On obtient une huile marron très visqueuse avec un rendement de 67 %.

**RMN ¹H** (300 MHz, ppm, Acetone D₆) δ 1,00 (t, 6H, J = 7,0) ; 3,32 (q, 4H, J = 7,0) ; 3,38 (s, 3H) ; 3,38-3,44 (m, 4H) ; 3,46-3,53 (m, 4H) ; 3,71-3,79 (m, 2H) ; 3,87 (t, 4H, J = 5,4) ; 4,17-4,25 (m, 2H) ; 4,41-4,63 (bs, 4H) ; 7,00-7,14 (m, 4H) ; 7,33-7,42 (m, 10H) ; 7,44-7,62 (m, 8H) ; 7,87 (d, 2H, J = 7,6) ; 7,91 (bd, 1H, 7,7).

### Synthèse du 9-(2-(2-éthoxyéthoxy)éthyl)-N,N-bis(9-(4-(2-(2-(2-méthoxyéthoxy)éthoxy)éthoxy)phényl)-9H-carbazol-3-yl)-9H-carbazol-3-amine

Dans un ballon bicol adapté à un réfrigérant, du bis(9-(4-(2-(2-(2-méthoxyéthoxy)éthoxy)éthoxy)phényl)-9H-carbazol-3-yl)amine (390 mg; 0,47 mmole), de la poudre de cuivre (120 mg ; 4 eq), du carbonate de potassium (520 mg ; 8 eq) du 18-Crown-6 (7 mg ; 0,06 eq) et du 9-(2-(2-éthoxyéthoxy)éthyl)- 3-iodo-9H-carbazole (250 mg, 1,3 eq) ont été ajoutés. Le milieu réactionnel a été dégazé, placé sous atmosphère inerte et 5 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice.

### Synthèse du 9-(4-(2-(2-éthoxyéthoxy)éthoxy)phényl)-N-(9-(4-(2-(2-éthoxyéthoxy)éthoxy)phényl)-9H-carbazol-3-yl)-N-(9-(4-(2-(2-(2-méthoxyéthoxy)éthoxy)éthoxy)phényl)-9H-carbazol-3-yl)-9H-carbazol-3-amine

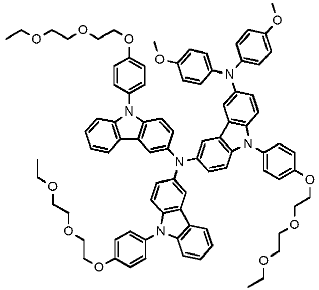

Dans un ballon de Schlenk de la 9-(4-(2-(2-(2-méthoxyéthoxy)éthoxy)éthoxy)phényl)-9H-carbazol-3-amine (198 mg ; 0,47 mmole), du 3-bromo-9-(4-(2-(2-éthoxyéthoxy)éthoxy)phényl)-9H-carbazole (540 mg ; 2,5 eq), de la tris-terbutylphosphine (19 µL ; 0,161 eq) et de l'acétate de palladium (8 mg, 0,08 eq) ont été ajoutés. Après avoir adapté le réacteur à un réfrigérant, le milieu réactionnel a été dégazé et placé sous atmosphère inerte. Du toluène sec (10 mL) a été alors ajouté au mélange de réactifs. Après agitation pendant 15 minutes, à température ambiante, du tert-butoxyde de sodium (450 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Celite. Le filtrat obtenu a été purifié par colonne chromatographie.

### Synthèse du 9-(4-(2-(2-éthoxyéthoxy)éthoxy)phényl)-N³,N³-bis(9-(4-(2-(2-éthoxyéthoxy)éthoxy)phényl)-9H-carbazol-3-yl)-N⁶,N⁶-bis(4-méthoxyphényl)-9H-carbazole-3,6-diamine

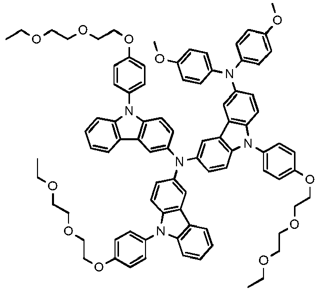

Dans un ballon de Schlenk s de la 9-(4-(2-(2-éthoxyéthoxy)éthoxy)phényl)-N³,N³-bis(4-méthoxyphényl)-9H-carbazole-3,6-diamine (292 mg ; 0,47 mmole), du 3-bromo-9-(4-(2-(2-éthoxyéthoxy)éthoxy)phényl)-9H-carbazole (540 mg ; 2,5 eq), de la tris-terbutylphosphine (19 µL ; 0,161 eq) et de l'acétate de palladium (8 mg, 0,08 eq) ont été insérés. Après avoir adapté le réacteur à un réfrigérant, le milieu réactionnel a été dégazé et placé sous atmosphère inerte. Du toluène sec (10 mL) a été alors ajouté au mélange de réactifs. Après agitation pendant 15 minutes, à température ambiante, du tert-butoxyde de sodium (450 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Célite. Le filtrat obtenu a été purifié par colonne chromatographie.

### Synthèse du N³-(6-(bis(4-méthoxyphényl)amino)-9-(4-(2-(2-éthoxyéthoxy)éthoxy)phényl)-9H-carbazol-3-yl)-9-(4-(2-(2-éthoxyéthoxy)éthoxy)phényl)-N³-(9-(4-(2-(2-éthoxyéthoxy)éthoxy)phényl)-6-nitro-9H-carbazol-3-yl)-N⁶,N⁶-bis(4-méthoxyphényl)-9H-carbazole-3,6-diamine

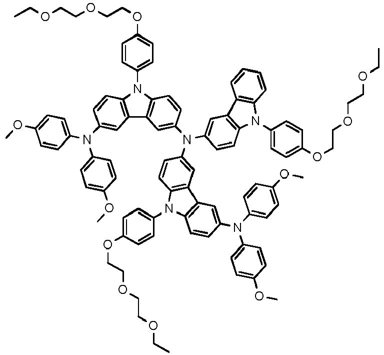

Dans un ballon de Schlenk de la 9-(4-(2-(2-éthoxyéthoxy)éthoxy)phényl)-9H-carbazol-3-amine (184 mg; 0,47 mmole), du 6-bromo-9-(4-(2-(2-éthoxyéthoxy)éthoxy)phényl)-N,N-bis(4-méthoxyphényl)-9H-carbazol-3-amine (800 mg ; 2,5 eq), de la tris-terbutylphosphine (19 µL ; 0,161 eq) et de l'acétate de palladium (8 mg, 0,08 eq) ont été insérés. Après avoir adapté le réacteur à un réfrigérant, le milieu réactionnel a été dégazé et placé sous atmosphère inerte. Du toluène sec (10 mL) a été alors ajouté au mélange de réactifs. Après agitation pendant 15 minutes, à température ambiante, du tert-butoxyde de sodium (450 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Celite. Le filtrat obtenu a été purifié par colonne chromatographie.

### Synthèse du N³,N³-bis(6-(bis(4-méthoxyphényl)amino)-9-(4-(2-(2-éthoxyéthoxy)éthoxy)phényl)-9H-carbazol-3-yl)-9-(4-(2-(2-éthoxyéthoxy)éthoxy)phényl)-N⁶,N⁶-bis(4-méthoxyphényl)-9H-carbazole-3,6-diamine

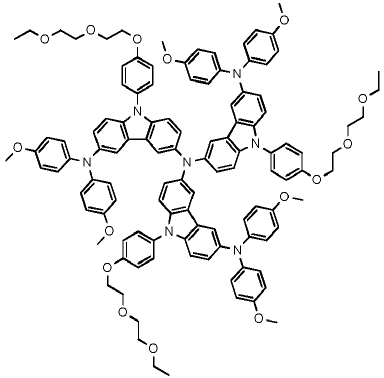

Dans un ballon de Schlenk ont été ajoutés de la 9-(4-(2-(2-éthoxyéthoxy)éthoxy)phényl)-*N³*,*N³*-bis(4-méthoxyphényl)-9*H*-carbazole-3,6-diamine (292 mg; 0,47 mmole), du 6-bromo-9-(4-(2-(2-éthoxyéthoxy)éthoxy)phényl)-*N*,*N*-bis(4-méthoxyphényl)-9*H*-carbazol-3-amine (810 mg ; 2,5 eq), de la tris-terbutylphosphine (19 µL ; 0,161 eq) et de l'acétate de palladium (8 mg, 0,08 eq). Après avoir adapté le réacteur à un réfrigérant, le milieu réactionnel a été dégazé et placé sous atmosphère inerte. Du toluène sec (10 mL) a été alors ajouté au mélange de réactifs. Après agitation pendant 15 minutes, à température ambiante, du tert-butoxyde de sodium (450 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Celite. Le filtrat obtenu a été purifié par colonne chromatographie.

### Synthèse du N-(9,9-bis(2-(2-éthoxyéthoxy)éthyl)-9H-fluoren-2-yl)-9-(4-(2-(2-éthoxyéthoxy)éthoxy)phényl)-N-(9-(4-(2-(2-éthoxyéthoxy)éthoxy)phényl)-9H-carbazol-3-yl)-9H-carbazol-3-amine

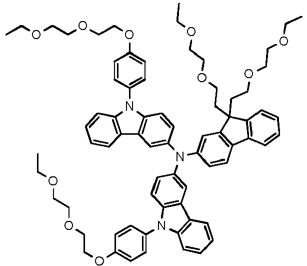

Dans un ballon de Schlenk ont été ajoutés de la 9,9-bis(2-(2-éthoxyéthoxy)éthyl)-9*H*-fluoren-2-amine (215 mg ; 0,47 mmole), du 3-bromo-9-(4-(2-(2-éthoxyéthoxy)éthoxy)phényl)-9*H-*carbazole (490 mg ; 2,5 eq), de la tris-terbutylphosphine (19 µL ; 0,161 eq) et de l'acétate de palladium (9 mg, 0,08 eq). Après avoir adapté le réacteur à un réfrigérant, le milieu réactionnel a été dégazé et placé sous atmosphère inerte. Du toluène sec (10 mL) a été alors ajouté au mélange de réactifs. Après agitation pendant 15 minutes, à température ambiante, du tert-butoxyde de sodium (460 mg, 10 eq) a été ajouté au mélange réactionnel et celui-ci a été porté au reflux du toluène. Des chromatographies sur couche mince ont été faites pour suivre l'évolution de la réaction. Une fois la réaction terminée, le brut a été filtré avec de la Celite. Le filtrat obtenu a été purifié par colonne chromatographie.

### Synthèse du tris(9,9-bis(2-méthoxyéthyl)-9H-fluoren-2-yl)amine

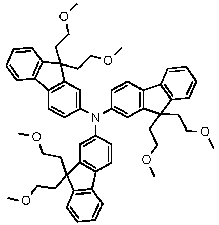

Dans un ballon bicol adapté à un réfrigérant, du 9,9-bis(2-méthoxyéthyl)-9*H*-fluoren-2-amine (280 mg ; 0,94 mmoles), de la poudre de cuivre (480 mg ; 8 eq), du carbonate de potassium (2,09 g ; 16 eq) du 18-Crown-6 (30 mg ; 0,12 eq) et du 2-iodo-9,9-bis(2-méthoxyéthyl)-9*H-*fluorène (960 mg, 2,5 eq) ont été insérés. Le milieu réactionnel a été dégazé, placé sous atmosphère inerte et 20 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice.

### Synthèse du N²,N²-bis(7-(bis(4-méthoxyphényl)amino)-9,9-bis(2-méthoxyéthyl)-9H-fiuoren-2-yl)-9,9-bis(2-méthoxyéthyl)-N⁷,N⁷-bis(4-méthoxyphényl)-9H-fluorene-2,7-diamine

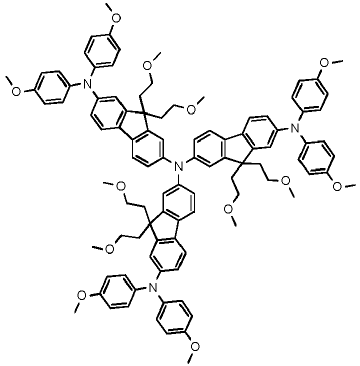

Dans un ballon bicol adapté à un réfrigérant, du 9,9-bis(2-méthoxyéthyl)-*N²,N²*-bis(4-méthoxyphényl)-9*H*-fluorene-2,7-diamine (276 mg; 0,53 mmoles), de la poudre de cuivre (270 mg ; 8 eq), du carbonate de potassium (1,16 g ; 16 eq), du 18-Crown-6 (17 mg ; 0,12 eq) et du 7-iodo-9,9-bis(2-méthoxyéthyl)-*N*,*N*-bis(4-méthoxyphényl)-9*H*-fluoren-2-amine (840 mg, 2,5 eq) ont été insérés. Le milieu réactionnel a été dégazé, placé sous atmosphère inerte et 5 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice.

### Synthèse du tris(9,9-bis(2-(2-éthoxyéthoxy)éthyl)-9H-fluoren-2-yl)amine

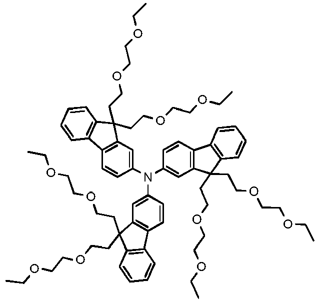

Dans un ballon bicol adapté à un réfrigérant, du 9,9-bis(2-(2-ethoxyethoxy)ethyl)-9H-fluoren-2-amine (170 mg; 0,41 mmoles), de la poudre de cuivre (210 mg ; 8 eq), du carbonate de potassium (920 mg ; 16 eq), du 18-Crown-6 (13 mg ; 0,12 eq) et du 9,9-bis(2-(2-ethoxyethoxy)ethyl)-2-iodo-9H-fluorene (420 mg, 2,5 eq) ont été insérés. Le milieu réactionnel a été dégazé, placé sous atmosphère inerte et 8 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice.

### Synthèse du N¹,N¹,N⁴,N⁴-tétrakis(9-(4-(2-méthoxyéthoxy)phényl)-9H-carbazol-3-yl)benzène-1,4-diamine (Exemple comparatif)

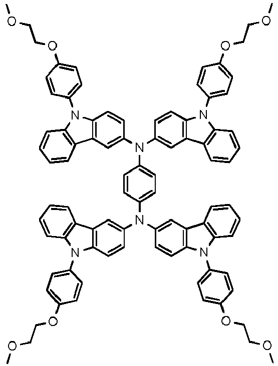

Dans un ballon bicol adapté à un réfrigérant, du benzène-1,4-diamine (49 mg; 0,45 mmole), de la poudre de cuivre (460 mg ; 16 eq), du carbonate de potassium (2,00 g ; 32 eq) du 18-Crown-6 (29 mg ; 0,24 eq) et du 3-iodo-9-(4-(2-méthoxyéthoxy)phényl)-9*H*-carbazole (1 g, 5 eq) ont été insérés. Le milieu réactionnel a été dégazé, placé sous atmosphère inerte et 18 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice.

### Synthèse du N¹,N¹,N⁴,N⁴-tétrakis(9-(2-(2-éthoxyéthoxy)éthyl)-9H-carbazol-3-yl)benzène-1,4-diamine (Exemple comparatif)

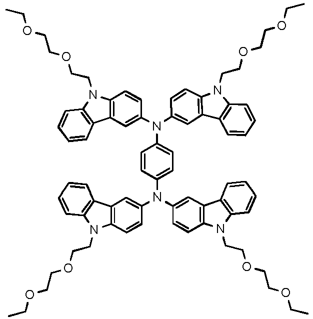

Dans un ballon bicol adapté à un réfrigérant, du benzène-1,4-diamine (53 mg; 0,49 mmole), de la poudre de cuivre (490 mg ; 16 eq), du carbonate de potassium (2,15 g ; 32 eq) du 18-Crown-6 (32 mg ; 0,24 eq) et du 9-(2-(2-éthoxyéthoxy)éthyl)-3-iodo-9*H*-carbazole (PEG2-Cz-I) (1 g, 5 eq) ont été insérés. Le milieu réactionnel a été dégazé, placé sous atmosphère inerte et 20 mL d'ortho-dichlorobenzène ont été ajoutés. Le mélange a été porté à 180°C. Des chromatographies sur couche mince ont été faites pour suivre l'évolution de la réaction. À la fin de la réaction, le mélange a été filtré sur Celite et le filtrat obtenu a été distillé sous pression réduite pour enlever le solvant. Le résidu a été purifié par chromatographie sur colonne de silice.

**RMN ¹H** (300 MHz, ppm, Acetone D₆) δ 0,99 (t, 12H, J = 7,0) ; 2,85 (s, 8H) ; 3,29 (q, 8H, J = 7,0); 3,34-3,42 (m, 8H); 3,44-3,53 (m, 8H); 3,79-3,93 (m, 8H) ; 6,87-7,02 (m, 2H) ; 7,05-7,20 (m, 3H) ; 7,55-7,77 (m, 17H) ; 7,89 (d, 4H, J = 7,7) ; 8,04 (d, 2H, J = 7,6) ; 8,11 (d, 2H, J = 1,9).

### Exemple 2 : Caractérisation des composés obtenus

Les matériaux synthétisés ont été caractérisés dans le but de les utiliser dans les DSSC comme semiconducteurs transporteurs de trous HTMs (Hole Transporting Materials) en combinaison avec un colorant commercial (le **D102)**. Les caractérisations réalisées concernent les propriétés thermiques, optiques et électrochimiques.

La figure suivante présente les structures des semiconducteurs qui ont été caractérisés et utilisés dans un dispositif photovoltaïque.

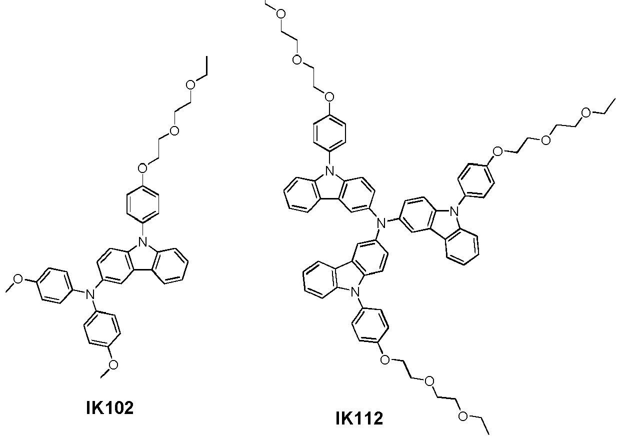

### A. Les propriétés thermiques

Les propriétés thermiques des semiconducteurs organiques ont été étudiées grâce à des mesures d'analyse thermogravimétrique (ATG) et de calorimétrie différentielle à balayage (DSC). La première mesure a permis d'estimer la température de dégradation (T_{d}) des molécules et de renseigner sur leur stabilité thermique. Cette mesure a été définie à 5% de perte de masse lors de cette analyse. La température de dégradation du composé IK 112 est de 367 °C.

### B. Propriétés optiques

Les propriétés optiques des semiconducteurs organiques ont été étudiées pour évaluer les zones d'absorption des verres moléculaires synthétisés et pour les comparer à l'absorption du colorant **D102**. Pour ce faire, des mesures d'absorption UV-Vis ont été effectuées sur des solutions des HTMs (dans le THF à 10⁻⁵ M) et du colorant **D102** (à 10⁻⁴ M dans l'acétonitrile).

Les absorptions maximales des HTMs **IK102** et **IK112** se sont situés entre 290 nm et 320 nm alors que le colorant D102 a absorbé entre 368 nm et 501 nm. Les HTMs n'ont pas absorbé dans la même zone que le colorant. En termes d'absorption, les verres moléculaires de la présente invention ont été compatibles avec le colorant **D102**. Par ailleurs, les coefficients d'absorption molaire des HTMs ont été de 10000 L.mol⁻¹.cm⁻¹ et 60000 L.mol⁻¹.cm⁻¹ respectivement pour **IK102** et **IK112**.

| | λ (nm) | ε (L.mol⁻¹.cm⁻¹) |
|---|---|---|
| IK102 | 307 | 10000 |
| IK112 | 292, 321 | 66000 |
| D102 | 368, 501 | - |

### C. Propriétés électrochimiques

Le comportement électrochimique des verres moléculaires synthétisés a été étudié. Pour pouvoir les utiliser comme HTMs des DSSC, ces matériaux doivent avoir la capacité de s'oxyder de manière réversible sans dégradation. Les niveaux énergétiques HOMO des matériaux IK102 et IK112 se sont situés au-dessus de celui du colorant (IK102 - 4.7 eV ; IK112 -5.1 eV ; D102 - 5.2 eV). Les matériaux ont été donc aptes à régénérer le colorant. La valeur du niveau HOMO du D102 a été prise dans la littérature (H. Melhem et al Adv. Energy Mater., 2011; 1: 908-916).

| | E¹ₒₓ(mV) | E¹_{red} (mV) | E¹ _{1/2} (mV) | E _{HOMO} (eV) |
|---|---|---|---|---|
| IK102 | 83 | 23 | 52 | - 4.7 |
| IK112 | - 290 | - 346 | - 318 | - 5.1 |
| D102 | | | | - 5.2 |

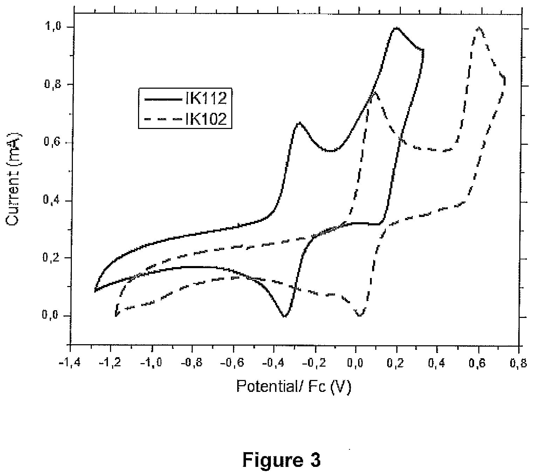

Le résultat est disponible sur la figure 3 qui indique le potentiel électrochimique des deux verres moléculaires testés (IK102 et IK112).

### Exemple 3 : Utilisation des semiconducteurs en dispositif photovoltaïque

Les semiconducteurs organiques **IK102** et **IK112** ont été utilisés comme des transporteurs de trous dans un dispositif photovoltaïque de type **ssDSSC** (solid state Dye Sensitized Solar Cell) en combinaison avec le colorant commercial **D102**.

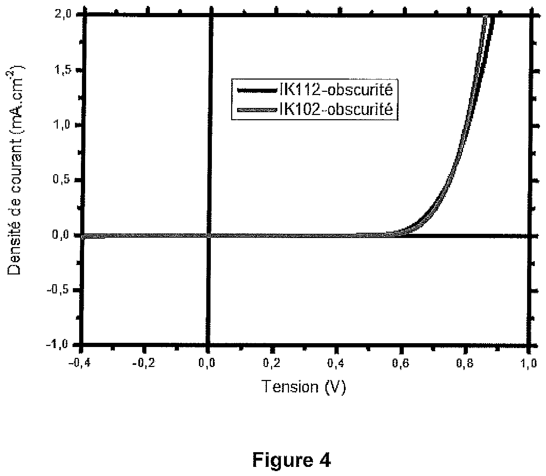

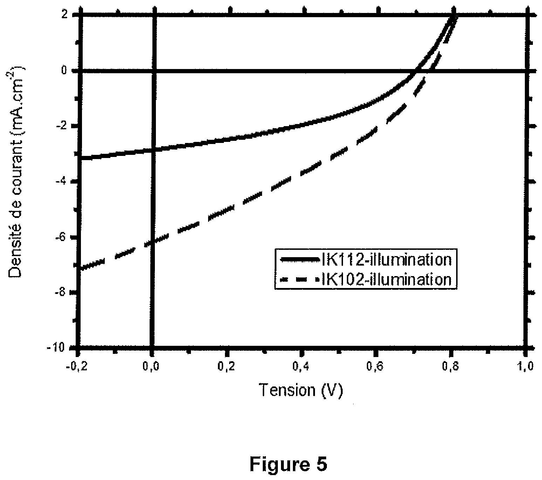

Le procédé de fabrication des **ssDSSC** est inspiré de la littérature L. Schmidt-Mende et al. , Adv. Mater., 2005 ; 17 : 813-815. La composition de la couche active a été la suivante : TiO₂/D102/HTM. Les résultats obtenus ont été présentés sur les Figures 4 et 5.

| | **J_{sc} (mA.cm⁻²)** | **V_{oc} (V)** | **FF (%)** | **Rdt. (%)** |
|---|---|---|---|---|
| **IK102** | 6.15 | 0.73 | 33 | 1.49 |
| **IK112** | 2.84 | 0.69 | 41 | 0.80 |

Dans l'obscurité, la figure 4 est la caractéristique J-V d'une diode avec une tension seuil d'environ 0.6 V. Sous illumination, un courant électrique apparait. Cela met en évidence l'effet photovoltaïque. Des rendements de 1.49% et 0.80% ont été respectivement obtenus grâce aux HTMs **IK102** et **IK112**. Ces résultats ont été obtenus sans optimisation de la fabrication des dispositifs photovoltaïques par rapport aux nouveaux semiconducteurs **IK102** et **IK112**.

## Revendications

1. Triarylamine de formule (IV) : 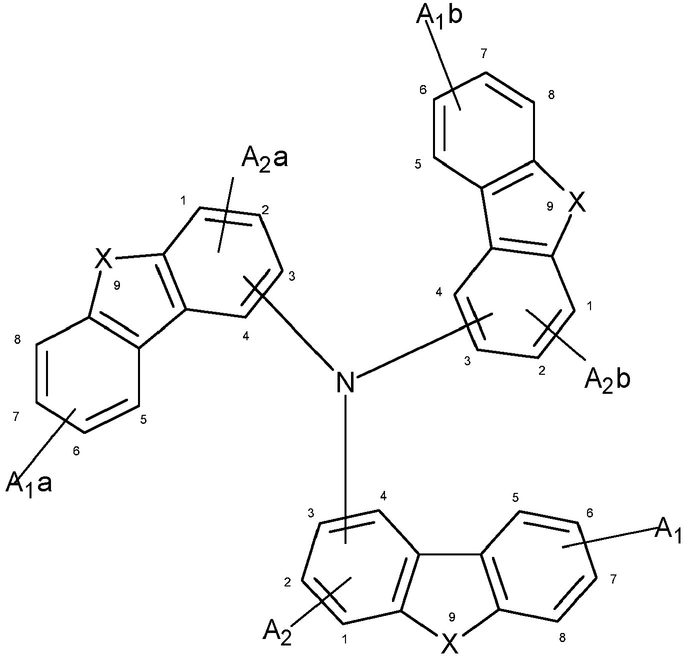 dans laquelle :
X est choisi dans le groupe constitué de :
**X¹** : N-[(CH₂)ₙ-O)]ₚ-R² ;
**X²** : N-R₁-{O-[(CH₂)ₙ-O)]ₚ-R²}ₘ ;
**X³** : CH-[(CH₂)ₙ-O)]ₚ-R² ;
**X⁴** : CH-R¹-{O-[(CH₂)ₙ-O)]ₚ-R²}ₘ ;
**X⁵** : C-{[(CH₂)ₙ-O)]ₚ-R²}₂ ; et
**X⁶** : C-**(**R¹-{O-[(CH₂)ₙ-O)]ₚ-R²}ₘ)₂ ; où :
R¹ est choisi dans le groupe constitué d'aryle ou hétéroaryle, de préférence aryle ;
n est un entier de 1 à 6, notamment 2 ou 3, en particulier 2 ;
p = est un entier de 1 à 20;
m est un entier de 1 à 6, notamment de 1 à 3, en particulier 1 ;
et lorsque m est supérieur à 1, les valeurs de p et de n dans chaque groupement -{O-[(CH₂)ₙ-O)]ₚ- peuvent être identiques ou différentes les unes des autres.
R² est un alkyle en C₁ à C₁₂; A¹, situé de préférence en position 6 ou 7 du cycle, est choisi dans le groupe constitué de H ;alkyle en C₁ à C₇ ; alcényle en C₂ à C₇ ; alcynyle en C₂ à C₇ ; aryle, hétéroaryle ; cycloalkyle en C₃ à C₈ ; NO₂, CF₃ ; CN ; N₃ ; F ; Cl ; NR^{a}R^{b}; OR^{c} ; SR^{c} ; C(=O)R^{d} ; ou un chromophore ; notamment H ou un chromophore ; où :
R^{a} est choisi dans le groupe constitué de H ; alkyle en C₁ à C₇ ; aryle, hétéroaryle, cycloalkyle en C₃ à C₈ ; ou R^{a} et R^{b} forment ensemble un (C₃-C₇)hétérocyclyle ;
R^{b} est choisi dans le groupe constitué de alkyle en C₁ à C₇ ; aryle, hétéroaryle, cycloalkyle en C₃ à C₈ ; ou R^{a} et R^{b} forment ensemble un (C₃-C₇)hétérocyclyle ;
R^{c} est choisi dans le groupe constitué de alkyle en C₁ à C₇ ; aryle, hétéroaryle, cycloalkyle en C₃ à C₈; C(=O)-alkyle en C₁ à C₇ ; C(=O)-aryle, C(=O)-hétéroaryle, C(=O)-cycloalkyle en C₃ à C₈ ;
R^{d} représente alkyle en C₁ à C₇ ; aryle, hétéroaryle, cycloalkyle en C₃ à C₈ ; NR^{a}R^{b}; OR^{c} ; SR^{c} ;
A², situé de préférence en position 2 ou 3 du cycle est choisi dans le groupe constitué de H ; alkyle en C₁ à C₇ ; alcényle en C₂ à C₇ ; alcynyle en C₂ à C₇ ; aryle, hétéroaryle ; cycloalkyle en C₃ à C₈ ; NO₂, CF₃ ; CN ; N₃ ; F ; Cl ; NR^{a}R^{b} ; OR^{c} ; SR^{c} ; C(=O)R^{d} ; ou un chromophore ; notamment H ; où :
R^{a} est choisi dans le groupe constitué de H ; alkyle en C₁ à C₇ ; aryle, hétéroaryle, cycloalkyle en C₃ à C₈ ; ou R^{a} et R^{b} forment ensemble un (C₃-C₇)hétérocyclyle ;
R^{b} est choisi dans le groupe constitué de alkyle en C₁ à C₇ ; aryle, hétéroaryle, cycloalkyle en C₃ à C₈ ; ou R^{a} et R^{b} forment ensemble un (C₃-C₇)hétérocyclyle ;
R^{c} est choisi dans le groupe constitué de alkyle en C₁ à C₇ ; aryle, hétéroaryle, cycloalkyle en C₃ à C₈ ; C(=O)-alkyle en C₁ à C₇ ; C(=O)-aryle, C(=O)-hétéroaryle, C(=O)-cycloalkyle en C₃ à C₈ ;
R^{d} représente alkyle en C₁ à C₇ ; aryle, hétéroaryle, cycloalkyle en C₃ à C₈ ; NR^{a}R^{b}; OR^{c} ; SR^{c}.
et A^{1a}, A^{2a}, A^{1b} et A^{2b} ont la signification donnée pour A¹ et A²,
A^{2a}, A^{2b} et A² d'une part et A^{1a}, A¹ et A^{1b} d'autre part pouvant être identiques ou différents l'un de l'autre.

2. Triarylamine de formule (IV) selon la revendication 1, dans laquelle A^{2a}, A^{2b} et A² sont identique entre eux et représentent de préférence H et A^{1a}, A¹ et A^{1b} sont identique entre deux.

3. Triarylamine selon l'une des revendications 1 ou 2, dans laquelle A¹, A^{1a} et A^{1b} représentent H, un aryle ou un chromophore, notamment choisi dans le groupe constitué des structures suivantes : 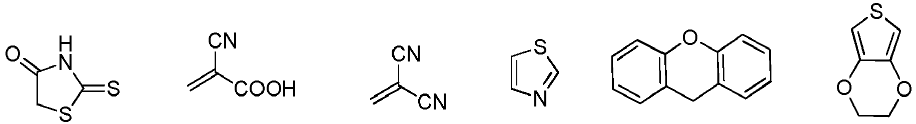 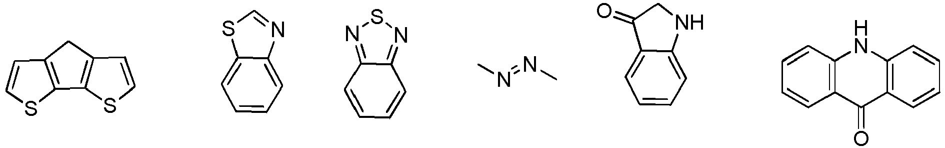 A¹, A^{1a} et A^{1b} représentant notamment H.

4. Triarylamine de formule (IV) selon la revendication 1 : 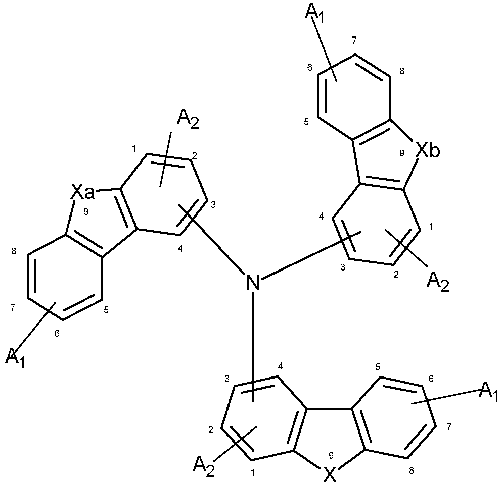 dans laquelle A¹ représente H, A² représente H, et X, Xa et Xb sont choisis parmi l'une des combinaisons suivantes :
| X | Xa | Xb |
|---|---|---|
| **X¹** : N-[(CH₂)ₙ-O)]ₚ-R² | **X¹** : N-[(CH₂)ₙ-O)]ₚ-R² | **X¹** : N-[(CH₂)ₙ-O)]ₚ-R² |
| **X¹** : N-[(CH₂)ₙ-O)]ₚ-R² | **X²** : N-R₁-{O-[(CH₂)ₙ-O)]ₚ-R²}ₘ ; | **X²** : N-R₁-{O-[(CH₂)ₙ-O)]ₚ-R²}ₘ |
| **X¹** : N-[(CH₂)ₙ-O)]ₚ-R² | **X³** : CH-[(CH₂)ₙ-O)]ₚ-R² | **X³** : CH-[(CH₂)ₙ-O)]ₚ-R² |
| **X¹** : N-[(CH₂)ₙ-O)]ₚ-R² | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X¹** : N-[(CH₂)ₙ-O)]ₚ-R² | **X⁵** : C-{[(CH₂)ₙ-O)]ₚ-R²}₂ | **X⁵** : C-{[(CH₂)ₙ-O)]ₚ-R²}₂ |
| **X¹** : N-[(CH₂)ₙ-O)]ₚ-R² | **X⁶** : C-**(**R¹-{O-[(CH₂)ₙ-O)]ₚ-R²}ₘ)₂ | **X⁶** : C-(R¹-{O-[(CH₂)ₙ-O)]ₚ-R²}ₘ)₂ |
| **X²** : N-R₁-{O-[(CH₂)ₙ-O)]ₚ-R²}ₘ | **X²** : N-R₁-{O-[(CH₂)ₙ-O)]ₚ-R²}ₘ | **X²** : N-R₁-{O-[(CH₂)ₙ-O)]ₚ-R²}ₘ |
| **X²** : N-RHO-[(CH₂)ₙ-O)]ₚ-R²}ₘ | **X¹** : N-[(CH₂)ₙ-O)]ₚ-R² | **X¹** : N-[(CH₂)ₙ-O)]ₚ-R² |
| **X²** : N-R₁-{O-[(CH₂)ₙ-O)]ₚ-R²}ₘ | **X³** : CH-[(CH₂)ₙ-O)]ₚ-R² | **X³** : CH-[(CH₂)ₙ-O)]ₚ-R² |
| **X²** : N-R₁-{O-[(CH₂)ₙ-O)]ₚ-R²}ₘ | **X⁴ :** N-R¹-{O-[(CH₂)ₙ-O)]ₚ-R²}ₘ | **X⁴ :** CH-R¹-{O-[(CH₂)ₙ-O)]ₚ-R²}ₘ |
| **X²** : N-R₁-{O-[(CH₂)ₙ-O)]ₚ-R²}ₘ | **X⁵** : C-{[(CH₂)ₙ-O)]ₚ-R²}₂ | **X⁵** : C-{[(CH₂)ₙ-O)]ₚ-R²}₂ |
| **X²** : N-R₁-{O-[(CH₂)ₙ-O)]ₚ-R²}ₘ | **X⁶** : C-**(**R¹-{O-[(CH₂)ₙ-O)]ₚ-R²}ₘ)₂ | **X⁶** : C-(R¹-{O-[(CH₂)ₙ-O)]ₚ-R²}ₘ)₂ |
| **X³** : CH-[(CH₂)ₙ-O)]ₚ-R² | **X³** : CH-[(CH₂)ₙ-O)]ₚ-R² | **X³** : CH-[(CH₂)ₙ-O)]ₚ-R² |
| **X³** : CH-[(CH₂)ₙ-O)]ₚ-R² | **X¹** : N-[(CH₂)ₙ-O)]ₚ-R² | **X¹** : N-[(CH₂)ₙ-O)]ₚ-R² |
| **X³** : CH-[(CH₂)ₙ-O)]ₚ-R² | **X²** : N-R₁-{O-[(CH₂)ₙ-O)]ₚ-R²}ₘ ; | **X²** : N-R₁-{O-[(CH₂)ₙ-O)]ₚ-R²}ₘ |
| **X³** : CH-[(CH₂)ₙ-O)]ₚ-R² | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X³** : CH-[(CH₂)ₙ-O)]ₚ-R² | **X⁵** : C-{[(CH₂)ₙ-O)]ₚ-R²}₂ | **X⁵** : C-{[(CH₂)ₙ-O)]ₚ-R²}₂ |
| **X³** : CH-[(CH₂)ₙ-O)]ₚ-R² | **X⁶** : C-(R¹-{O-[(CH₂)ₙ-O)]ₚ-R²}ₘ)₂ | **X⁶** : C-(R¹-{O-[(CH₂)ₙ-O)]ₚ-R²}ₘ)₂ |
| **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X⁴ :** CH-R¹-{O-[(CH₂)ₙ-O)]ₚ-R²}ₘ | **X¹** : N-[(CH₂)ₙ-O)]ₚ-R² | **X¹** : N-[(CH₂)ₙ-O)]ₚ-R² |
| **X⁴ :** CH-R¹-{O-[(CH₂)ₙ-O)]ₚ-R²}ₘ | **X²** : N-R₁-{O-[(CH₂)ₙ-O)]ₚ-R²}ₘ ; | **X²** : N-R₁-{O-[(CH₂)ₙ-O)]ₚ-R²}ₘ |
| **X⁴ :** CH-R¹-{O-[(CH₂)ₙ-O)]ₚ-R²}ₘ | **X³** : CH-[(CH₂)ₙ-O)]ₚ-R² | **X³** : CH-[(CH₂)ₙ-O)]ₚ-R² |
| **X⁴ :** CH-R¹-{O-[(CH₂)ₙ-O)]ₚ-R²}ₘ | **X⁵** : C-{[(CH₂)ₙ-O)]ₚ-R²}₂ | **X⁵** : C-{[(CH₂)ₙ-O)]ₚ-R²}₂ |
| **X⁴ :** CH-R¹-{O-[(CH₂)ₙ-O)]ₚ-R²}ₘ | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| **X⁵** : C-{[(CH₂)ₙ-O)]ₚ-R²}₂ | **X⁵** : C-{[(CH₂)ₙ-O)]ₚ-R²}₂ | **X⁵** : C-{[(CH₂)ₙ-O)]ₚ-R²}₂ |
| **X⁵** : C-{[(CH₂)ₙ-O)]ₚ-R²}₂ | **X¹** : N-[(CH₂)ₙ-O)]ₚ-R² | **X¹** : N-[(CH₂)ₙ-O)]ₚ-R² |
| **X⁵** : C-{[(CH₂)ₙ-O)]ₚ-R²}₂ | **X²** : N-R₁-{O-[(CH₂)ₙ-O)]ₚ-R²}ₘ ; | **X²** : N-R₁-{O-[(CH₂)ₙ-O)]ₚ-R²}ₘ |
| **X⁵** : C-{[(CH₂)ₙ-O)]ₚ-R²}₂ | **X³** : CH-[(CH₂)ₙ-O)]ₚ-R² | **X³** : CH-[(CH₂)ₙ-O)]ₚ-R² |
| **X⁵** : C-{[(CH₂)ₙ-O)]ₚ-R²}₂ | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X⁵** : C-{[(CH₂)ₙ-O)]ₚ-R²}₂ | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| **X⁶** : C-(R¹-{O-[(CH₂)ₙ-O)]ₚ-R²}ₘ)₂ | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| **X⁶** : C-(R¹-{O-[(CH₂)ₙ-O)]ₚ-R²}ₘ)₂ | **X¹** : N-[(CH₂)ₙ-O)]ₚ-R² | **X¹** : N-[(CH₂)ₙ-O)]ₚ-R² |
| **X⁶** : C-**(**R¹-{O-[(CH₂)ₙ-O)]ₚ-R²}ₘ)₂ | **X²** : N-R₁-{O-[(CH₂)ₙ-O)]ₚ-R²}ₘ ; | **X²** : N-R₁-{O-[(CH₂)ₙ-O)]ₚ-R²}ₘ |
| **X⁶** : C-**(**R¹-{O-[(CH₂)ₙ-O)]ₚ-R²}ₘ)₂ | **X³** : CH-[(CH₂)ₙ-O)]ₚ-R² | **X³** : CH-[(CH₂)ₙ-O)]ₚ-R² |
| **X⁶** : C-**(**R¹-{O-[(CH₂)ₙ-O)]ₚ-R²}ₘ)₂ | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X⁶** : C-**(**R¹-{O-[(CH₂)ₙ-O)]ₚ-R²}ₘ)₂ | **X⁵** : C-{[(CH₂)ₙ-O)]ₚ-R²}₂ | **X⁵** : C-{[(CH₂)ₙ-O)]ₚ-R²}₂ |

5. Utilisation de composés pi-conjugués selon l'une des revendications 1 à 4 en tant que semi-conducteurs, notamment des semi-conducteurs organiques, ou de colorants.

6. Dispositif optoélectronique tels qu'une cellule solaire photovoltaïque à colorant photosensible (DSSC), une cellule solaire photovoltaïque à quantum dot, une cellule solaire photovoltaïque hybride, une cellule solaire photovoltaïque organique, une cellule solaire photovoltaïque à pérovskite, une diode électroluminescente organique (OLEDs) et un transistor organique à effet de champ (OFETs) comprenant au moins un composé selon l'une des revendications 1 à 4.

## Patentansprüche

1. Triarylamin der Formel (IV) 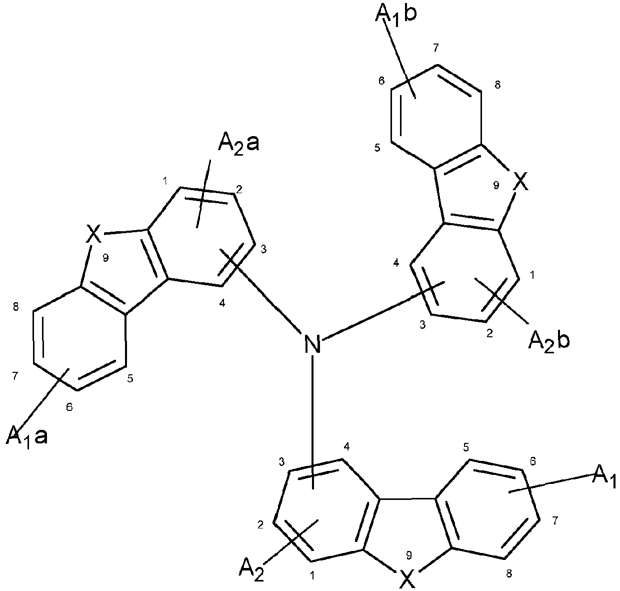 wobei:
X ausgewählt ist aus der Gruppe bestehend aus:
X¹ : N-[(CH₂)ₙ-O)]ₚ-R²;
X² : N-R₁-{O-[(CH₂)ₙ-O)]ₚ-R²}m;
X³ : CH-[(CH₂)ₙ-O)]ₚ-R²
X⁴ : CH-R¹-{O-[(CH₂)ₙ-O)]ₚ-R²}ₘ;
X⁵ : C-{[(CH₂)ₙ-O)]ₚ-R²}₂; und
X⁶ : C-(R¹-{O-[(CH₂)ₙ-O)]ₚ-R²}ₘ)₂; wobei:
R¹ ausgewählt ist aus der Gruppe bestehend aus Aryl oder Heteroaryl, vorzugsweise Aryl;
n für eine ganze Zahl von 1 bis 6 steht, insbesondere 2 oder 3, bevorzugt 2;
p = für eine ganze Zahl von 1 bis 20 steht;
m für eine ganze Zahl von 1 bis 6 steht, insbesondere 1 bis 3, bevorzugt 1;
und wenn m größer als 1 ist, können die Werte von p und n in jeder Gruppe -{O-[(CH₂)ₙ-O)]ₚ- gleich oder verschieden voneinander sein.
R² für ein C₁-C₁₂-Alkyl steht; A¹, vorzugsweise in der Ringposition 6 oder 7, ausgewählt ist aus der Gruppe bestehend aus H; C₁-C₇-Alkyl; C₂-C₇-Alkenyl; C₂-C₇-Alkinyl; Aryl, Heteroaryl; C₃-C₈-Cycloalkyl; NO₂, CF₃; CN; N₃; F; Cl; NR^{a}R^{b}; OR^{c}; SR^{c}; C(=O)R^{d}; oder einem Chromophor; insbesondere H oder einem Chromophor; wobei:
R^{a} ausgewählt ist aus der Gruppe bestehend aus H; C₁-C₇-Alkyl; Aryl, Heteroaryl, C₃-C₈-Cycloalkyl; oder R^{a} und R^{b} zusammen ein (C₃-C₇)Heterocyclyl bilden;
R^{b} ausgewählt ist aus der Gruppe bestehend aus C₁-C₇-Alkyl; Aryl, Heteroaryl, C₃-C₈-Cycloalkyl; oder R^{a} und R^{b} zusammen ein (C₃-C₇)-Heterocyclyl bilden;
R^{c} ausgewählt ist aus der Gruppe bestehend aus C₁-C₇-Alkyl; Aryl, Heteroaryl, C₃-C₈-Cycloalkyl; C(=O)-C₁-C₇-Alkyl; C(=O)-Aryl, C(=O)-Heteroaryl, C(=O)-C₃-C₈-Cycloalkyl;
R^{d} für C₁-C₇-Alkyl; Aryl, Heteroaryl, C₃-C₈-Cycloalkyl; NR^{a}R^{b}; OR^{c}; SR^{c} steht;
A², vorzugsweise in der Ringposition 2 oder 3, ausgewählt ist aus der Gruppe bestehend aus H; C₁-C₇-Alkyl; C₂-C₇-Alkenyl; C₂-C₇-Alkinyl; Aryl, Heteroaryl; C₃-C₈-Cycloalkyl; NO₂, CF₃; CN; N₃; F; Cl; NR^{a}R^{b}; OR^{c}; SR^{c}; C(=O)R^{d}; oder einem Chromophor; insbesondere H; wobei:
R^{a} ausgewählt ist aus der Gruppe bestehend aus H; C₁-C₇-Alkyl; Aryl, Heteroaryl, C₃-C₈-Cycloalkyl; oder R^{a} und R^{b} zusammen ein (C₃-C₇)-Heterocyclyl bilden;
R^{b} ausgewählt ist aus der Gruppe bestehend aus C₁-C₇-Alkyl; Aryl, Heteroaryl, C₃-C₈-Cycloalkyl; oder R^{a} und R^{b} zusammen ein (C₃-C₇)-Heterocyclyl bilden;
R^{c} ausgewählt ist aus der Gruppe bestehend aus C₁-C₇-Alkyl; Aryl, Heteroaryl, C₃-C₈-Cycloalkyl; C(=O)-C₁-C₇-Alkyl; C(=O)-Aryl, C(=O)-Heteroaryl, C(=O)-C₃-C₈-Cycloalkyl;
R^{d} für C₁-C₇-Alkyl; Aryl, Heteroaryl, C₃-C₈-Cycloalkyl; NR^{a}R^{b}; OR^{c}; SR^{c} steht;
und A^{1a}, A^{2a}, A^{1b} und A^{2b} die für A¹ und A² angegebene Bedeutung haben,
wobei A^{2a}, A^{2b} und A² einerseits und A^{1a}, A¹ und A^{1b} andererseits gleich oder verschieden voneinander sein können.

2. Triarylamin der Formel (IV) nach Anspruch 1, wobei A^{2a}, A^{2b} und A² untereinander gleich sein können und vorzugsweise für H stehen, und A^{1a}, A¹ und A^{1b} untereinander gleich sind.

3. Triarylamin nach einem der Ansprüche 1 oder 2, wobei A¹, A^{1a} und A^{1b} für H, Aryl oder einen Chromophor stehen, insbesondere ausgewählt aus der Gruppe bestehend aus den folgenden Strukturen: 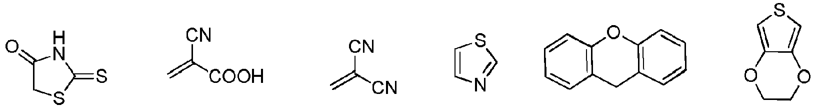 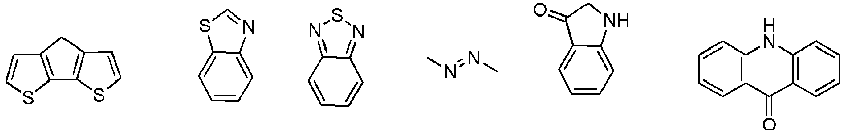 wobei A¹, A^{1a} und A^{1b} insbesondere für H stehen.

4. Triarylamin der Formel (IV) nach Anspruch 1: 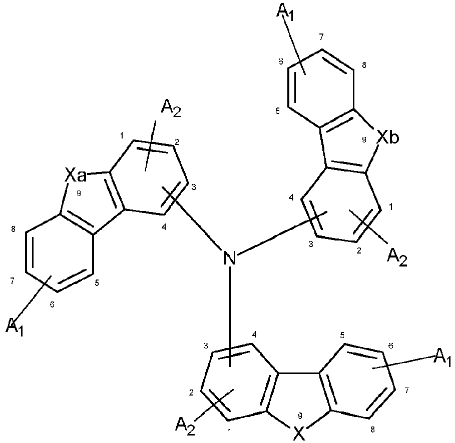 wobei A¹ für H steht, A² für H steht, und X, Xa und Xb ausgewählt sind aus einer der folgenden Kombinationen.
| X | Xa | Xb |
|---|---|---|
| **X¹** : N-[(CH₂)ₙ-O)]ₚ-R² | **X¹** : N-[(CH₂)ₙ-O)]ₚ-R² | **X¹** : N-[(CH₂)ₙ-O)]ₚ-R² |
| **X¹** : N-[(CH₂)ₙ-O)]ₚ-R² | **X²** : N-R₁-{O-[(CH₂)ₙ-O)]ₚ-R²}ₘ ; | **X²** : N-R₁-{O-[(CH₂)ₙ-O)]ₚ-R²}ₘ |
| **X¹** : N-R₁-{O-[(CH₂)ₙ-O)]ₚ- | **X³ :** N-R₁-{O-[(CH₂)ₙ-O)]ₚ-R² | **X³ :** N-R₁-{O-[(CH₂)ₙ-O)]ₚ-R² |
| **X¹** : N-[(CH₂)ₙ-O)]ₚ-R² | **X⁴** : CH-R¹-{O-[(CH₂)ₙ-O)]ₚ-R²}ₘ | **X⁴ :** CH-R¹-{O-[(CH₂)ₙ-O)]ₚ-R²}ₘ |
| **X¹ :** N-[(CH₂)ₙ-O)]ₚ-R² | **X⁵** : C-{[(CH₂)ₙ-O)]ₚ-R²}₂ | **X⁵ :** C-{[(CH₂)ₙ-O)]ₚ-R²}₂ |
| **X¹** : N-[(CH₂)ₙ-O)]ₚ-R² | **X⁶** : C-(R¹-{O-[(CH₂)ₙ-O)]ₚ-R²}ₘ)₂ | **X⁶** : C-(R¹-{O-[(CH₂)ₙ-O)]ₚ-R²}ₘ)₂ |
| **X² :** N-R₁-{O-[(CH₂)ₙ-O)]ₚ-R²}ₘ | **X²** : N-R₁-{O-[(CH₂)ₙ-O)]ₚ-R²}ₘ | **X²** : N-R₁-{O-[(CH₂)ₙ-O)]ₚ-R²}ₘ |
| **X² :** N-R₁-{O-[(CH₂)ₙ-O)]ₚ-R²}ₘ | **X¹** : N-[(CH₂)ₙ-O)]ₚ-R² | **X¹** : N-[(CH₂)ₙ-O)]ₚ-R² |
| **X² :** N-R₁-{O-[(CH₂)ₙ-O)]ₚ-R²}ₘ | **X³ :** N-R₁-{O-[(CH₂)ₙ-O)]ₚ-R² | **X³ :** N-R₁-{O-[(CH₂)ₙ-O)]ₚ-R² |
| **X² :** N-R₁-{O-[(CH₂)ₙ-O)]ₚ-R²}ₘ | **X⁴ :** CH-R¹-{O-[(CH₂)ₙ-O)]ₚ-R²}ₘ | **X⁴** : CH-R¹-{O-[(CH₂)ₙ-O)]ₚ-R²}ₘ |
| **X² :** N-R₁-{O-[(CH₂)ₙ-O)]ₚ-R²}ₘ | **X⁵** : C-{[(CH₂)ₙ-O)]ₚ-R²}₂ | **X⁵** : C-{[(CH₂)ₙ-O)]ₚ-R²}₂ |
| **X² :** N-R₁-{O-[(CH₂)ₙ-O)]ₚ-R²}ₘ | **X⁶ :** C-**(**R¹-{O-[(CH₂)ₙ-O)]ₚ-R²}ₘ)₂ | **X⁶ :** C-**(**R¹-{O-[(CH₂)ₙ-O)]ₚ-R²}ₘ)₂ |
| **X³ :** CH-[(CH₂)ₙ-O)]ₚ-R² | **X³ :** CH-[(CH₂)ₙ-O)]ₚ-R² | **X³ :** CH-[(CH₂)ₙ-O)]ₚ-R² |
| **X³ :** CH-[(CH₂)ₙ-O)]ₚ-R² | **X¹** : N-[(CH₂)ₙ-O)]ₚ-R² | **X¹** : N-[(CH₂)ₙ-O)]ₚ-R² |
| **X³ :** CH-[(CH₂)ₙ-O)]ₚ-R² | **X²** : N-R₁-{O-[(CH₂)ₙ-O)]ₚ-R²}ₘ ; | **X² :** N-R₁-{O-[(CH₂)ₙ-O)]ₚ-R²}ₘ |
| **X³ :** CH-[(CH₂)ₙ-O)]ₚ-R² | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X³ :** N-R₁-{O-[(CH₂)ₙ-O)]ₚ-R² | **X⁵** : C-{[(CH₂)ₙ-O)]ₚ-R²}₂ | **X⁵** : C-{[(CH₂)ₙ-O)]ₚ-R²}₂ |
| **X³ :** N-R₁-{O-[(CH₂)ₙ-O)]ₚ-R² | **X⁶ :** C-**(**R¹-{O-[(CH₂)ₙ-O)]ₚ-R²}ₘ)₂ | **X⁶ :** C-**(**R¹-{O-[(CH₂)ₙ-O)]ₚ-R²}ₘ)₂ |
| **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X⁴ :** CH-R¹-{O-[(CH₂)ₙ-O)]ₚ-R²}ₘ | **X¹** : N-[(CH₂)ₙ-O)]ₚ-R² | **X¹** : N-[(CH₂)ₙ-O)]ₚ-R² |
| **X⁴ :** CH-R¹-{O-[(CH₂)ₙ-O)]ₚ-R²}ₘ | **X²** : N-R₁-{O-[(CH₂)ₙ-O)]ₚ-R²}ₘ ; | **X² :** N-R₁-{O-[(CH₂)ₙ-O)]ₚ-R²}ₘ |
| **X⁴ :** CH-R¹-{O-[(CH₂)ₙ-O)]ₚ-R²}ₘ | **X³ :** CH-[(CH₂)ₙ-O)]ₚ-R² | **X³ :** CH-[(CH₂)ₙ-O)]ₚ-R² |
| **X⁴** : CH-R¹-{O-[(CH₂)ₙ-O)]ₚ-R²}ₘ | **X⁵** : C-{[(CH₂)ₙ-O)]ₚ-R²}₂ | **X⁵** : C-{[(CH₂)ₙ-O)]ₚ-R²}₂ |
| **X⁴ :** CH-R¹-{O-[(CH₂)ₙ-O)]ₚ-R²}ₘ | **X⁶ :** C-**(**R¹-{O-[(CH₂)ₙ-O)]ₚ-R²}ₘ)₂ | **X⁶ :** C-**(**R¹-{O-[(CH₂)ₙ-O)]ₚ-R²}ₘ)₂ |
| **X⁵ :** C-{[(CH₂)ₙ-O)]ₚ-R²}₂ | **X⁵ :** C-{[(CH₂)ₙ-O)]ₚ-R²}₂ | **X⁵ :** C-{[(CH₂)ₙ-O)]ₚ-R²}₂ |
| **X⁵ :** C-{[(CH₂)ₙ-O)]ₚ-R²}₂ | **X¹ :** N-[(CH₂)ₙ-O)]ₚ-R² | **X¹ :** N-[(CH₂)ₙ-O)]ₚ-R² |
| **X⁵** : C-{[(CH₂)ₙ-O)]ₚ-R²}₂ | **X² :** N-R₁-{O-[(CH₂)ₙ-O)]ₚ-R²}ₘ ; | **X²** : N-R₁-{O-[(CH₂)ₙ-O)]ₚ-R²}ₘ |
| **X⁵** : C-{[(CH₂)ₙ-O)]ₚ-R²}₂ | **X³ :** CH-[(CH₂)ₙ-O)]ₚ-R² | **X³ :** CH-[(CH₂)ₙ-O)]ₚ-R² |
| **X⁵** : C-{[(CH₂)ₙ-O)]ₚ-R²}₂ | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X⁵** : C-{[(CH₂)ₙ-O)]ₚ-R²}₂ | **X⁶ :** C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| **X⁶ :** C-(R¹-{O-[(CH₂)ₙ-O)]ₚ-R²}ₘ)₂ | **X⁶ :** C-(R¹-{O-[(CH₂)ₙ-O)]ₚ-R²}ₘ)₂ | **X⁶ :** C-**(**R¹-{O-[(CH₂)ₙ-O)]ₚ-R²}ₘ)₂ |
| **X⁶ :** C-**(**R¹-{O-[(CH₂)ₙ-O)]ₚ-R²}ₘ)₂ | **X¹ :** N-[(CH₂)ₙ-O)]ₚ-R² | **X¹** : N-[(CH₂)ₙ-O)]ₚ-R² |
| **X⁶ :** C-**(**R¹-{O-[(CH₂)ₙ-O)]ₚ-R²}ₘ)₂ | **X²** : N-R₁-{O-[(CH₂)ₙ-O)]ₚ-R²}ₘ ; | **X² :** N-R₁-{O-[(CH₂)ₙ-O)]ₚ-R²}ₘ |
| **X⁶ :** C-**(**R¹-{O-[(CH₂)ₙ-O)]ₚ-R²}ₘ)₂ | **X³** : CH-[(CH₂)ₙ-O)]ₚ-R² | **X³ :** CH-[(CH₂)ₙ-O)]ₚ-R² |
| **X⁶** : C-**(**R¹-{O-[(CH₂)ₙ-O)]ₚ-R²}ₘ)₂ | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X⁶ :** C-**(**R¹-{O-[(CH₂)ₙ-O)]ₚ-R²}ₘ)₂ | **X⁵ :** C-{[(CH₂)ₙ-O)]ₚ-R²}₂ | **X⁵** : C-{[(CH₂)ₙ-O)]ₚ-R²}₂ |

5. Verwendung von pi-konjugierten Verbindungen nach einem der Ansprüche 1 bis 4 als Halbleiter, insbesondere organische Halbleiter, oder als Farbstoffe.

6. Optoelektronische Vorrichtung wie beispielsweise eine photovoltaische Solarzelle mit photosensitivem Farbstoff (DSSC), eine photovoltaische Quantenpunkt-Solarzelle, eine photovoltaische Hybrid-Solarzelle, eine photovoltaische organische Solarzelle, eine photovoltaische Perowskit-Solarzelle, eine organische Leuchtdiode (OLEDs) und ein organischer Feldeffekttransistor (OFETs), die mindestens eine Verbindung nach einem der Ansprüche 1 bis 4 umfasst.

## Claims

1. Triarylamine of formula (IV): 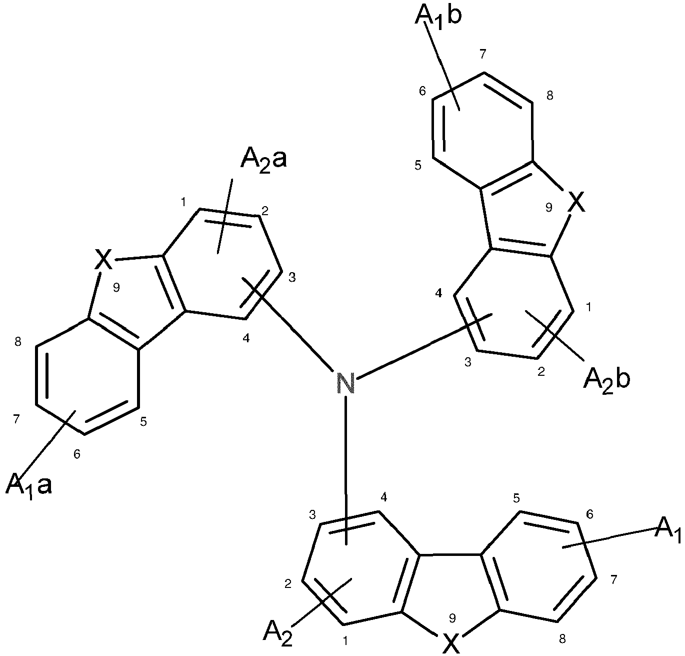 wherein:
X is chosen from the group consisting of:
**X¹** : N-[(CH₂)ₙ-O)]ₚ-R² ;
**X²** : N-R₁-{O-[(CH₂)ₙ-O)]ₚ-R²}ₘ;
**X³** : CH-[(CH₂)ₙ-O)]ₚ-R² ;
**X⁴** : CH-R¹-{O-[(CH₂)ₙ-O)]ₚ-R²}ₘ ;
**X⁵** : C-{[(CH₂)ₙ-O)]ₚ-R²}₂ ; and
**X⁶** : C-(R¹-{O-[(CH₂)ₙ-O)]ₚ-R²}ₘ)₂ ; or:
R¹ is selected from the group consisting of aryl or heteroaryl; preferably aryl;
n is an integer from 1 to 6, especially 2 or 3, in particular 2;
p = is an integer from 1 to 20;
m is an integer from 1 to 6, especially from 1 to 3, in particular 1;
and when m is greater than 1, the values of p and n in each group -{O-[(CH₂)ₙ-O)]ₚ- may be identical or different from each other.
R² is C₁ -C₁₂ alkyl; A¹, preferably located in position 6 or 7 of the ring, is selected from the group consisting of H; C₁-C₇ alkyl; C₂-C₇ alkenyl; C₂-C₇ alkynyl; aryl, heteroaryl; C₃-C₈ cycloalkyl; NO₂, FC₃; CN; N₃; F; Cl; NR^{a}R^{b}; OR^{c}; SR^{c}; C(=O)R^{d}; or a chromophore; in particular H or a chromophore; wherein:
R^{a} is selected from the group consisting of H; C₁-C₇ alkyl; aryl, heteroaryl, C₃-C₈ cycloalkyl; or R^{a} and R^{b} together form a (C₃-C₇) heterocyclyl;
R^{b} is selected from the group consisting of C₁ -C₇ alkyl; aryl, heteroaryl, C₃-C₈ cycloalkyl; or R^{a} and R^{b} together form a (C₃-C₇) heterocyclyl;
R^{c} is selected from the group consisting of C₁-C₇ alkyl; aryl, heteroaryl, C₃-C₈ cycloalkyl; C (=O) -C₁-C₇ alkyl; C(=O)-aryl, C(=O)-heteroaryl, C(=O)-C₃-C₈ cycloalkyl,
R^{d} represents C₁-C₇ alkyl; aryl, heteroaryl, C₃-C₈ cycloalkyl; NR^{a}R^{b}; OR^{c}; SR^{c};
A², preferably located in position 2 or 3 of the ring is selected from the group consisting of H; C₁-C₇ alkyl; C₂-C₇ alkenyl; C₂-C₇ alkynyl; aryl, heteroaryl; C₃-C₈ cycloalkyl; NO₂, FC₃; CN; N₃; F ; Cl; NR^{a}R^{b}; OR^{c}; SR^{c}; C(=O)R^{d}; or a chromophore; especially H; where:
R^{a} is selected from the group consisting of H; C₁-C₇ alkyl; aryl, heteroaryl, C₃-C₈ cycloalkyl; or R^{a} and R^{b} together form a (C₃-C₇) heterocyclyl;
R^{b} is selected from the group consisting of C₁-C₇ alkyl; aryl, heteroaryl, C₃-C₈ cycloalkyl; or R^{a} and R^{b} together form a (C₃-C₇) heterocyclyl;
R^{c} is selected from the group consisting of C₁-C₇ alkyl; aryl, heteroaryl, C₃-C₈ cycloalkyl; C (=O) -C₁-C₇ alkyl; C(=O)-aryl, C(=O)-heteroaryl, C(=O)- C₃-C₈ cycloalkyl,
R^{d} represents C₁-C₇ alkyl; aryl, heteroaryl, C₃-C₈ cycloalkyl; NR^{a}R^{b}; OR^{c}; SR^{c};
and A^{1a}, A^{2a}, A^{1b} and A^{2b} have the meaning given for A¹ and A²,
A^{2a}, A^{2b} and A² on the one hand and A^{1a}, A¹ and A^{1b} on the other hand may be identical or different from each other.

2. Triarylamine of formula (IV) according to claim 1, wherein A^{2a}, A^{2b} and A² are identical to each other and preferably represent H and A^{1a}, A¹ and A^{1b} are identical to each other.

3. Triarylamine according to one of claims 1 or 2, wherein A¹, A^{1a} and present A^{1b} represent H, aryl or a chromophore, in particular selected from the group consisting of the following structures: 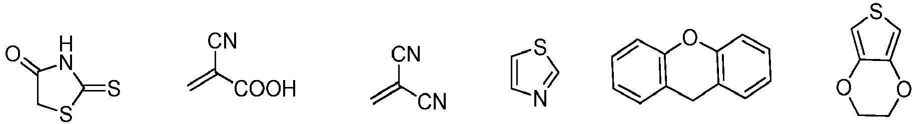 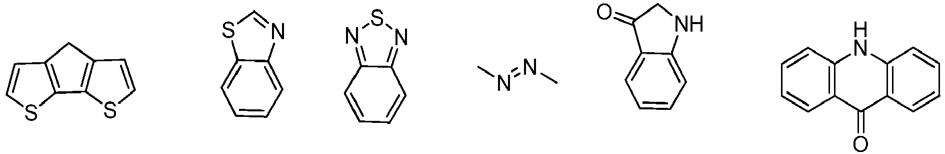 A¹, A^{1a} and A^{1b} represent in particular H.

4. Triarylamine of formula (IV) according to claim 1: 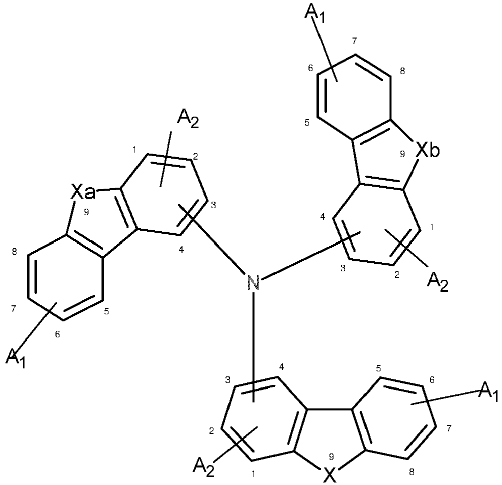 wherein A¹ represents H, A² represents H, and X, Xa and Xb are chosen from one of the following combinations:
| X | Xa | Xb |
|---|---|---|
| **X¹** : N-[(CH₂)ₙ-O)]ₚ-R² | **X¹** : N-[(CH₂)ₙ-O)]ₚ-R² | **X¹** : N-[(CH₂)ₙ-O)]ₚ-R² |
| **X¹** : N-[(CH₂)ₙ-O)]ₚ-R² | **X² :** N-R₁-{O-[(CH₂)ₙ-O)]ₚ-R²}ₘ ; | **X² :** N-R₁-{O-[(CH₂)ₙ-O)]ₚ-R²}ₘ |
| **X¹** : N-[(CH₂)ₙ-O)]ₚ-R² | **X³** : CH-[(CH₂)ₙ-O)]ₚ-R² | **X³** : CH-[(CH₂)ₙ-O)]ₚ-R² |
| **X¹** : N-[(CH₂)ₙ-O)]ₚ-R² | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X¹** : N-[(CH₂)ₙ-O)]ₚ-R² | **X⁵** : C-{[(CH₂)ₙ-O)]ₚ-R²}₂ | **X⁵** : C-{[(CH₂)ₙ-O)]ₚ-R²}₂ |
| **X¹** : N-[(CH₂)ₙ-O)]ₚ-R² | **X⁶** : C-(R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| **X² :** N-R₁-{O-[(CH₂)ₙ-O)]ₚ-R²}ₘ | **X² :** N-R₁-{O-[(CH₂)ₙ-O)]ₚ-R²}ₘ | **X² :** N-R₁-{O-[(CH₂)ₙ-O)]ₚ-R²}ₘ |
| **X² :** N-R₁-{O-[(CH₂)ₙ-O)]ₚ-R²}ₘ | **X¹** : N-[(CH₂)ₙ-O)]ₚ-R² | **X¹** : N-[(CH₂)ₙ-O)]ₚ-R² |
| **X² :** N-R₁-{O-[(CH₂)ₙ-O)]ₚ-R²}ₘ | **X³** : CH-[(CH₂)ₙ-O)]ₚ-R² | **X³** : CH-[(CH₂)ₙ-O)]ₚ-R² |
| **X² :** N-R₁-{O-[(CH₂)ₙ-O)]ₚ-R²}ₘ | **X⁴ :** CH-R¹-{O-[(CH₂)ₙ-O)]ₚ-R²}ₘ | **X⁴ :** CH-R¹-{O-[(CH₂)ₙ-O)]ₚ-R²}ₘ |
| **X² :** N-R₁-{O-[(CH₂)ₙ-O)]ₚ-R²}ₘ | **X⁵** : C-{[(CH₂)ₙ-O)]ₚ-R²}₂ | **X⁵** : C-{[(CH₂)ₙ-O)]ₚ-R²}₂ |
| **X² :** N-R₁-{O-[(CH₂)ₙ-O)]ₚ-R²}ₘ | **X⁶** : C-**(**R¹-{O-[(CH₂)ₙ-O)]ₚ-R²}ₘ)₂ | **X⁶** : C-**(**R¹-{O-[(CH₂)ₙ-O)]ₚ-R²}ₘ)₂ |
| **X³** : CH-[(CH₂)ₙ-O)]ₚ-R² | **X³** : CH-[(CH₂)ₙ-O)]ₚ-R² | **X³** : CH-[(CH₂)ₙ-O)]ₚ-R² |
| **X³** : CH-[(CH₂)ₙ-O)]ₚ-R² | **X¹** : N-[(CH₂)ₙ-O)]ₚ-R² | **X¹** : N-[(CH₂)ₙ-O)]ₚ-R² |
| **X³** : CH-[(CH₂)ₙ-O)]ₚ-R² | **X² :** N-R₁-{O-[(CH₂)ₙ-O)]ₚ-R²}ₘ ; | **X² :** N-R₁-{O-[(CH₂)ₙ-O)]ₚ-R²}ₘ |
| **X³** : CH-[(CH₂)ₙ-O)]ₚ-R² | **X⁴** : CH-R¹-{O-[(CH₂)ₙ-O)]ₚ-R²}ₘ | **X⁴** : CH-R¹-{O-[(CH₂)ₙ-O)]ₚ-R²}ₘ |
| **X³** : CH-[(CH₂)ₙ-O)]ₚ-R² | **X⁵ :** C-{[(CH₂)ₙ-O)]ₚ-R²}₂ | **X⁵ :** C-{[(CH₂)ₙ-O)]ₚ-R²}₂ |
| **X³ :** CH-[(CH₂)ₙ-O)]ₚ-R² | **X⁶** : C-(R¹-{O-[(CH₂)ₙ-O)]ₚ-R²}ₘ)₂ | **X⁶** : C-(R¹-{O-[(CH₂)ₙ-O)]ₚ-R²}ₘ)₂ |
| **X⁴** : CH-R¹-{O-[(CH₂)ₙ-O)]p-R²}ₘ | **X⁴** : CH-R¹-{O-[(CH₂)ₙ-O)]ₚ-R²}ₘ | **X⁴** : CH-R¹-{O-[(CH₂)ₙ-O)]ₚ-R²}ₘ |
| **X⁴** : CH-R¹-{O-[(CH₂)ₙ-O)]p-R²}ₘ | **X¹** : N-[(CH₂)ₙ-O)]ₚ-R² | **X¹** : N-[(CH₂)ₙ-O)]ₚ-R² |
| **X⁴** : CH-R¹-{O-[(CH₂)ₙ-O)]ₚ-R²}ₘ | **X²** : N-R₁-{O-[(CH₂)ₙ-O)]ₚ-R²}ₘ ; | **X²** : N-R₁-{O-[(CH₂)ₙ-O)]ₚ-R²}ₘ |
| **X⁴** : CH-R¹-{O-[(CH₂)ₙ-O)]ₚ-R²}ₘ | **X³** : CH-[(CH₂)ₙ-O)]ₚ-R² | **X³** : CH-[(CH₂)ₙ-O)]ₚ-R² |
| **X⁴** : CH-R¹-{O-[(CH₂)ₙ-O)]ₚ-R²}ₘ | **X⁵ :** C-{[(CH₂)ₙ-O)]ₚ-R²}₂ | **X⁵ :** C-{[(CH₂)ₙ-O)]ₚ-R²}₂ |
| **X⁴** : CH-R¹-{O-[(CH₂)ₙ-O)]ₚ-R²}ₘ | **X⁶** : C-(R¹-{O-[(CH₂)ₙ-O)]ₚ-R²}ₘ)₂ | **X⁶** : C-(R¹-{O-[(CH₂)ₙ-O)]ₚ-R²}ₘ)₂ |
| **X⁵ :** C-{[(CH₂)ₙ-O)]ₚ-R²}₂ | **X⁵ :** C-{[(CH₂)ₙ-O)]ₚ-R²}₂ | **X⁵ :** C-{[(CH₂)ₙ-O)]ₚ-R²}₂ |
| **X⁵ :** C-{[(CH₂)ₙ-O)]ₚ-R²}₂ | **X¹** : N-[(CH₂)ₙ-O)]ₚ-R² | **X¹** : N-[(CH₂)ₙ-O)]ₚ-R² |
| **X⁵ :** C-{[(CH₂)ₙ-O)]ₚ-R²}₂ | **X²** : N-R₁-{O-[(CH₂)ₙ-O)]ₚ-R²}ₘ ; | **X²** : N-R₁-{O-[(CH₂)ₙ-O)]ₚ-R²}ₘ |
| **X⁵ :** C-{[(CH₂)ₙ-O)]ₚ-R²}₂ | **X³** : CH-[(CH₂)ₙ-O)]ₚ-R² | **X³** : CH-[(CH₂)ₙ-O)]ₚ-R² |
| **X⁵ :** C-{[(CH₂)ₙ-O)]ₚ-R²}₂ | **X⁴** : CH-R¹-{O-[(CH₂)ₙ-O)]ₚ-R²}ₘ | **X⁴** : CH-R¹-{O-[(CH₂)ₙ-O)]ₚ-R²}ₘ |
| **X⁵ :** C-{[(CH₂)ₙ-O)]ₚ-R²}₂ | **X⁶** : C-(R¹-{O-[(CH₂)ₙ-O)]ₚ-R²}ₘ)₂ | **X⁶** : C-(R¹-{O-[(CH₂)ₙ-O)]ₚ-R²}ₘ)₂ |
| **X⁶** : C-(R¹-{O-[(CH₂)ₙ-O)]ₚ-R²}ₘ)₂ | **X⁶** : C-(R¹-{O-[(CH₂)ₙ-O)]ₚ-R²}ₘ)₂ | **X⁶** : C-(R¹-{O-[(CH₂)ₙ-O)]ₚ-R²}ₘ)₂ |
| **X⁶** : C-(R¹-{O-[(CH₂)ₙ-O)]ₚ-R²}ₘ)₂ | **X¹** : N-[(CH₂)ₙ-O)]ₚ-R² | **X¹** : N-[(CH₂)ₙ-O)]ₚ-R² |
| **X⁶** : C-(R¹-{O-[(CH₂)ₙ-O)]ₚ-R²}ₘ)₂ | **X² :** N-R₁-{O-[(CH₂)ₙ-O)]ₚ-R²}ₘ ; | **X² :** N-R₁-{O-[(CH₂)ₙ-O)]ₚ-R²}ₘ |
| **X⁶** : C-(R¹-{O-[(CH₂)ₙ-O)]ₚ-R²}ₘ)₂ | **X³** : CH-[(CH₂)ₙ-O)]ₚ-R² | **X³** : CH-[(CH₂)ₙ-O)]ₚ-R² |
| **X⁶** : C-(R¹-{O-[(CH₂)ₙ-O)]ₚ-R²}ₘ)₂ | **X⁴** : CH-R¹-{O-[(CH₂)ₙ-O)]ₚ-R²}ₘ | **X⁴** : CH-R¹-{O-[(CH₂)ₙ-O)]p-R²}ₘ |
| **X⁶** : C-(R¹-{O-[(CH₂)ₙ-O)]ₚ-R²}ₘ)₂ | **X⁵ :** C-{[(CH₂)ₙ-O)]ₚ-R²}₂ | **X⁵ :** C-{[(CH₂)ₙ-O)]ₚ-R²}₂ |

5. Use of pi-conjugated compounds according to one of claims 1 to 4 as semiconductors, in particular organic semiconductors, or dyes.

6. Optoelectronic device such as a photosensitive dye photovoltaic solar cell (DSSC), a quantum dot photovoltaic solar cell, a hybrid photovoltaic solar cell, an organic photovoltaic solar cell, a perovskite photovoltaic solar cell, an organic light emitting diode (OLEDs) and an organic field-effect transistor (OFETs) comprising at least one compound according to one of claims 1 to 4.
